(19) **Europäisches Patentamt / European Patent Office / Office européen des brevets**

(11) **EP 4 783 162 A1**

(12) **EUROPEAN PATENT APPLICATION**
published in accordance with Art. 153(4) EPC

(43) Date of publication:
29.07.2026 Bulletin 2026/31

(21) Application number: 25765460.8

(22) Date of filing: 08.08.2025

(51) International Patent Classification (IPC):
*G10L 17/26* (2013.01)     *G10L 25/63* (2013.01)
*G10L 15/24* (2013.01)     *G10L 25/30* (2013.01)

(52) Cooperative Patent Classification (CPC):
**G10L 25/63; G06V 40/20**

(86) International application number:
**PCT/CN2025/113694**

(87) International publication number:
**WO 2026/118524 (11.06.2026 Gazette 2026/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 06.12.2024 CN 202411793938

(71) Applicant: **Beijing Baidu Netcom
Science Technology Co., Ltd.
Beijing 100085 (CN)**

(72) Inventors:
• **WU, Chengcheng
  Beijing 100085 (CN)**
• **JIA, Dianlong
  Beijing 100085 (CN)**
• **HE, Zhenjiang
  Beijing 100085 (CN)**

• **YAN, Hao
  Beijing 100085 (CN)**
• **RAN, Yong
  Beijing 100085 (CN)**
• **LIU, Weiyun
  Beijing 100085 (CN)**
• **LIU, Haifeng
  Beijing 100085 (CN)**
• **WANG, Zeyu
  Beijing 100085 (CN)**
• **ZHANG, Ye
  Beijing 100085 (CN)**
• **LIU, Baoju
  Beijing 100085 (CN)**

(74) Representative: **Hannke Bittner & Partner mbB
Regensburg
Prüfeninger Straße 1
93049 Regensburg (DE)**

(54) **ANIMAL LANGUAGE CONVERSION METHOD AND APPARATUS, ANIMAL EMOTION RECOGNITION METHOD AND APPARATUS, AND MODEL TRAINING METHOD AND APPARATUS**

(57) The present disclosure provides an animal language translation method, an emotion recognition method, and a model training method and apparatus, which relates to the field of artificial intelligence technology, particularly to the field of machine learning technology, deep learning technology, natural language processing technology and the like. A specific implementation solution involves acquiring animal-related multimodal data; preprocessing the multimodal data to obtain fused multimodal data; recognizing current animal emotions based on the fused multimodal data to obtain animal emotion recognition results; performing semantic mapping and language translation on the emotion recognition results to translate animal languages into human languages to obtain language translation results.

FIG. 1

# EP 4 783 162 A1

## Description

The present disclosure claims the priority of Chinese patent application No. 202411793938.3 entitled "ANIMAL LANGUAGE TRANSLATION METHOD, APPARATUS, ELECTRONIC DEVICE AND STORAGE MEDIUM" filed to the China National Intellectual Property Administration on December 06, 2024, the contents of which are hereby incorporated by reference in their entireties.

## TECHNICAL FIELD

The present disclosure relates to the field of artificial intelligence technology, and particularly to the field of machine learning technology, deep learning technology, natural language processing technology and the like.

## BACKGROUND

A technology currently available in the market attempts to interpret emotion worlds of animals through some basic animal vocalization and behavior translation apparatuses and pet emotion analysis tools using an artificial intelligence image recognition technology. However, these approaches are typically limited to superficial interpretations of animal behaviors. These technologies cannot decipher intricacies of animal emotions, and consequently are incapable of enabling profound real-time emotion comprehension and bidirectional human-animal communication.

## SUMMARY

The present disclosure provides an animal language translation method, an emotion recognition method, a model training method and apparatus.

According to an aspect of the present disclosure, provided is an animal language translation method, including:

acquiring animal-related multimodal data, wherein the multimodal data includes animal sound data, animal behavior data and animal physical sign data;

preprocessing the multimodal data to obtain fused multimodal data;

recognition current animal motions based on the fused multimodal data to obtain animal emotion recognition results; and

performing semantic mapping and language translation on the emotion recognition results to translate animal languages into human languages to obtain language translation results.

According to an aspect of the present disclosure, provided is a method for training an animal emotion recognition model in an animal language translation model, including:

acquiring animal-related multimodal data;

preprocessing the multimodal data to obtain fused multimodal data;

inputting the fused multimodal data into the animal emotion recognition model to recognize current animal motions to obtain animal emotion recognition results; and

training the animal emotion recognition model based on the emotion recognition results, wherein semantic mapping and language translation are performed on the emotion recognition results output by the animal emotion recognition model to translate animal languages into human languages to obtain language translation results.

According to another aspect of the present disclosure, provided is an animal emotion recognition method, including:

acquiring animal-related multimodal data;

preprocessing the multimodal data to obtain fused multimodal data;

recognition current animal motions based on the fused multimodal data to obtain animal emotion recognition results.

According to further aspect of the present disclosure, provided is a method for training an animal emotion recognition model, including:

acquiring animal-related multimodal data;

preprocessing the multimodal data to obtain fused multimodal data;

inputting the fused multimodal data into the animal emotion recognition model to recognize current animal motions to obtain animal emotion recognition results; and

training the animal emotion recognition model based on the emotion recognition results.

According to yet another aspect of the present disclosure, provided is an animal language translation apparatus, including:

a first acquisition module, configured to acquire animal-related multimodal data, wherein the multimodal data includes animal sound data, animal behavior data and animal physical sign data;

a first preprocessing module, configured to preprocess the multimodal data to obtain fused multimodal data;

a first recognition module, configured to recognize current animal motions based on the fused multimodal data to obtain animal emotion recognition results; and

a translation module, configured to perform semantic mapping and language translation on the emotion recognition results to translate animal languages into human languages to obtain language translation results.

According to yet further aspect of the present disclosure, provided is an apparatus for training an animal emotion recognition model in an animal language translation model, including:

a second acquisition module, configured to acquire animal-related multimodal data;

a second preprocessing module, configured to preprocess the multimodal data to obtain fused multimodal data;

a second recognition module, configured to input the fused multimodal data into the animal emotion recognition model to recognize current animal motions to obtain animal emotion recognition results; and

a training module, configured to, based on the emotion recognition results, train the animal emotion recognition model, wherein semantic mapping and language translation are performed on the emotion recognition results output by the animal emotion recognition model to translate animal languages into human languages to obtain language translation results.

According to further aspect of the present disclosure, provided is an animal emotion recognition apparatus, including:

a first acquisition module, configured to acquire animal-related multimodal data;

a first preprocessing module, configured to preprocess the multimodal data to obtain fused multimodal data; and

a first recognition module, configured to recognize current animal motions based on the fused multimodal data to obtain animal emotion recognition results.

According to further aspect of the present disclosure, provided is an apparatus for training an animal emotion recognition model, including:

a second acquisition module, configured to acquire animal-related multimodal data;

a second preprocessing module, configured to preprocess the multimodal data to obtain fused multimodal data;

a second recognition module, configured to input the fused multimodal data into the animal emotion recognition model to recognize current animal motions to obtain animal emotion recognition results; and

a training module, configured to train the animal emotion recognition model based on the emotion recognition results.

According to further aspect of the present disclosure, provided is an electronic device, including:

at least one processor; and

a memory communicatively connected to the at least one processor, wherein

the memory stores an instruction executable by the at least one processor, and the instruction is executable by the at least one processor to cause the at least one processor to perform any one of embodiments of the present disclosure.

According to further aspect of the present disclosure, provided is a non-transitory computer-readable storage medium storing a computer instruction, wherein the computer instruction is used to cause a computer to perform the method of any one of embodiments of the present disclosure.

According to further aspect of the present disclosure, provided is a computer program product including a computer program that, when being executed by a processor, implements the method according to any one of embodiments of the present disclosure.

It should be understood that statements described in this section are not intended to identify key or essential features of embodiments of the present disclosure, nor limit the scope of the present disclosure. Further features of the present disclosure will become apparent from the following description.

BRIEF DESCRIPTION OF THE DRAWINGS

Accompanying drawings are used for better understanding the present solution, and do not constitute limitations to the present disclosure. In the accompanying drawings:

FIG. 1 is a schematic flowchart of an animal language translation method according to an embodiment of the present disclosure.
FIG. 2 is a schematic flowchart of acquiring multimodal data according to an embodiment of the present disclosure.
FIG. 3 is a schematic flowchart of preprocessing multimodal data according to an embodiment of the present disclosure.
FIG. 4 is a schematic flowchart of obtaining animal emotion recognition results according to an embodiment of the present disclosure.
FIG. 5 is a schematic flowchart of obtaining multimodal feature vectors according to an embodiment of the present disclosure.
FIG. 6 is a schematic flowchart of obtaining animal emotion recognition results according to an embodiment of the present disclosure.
FIG. 7 is a schematic flowchart of a method for training an animal emotion recognition model according to an embodiment of the present disclosure.
FIG. 8 is a schematic flowchart of fusing fused features at the same time step by a mid-level attention network according to an embodiment of the present disclosure.
FIG. 9 is a schematic structural diagram of a fusion network for fusing multimodal features at the same time step according to an embodiment of the present disclosure.
FIG. 10 is a schematic flowchart of training an animal emotion recognition model through adversarial training according to an embodiment of the present disclosure.
FIG. 11 is another schematic flowchart of training an animal emotion recognition model through adversarial training according to an embodiment of the present disclosure.
FIG. 12 is a schematic flowchart of training an animal emotion recognition model based on emotion recognition results according to an embodiment of the present disclosure.
FIG. 13 is a structural diagram of a main framework of an animal emotion recognition model according to an embodiment of the present disclosure.
FIG. 14 is a schematic flowchart of training an animal emotion recognition model based on pseudo labels according to an embodiment of the present disclosure.
FIG. 15 is a schematic flowchart of generating pseudo labels of animal emotions according to an embodiment of the present disclosure.
FIG. 16 is another schematic flowchart of generating pseudo labels of animal emotions according to an embodiment of the present disclosure.
FIG. 17 is a schematic flowchart of optimizing an animal emotion recognition model according to an embodiment of the present disclosure.
FIG. 18 is a schematic flowchart of translating animal languages into human languages according to an embodiment of the present disclosure.
FIG. 19 is a schematic flowchart of an animal emotion recognition method according to an embodiment of the present disclosure.
FIG. 20 is a schematic flowchart of a method for training an animal emotion recognition model in an animal language translation model according to an embodiment of the present disclosure.
FIG. 21 is a schematic flowchart of updating emotion labels according to an embodiment of the present disclosure.
FIG. 22 is a schematic structural diagram of an animal emotion recognition apparatus according to an embodiment of the present disclosure.
FIG. 23 is a schematic structural diagram of an apparatus for training an animal emotion recognition model according to an embodiment of the present disclosure.
FIG. 24 is a schematic structural diagram of an animal language translation apparatus according to an embodiment of the present disclosure.
FIG. 25 is a schematic structural diagram of an apparatus for training an animal emotion recognition model in a language translation model according to an embodiment of the present disclosure.
FIG. 26 is a block diagram of an electronic device used to implement any method of embodiments of the present disclosure.

DETAILED DESCRIPTION

Exemplary embodiments of the present disclosure will be described hereinafter with reference to accompanying drawings, which include various details of embodiments of the present disclosure to facilitate understanding, and which should be considered as exemplary only. Therefore, those ordinarily skilled in the art should realize that various changes and modifications can be made to embodiments described herein without departing from the scope and spirit of the present disclosure. Likewise, for the sake of clarity and brevity, descriptions of conventional functions and structures are omitted from the following description.

Terms "first" and "second" and the like used in the present disclosure are intended to distinguish similar objects and do not necessarily describe a specific sequence or chronological order. In addition, terms "including" and "having" as well as any variations thereof are intended to cover non-exclusive inclusions, such as including a series of steps or units. Methods, systems, products or devices do not necessarily need to be limited to those clearly listed, but may include further steps or units that are not clearly listed or inherent to these processes, methods, products or devices.

It should be noted that operations shown in flowcharts of embodiments of the present disclosure may be executed in any chronological order or a plurality of operations may be executed concurrently, unless it is explicitly stated that different operations can be executed in a chronological order or that different operations can be executed in a chronological order in terms of a technical implementation.

At present, technologies available on the market regarding the human-animal communication can be broadly categorized into two approaches as below.

A first approach is to use a simple translation apparatus for animal vocalizations and behaviors to achieve an animal emotion translation function mainly based on "a voiceprint database and a simple algorithm". A typical example of the first approach is a certain commercially available pet behavior and sound recognition device, which can use a relatively simple sensor to capture animal sounds and some typical actions, and then match them to a pre-built emotion library for simple emotion mapping, such as recognizing dog barks as representing requests for food, anger and the like.

A second approach is a pet emotion analysis tool based on AI (Artificial Intelligence) image recognition, which introduces image processing and AI technology to help a user understand emotion responses of pets. For instance, some companies analyze different facial expressions of animals by capturing animal faces and actions in camera image data in combination an existing trained deep learning model, thereby recognizing specific expressions such as happiness, anger, sadness and confusion.

With respect to the two approaches described above, there are a plurality of limitations in understanding and translating animal emotions. Firstly, emotion translation is oversimplified and relies on preset voiceprint data and behavior classification, which cannot continuously track emotion changes, resulting in insufficient accuracy in complex scenarios. Secondly, the lack of multimodal fusion analysis and the excessive reliance on a single information source restrict the comprehensiveness and accuracy of the emotion translation. Thirdly, the insufficient temporal detection and the lack of the capability of continuously tracking emotion states result in a sluggish perception of the emotion changes. In addition, the absence of an adaptive learning and tuning mechanism leads to a difficulty in optimization and iteration in the face of unfamiliar emotion patterns, which limits the flexibility and scalability of the system. Finally, edge computing is not available, resulting in insufficient real-time performance, which affects the instant human-animal interaction. These limitations collectively contribute to shortcomings of the prior art in achieving deep real-time cross-species emotion communication.

In order to solve at least one of the technical problems, the present disclosure provides an animal emotion recognition method. Animal emotions in embodiments of the present disclosure may include temporary emotions expressed by the animals, such as anger and sadness, and further include animal needs, such as a request for food. Therefore, the animal emotions in embodiments of the present disclosure mainly involve transforming animal-specific expressions into human-understandable expressions, in order to facilitate the understanding of the animal behaviors and needs.

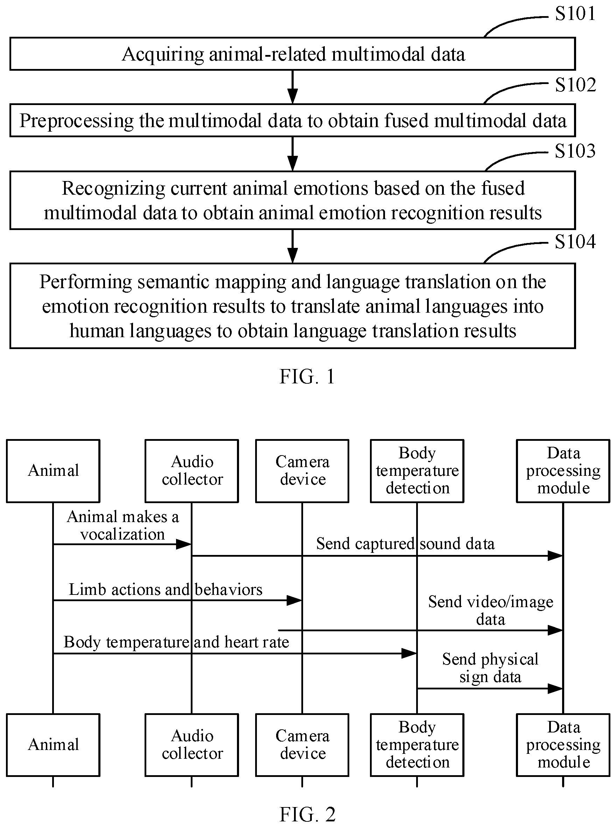

Reference is made to FIG. 1 which is a schematic view of steps of an animal language translation method according to an embodiment of the present disclosure. The animal language translation method can be applied to a server side. The method includes the following steps.

At a step S101, animal-related multimodal data is acquired.

During implementation, the multimodal data includes animal sound data, animal behavior data and animal physical sign data. During implementation, types of modalities can be increased or decreased according to actual needs, which is not limited in embodiments of the present disclosure.

Specifically, the acquiring animal-related multimodal data involves collecting information of different modalities by various sensors to comprehensively understand animal states and emotions. the "animal sound data" refers to various vocalizations, which are captured through an audio sensor, made by the animals, which can reflect the animal emotions and needs. The "animal behavior data" refers to actions (such as facial expressions and body actions) and postures, which are recorded by a video camera, of the animals, which help analyze their behavior patterns and emotion expressions. The "animal physical sign data" refers to physiological indicators, which are monitored by a physiological sensor, of the animals, such as heart rates and body temperatures, which can provide the physiological basis for the animal emotion states.

In this way, by obtaining the animal-related multimodal data, including sound, behavior and physical sign data, the animal emotions and behaviors can be comprehensively captured, providing more accurate emotion analysis and behavior understanding, enhancing human-animal communication and interaction, improving the animal welfare, and increasing the responsiveness of the humans to the animal behaviors.

During implementation, the multimodal data can be collected by a terminal device. Data of each modality can be collected by a corresponding sensor. At least one sensor can be integrated in the same terminal device, which is not limited in embodiments of the present disclosure. The multimodal data can be transmitted to a server after being collected by the terminal device. The collected multimodal data can be stored in the server for the server to acquire the multimodal data and further perform the animal language translation method and/or the animal emotion recognition method provided in embodiments of the present disclosure.

At a step S102, the multimodal data is preprocessed to obtain fused multimodal data.

Specifically, the preprocessing the multimodal data can include a series of processing steps on raw data collected from different sources such as audio, video and physiological sensors. For example, the preprocessing the multimodal data can include denoising, normalization, feature extraction and the like on the multimodal data to facilitate analysis and understanding. Moreover, the "fused multimodal data" refers to integration of these preprocessed data into a unified dataset, which involves time alignment (ensuring that all the data are synchronized in time), feature fusion (merging features of different modalities into a comprehensive feature vector), and data synchronization (handling differences in timestamps and sampling rates of different data streams). By means of such preprocessing and fusion, a comprehensive data view can be provided, making subsequent emotion recognition and language translation more accurate and efficient. The feature fusion here can involve concatenating data of different modalities together, such that the fused multimodal data still retains the independence of each modality for subsequent processing.

In this way, by preprocessing the multimodal data such as animal sounds, behaviors, and physical signs and then fusing these data into the unified dataset, the consistency and usability of the data can be improved, making emotion recognition and language translation more accurate, enhancing the comprehensive understanding capability of the system for the animal behaviors and emotion states, and improving the efficiency and effectiveness of the cross-species communication.

During implementation, at least one of the preprocessing operations described above can be completed by the terminal device.

At a step S103, current animal motions are recognized based on the fused multimodal data to obtain animal emotion recognition results.

Specifically, after the fused multimodal data is obtained, the current animal emotions are recognized based on the fused multimodal data. Particularly, the animal emotion states are analyzed and determined by utilizing the unified dataset integrating information such as the animal sounds, behaviors and physical signs and adopting machine learning and deep learning technologies. The "emotion recognition" here refers to recognition of the animal emotion states, such as anxiety, excitement or relaxation, by analyzing features extracted from these multimodal data. To obtain the animal emotion recognition results, for example, the fused data can be input into a trained emotion recognition model, and the trained emotion recognition model outputs the animal emotion recognition results by comparing features of known emotion states. In this way, a basis for subsequent language translation and human-computer interaction can be provided. The animal emotion recognition model can be pre-selected and trained separately for recognizing the animal emotions. Processes of a training process and an inference process for recognizing the animal emotions are consistent, and the training the animal emotion recognition model will be illustrated below.

In this way, analyzing the fused multimodal data including the animal sounds, behaviors and physiological signs is beneficial to accurately recognize current animal emotion states. Such a process not only improves the accuracy of understanding and responding to the animal emotions, but also enhances the human-animal communication, enabling humans to better interpret the animal needs and emotions, thereby improving the animal welfare and strengthen the human-animal companionship.

At a step S104, semantic mapping and language translation are performed on the emotion recognition results to translate animal languages into human languages to obtain language translation results.

Specifically, the "performing semantic mapping and language translation on the emotion recognition results" refers to a process of transforming animal emotion states obtained by analyzing the multimodal data, such as anxiety, excitement or happiness, into human-understandable language expressions. Specifically, such a process involves establishing, by a pre-trained language model and a deep learning technology, a corresponding relationship (referred to be as the "semantic mapping") between animal emotion features and corresponding expressions in the human languages. Subsequently, these mapping results are converted into specific text or speech outputs to achieve the "language translation". Finally, the "language translation results" refer to translation of animal emotions and intentions into forms of the human languages, enabling human users to understand the animal "languages" more intuitively and conveniently, thereby achieving effective cross-species communication.

In this way, by performing semantic mapping and language translation on the emotion recognition results, non-speech communication of the animals can be converted into the human-understandable languages. Such a process not only eliminates human-animal communication obstacles, but also substantially augments the interpretation of humans to the animal emotions and needs, making human-animal interactions more harmonious while providing innovative perspectives and tools for the animal welfare and behavior research.

The present disclosure achieves comprehensive capture and accurate recognition of the animal emotions by acquiring the animal-related multimodal data. Further, the animal emotion states and intentions are transformed into human-understandable languages by the semantic mapping and language translation technologies, greatly enhancing the human-animal communication capability, improving the accuracy of interpretating animal emotion and real-time performance of the human-animal interaction, and providing a new human-animal communication way. That is, this solution can accurately recognize the current animal emotion states and translate them into the human languages, thereby strengthening human-animal emotion communication and interpretation, and improving the accuracy and efficiency of the cross-species communication.

In some alternate embodiments, the acquiring the animal-related multimodal data includes: collecting sound wave information made by the animals to obtain animal sound data; collecting animal body languages and action changes to obtain animal behavior data; and collecting physiological indicators of the animals to obtain animal physical sign data.

Specifically, the capturing vocalizations made by the animals by an audio collector is implemented as collecting sound wave information made by the animals to obtain the "animal sound data". Moreover, the animal body languages and action changes are collected by a visual sensor such as a video camera to obtain the "animal behavior data" reflecting activities and non-speech behaviors of the animals. In addition, the "animal physical sign data" such as heartbeats and body temperatures of the animals are monitored by a physiological sensor to provide important information for interpreting the physiological states and emotions of the animals. The communication means and emotion states of the animals can be comprehensively captured by integrating these multimodal data, creating a robust basis for further analysis and accurate emotion recognition.

For a better understanding of solutions of embodiments of the present disclosure, the following examples are given as shown in FIG. 2 which is a schematic flowchart of obtaining multimodal data according to an embodiment of the present disclosure. Firstly, the vocalizations made by the animals are captured by the audio collector in real time. Then, the captured sound data is transmitted to a data processing module by the audio collector, and can be subjected to noise processing by the data processing module using a corresponding audio filter so as to reduce background interference. For example, for dog barks, dimensional information such as pitch, amplitude, duration, frequency and breakpoint changes are sampled together to capture all the information of the dog barks. The data processing module can be provided at a terminal device or on a server.

The collecting the animal behavior data can be implemented as obtaining, by a camera device (such as a high-definition camera and an infrared camera), animal body actions and behaviors, such as analysis of postures like wagging tails, jumping and lying down, supplemented by specific animal body characterization features (ear perking up, pupil dilation, and the like). Then, the captured video/image data is transmitted to the data processing module.

The collecting the animal physical sign data can be implemented as collecting, by a high-precision contact or non-contact body temperature detection sensor, heart rates and body temperatures and then transmitting the collected physical sign data to the data processing module.

In this way, comprehensive animal sound data, behavior data, and physical sign data can be obtained by collecting the sound wave information, body languages and action changes made by the animals, as well as physiological indicators. Integrating these pieces of multidimensional information provides a comprehensive and accurate database for deeply interpretating and analyzing the animal emotions and behaviors, enabling humans to more accurately interpret animal communication intentions and physiological states, strengthening the human-animal non-speech communication, and optimizing the animal husbandry and training efficacy, while developing novel methodologies for animal health surveillance and behavior research.

In some alternate embodiments, the preprocessing the multimodal data to obtain fused multimodal data includes:

denoising the multimodal data for cleaning the data to obtain the cleaned multimodal data;
normalizing the cleaned multimodal data to obtain normalized multimodal data; and
temporally aligning and fusing the normalized multimodal data to obtain the fused multimodal data.

Specifically, the denoising the multimodal data for cleaning the data involves removing noise and interference from audio and video data by using a signal processing technology to improve the data quality and obtain cleaned multimodal data. Next, the "normalizing the cleaned multimodal data" involves converting data from different sources and different scales into a unified format or scale, such that machine learning model can process the data more effectively to obtain normalized multimodal data. Finally, the "temporally aligning and fusing the normalized multimodal data" involves temporally aligning data of different modalities (such as aligning through timestamps) and merging them into a unified dataset, which ensures the temporal consistency of the data and integrates information from different sensors. Finally, the fused multimodal data is obtained, that is, data of different modalities are concatenated. The fused multimodal data provides an accurate and comprehensive database for subsequent emotion recognition and behavior analysis.

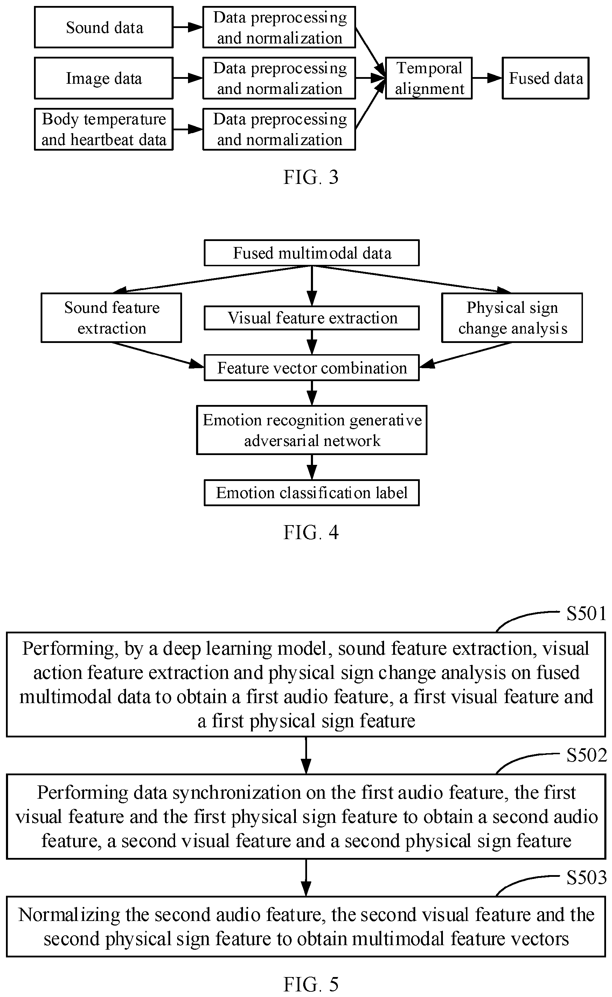

For a better understanding of solutions of embodiments of the present invention, the following examples will be given as shown in FIG. 3 which is a schematic flowchart of preprocessing multimodal data according to an embodiment of the present disclosure. Firstly, sound data, image data, as well as body temperature and heartbeat data in the multimodal data are preprocessed and normalized, respectively. The preprocessing includes denoising and cleaning for processing invalid parts in audio and visual information, for example, filtering out noise (such as wind and speech) possibly generated by humans, such that audio signals are clearly and easily recognized. Background motions and objects in the video frames on the image are removed to retain only key information, such as animal behavior changes. Next, data normalization is required to be performed on both audio signals and video and physical sign information, converting them into unified standards and expressing them into feature vectors handled by a machine learning algorithm.

Finally, the multimodal data is temporally aligned and fused. The data alignment, which resolves spatiotemporal differences in collecting signals of different modalities, is a prerequisite for the data fusion. For example, a dog barking event frequently shows a corresponding temporal offset from body actions or body temperature variations. These data can be temporally calibrated before being input, such that multimodal inputs in translation tasks share a unified reference point.

During implementation, sensors of different modalities can collect the data based on their respective sampling rates. Each data frame collected by the terminal device can be timestamped correspondingly by the terminal device. When the temporal alignment is performed, such an operation can be completed based on timestamps. For example, one of the modalities is selected as a reference modality, and a timestamp of the reference modality is a reference timestamp. In addition, a time difference threshold can be determined based on a difference in sampling rates of different modalities. Data with a temporal difference between timestamps of further modalities and the reference timestamp within such a time difference threshold range serves as data generated at the same time as the reference timestamp, thereby aligning data of different modalities based on the timestamps so as to achieve the temporal alignment.

Assuming that the animal sound data, also known as audio data, has the highest sampling rate, the timestamp of the audio can be used as the reference timestamp. In the data of the video modality and the physical sign modality, data around the reference timestamp is searched respectively (i.e., a difference between the timestamps of the further modalities and the reference timestamp is within the time difference threshold range), in order to temporally aligning the data of the video modality and the data of the physical sign modality to the data of the audio modality, respectively. Certainly, it should be understood that if sampling rates of the video modality and the physical sign modality are not consistent, a temporal difference threshold used to search for the data of the video modality and the temporal difference threshold used to search for the data of the physical sign modality may be different. Therefore, regardless of the method used, it is possible to align times of collecting the data of different modalities, which is not limited in embodiments of the present disclosure.

Further, during implementation, the sampling rates of the data of the different modalities can be aligned through data synchronization due to the difference in sampling rates of the data of the different modalities. For example, if the sampling rates of the video modality and the physical sign modality are low, the data of the video modality and the physical sign modality can be upsampled after being temporally aligned, such that the frame counts of the data in the individual modalities within the same time window are the same for subsequent recognition of the animal emotions.

During implementation, it is possible to enable the data of different modalities to be temporally aligned based on a temporal synchronization mechanism. For example, a NTP (Network Time Protocol) is a standard protocol for temporal synchronization between various devices in a network. In embodiments of the present disclosure, the NTP is used to ensure that system clocks for collecting audio, video and physiological signals are precisely synchronized, thereby ensuring global consistency of timestamps assigned to all the data frames and avoiding data mismatches caused by clock drift during subsequent alignment.

During implementation, it is possible to support an edge device for preprocessing the data. The preprocessing typically includes: data cleaning, sampling rate conversion (i.e., the data synchronization for synchronizing the sampling rates), frame energy normalization, and the like. The edge device can be understood as an intelligent terminal device deployed on a collection end (such as an embedded collection box, a smart collar, and the like). The edge device is directly responsible for raw data collection and at least a part of the preprocessing.

The data cleaning may include outlier detection and removal. Outlier definition depends on a physical property of the data of each modality and a sampling environment. For example, a physiological signal exceeding a physiological threshold (such as a dog heart rate>240bpm) may be considered anomalous. Likewise, artifacts in the sampled signal, such as abrupt changes, dropouts, or energy levels that are abnormally low or exceed a hardware limit, also constitute anomalies. During implementation, the outlier detection can be performed by setting a statistical interval based on a $3\sigma$

principle or a machine learning method (such as isolation forest or LOF), and detected outliers can be removed.

The preprocessing can further incorporate timestamping, which involves timestamping each collected data frame for subsequent temporal alignment.

The preprocessing can further incorporate data fusion, that is, the data of different modalities are concatenated to enable multimodal data collected at the same time to be fused, thereby representing different performances of the animals at the same time from different modalities.

It is possible to support resumable data transfer and cache management for the collected multimodal data, which can be implemented as follows: all the collected data are timestamped with high precision. During the resumable data transfer, timestamp queues of the data of each modality are compared. Data to be retransmitted is then inserted into a correct temporal position, thereby restoring data synchronization of a plurality of modalities. An "alignment buffer" can be introduced to temporarily buffer multimodal data within incomplete time windows. Data of all the modalities within the current time window is output downstream after being supplemented. Certainly, if the data within the current time window is remains incomplete by the onset of the next time window, the data can still be transmitted downstream for subsequent processing based on the data within the current time window.

In this way, by performing denoising, normalization, temporal alignment and fusion on the multimodal data, the quality, consistency and usability of the data can be significantly improved, making the features extracted from animal sounds, behaviors and physical signs more accurate and reliable. Such a process not only enhances the accuracy of data analysis, but also improves the accuracy of the model in recognizing the animal emotions and behaviors, providing a solid data foundation for achieving efficient and accurate cross-species communication.

In some embodiments, the data of different modalities is concatenated in a standardized format to obtain the fused multimodal data, and then the fused multimodal data is transmitted.

For example, a data structure of a single modality may include a timestamp, a modality identifier collected at the timestamp, and a data value of the modality. After time steps are aligned, data of a plurality of modalities corresponding to the same timestamp can be concatenated in a standardized format. The data structure in the standardized format may include a timestamp and data of a plurality of modalities corresponding to the timestamp. For example, the data structure includes animal sound data corresponding to an audio identifier, image data corresponding to a video identifier, and animal physical sign data corresponding to a physical sign identifier. Certainly, due to different sampling rates, data of some modalities may be missed at certain timestamps. If data synchronization is performed in a preprocessing stage, it is possible to ensure that as much complete multimodal data as possible exists at the same timestamp. If no data synchronization is performed in the preprocessing stage, it is possible to allow missing data of some modalities at certain timestamps. In this case, data synchronization can be deferred to a later stage and performed on higher-level features extracted by a neural network model.

In some alternate embodiments, the recognizing current animal motions based on the fused multimodal data to obtain animal emotion recognition results includes:

performing, by a deep learning model, sound feature extraction, visual action feature extraction and physical sign change analysis on the fused multimodal data to obtain multimodal feature vectors, wherein the multimodal feature vectors include feature expressions of different modalities, for example, includes features of an audio modality, a video modality and a physical sign modality, in order to accurately understand features of the modalities subsequently through a generative adversarial network and mine feature associations between different modalities; and
performing emotion analysis, by the generative adversarial network, on the multimodal feature to obtain the animal emotion recognition results.

Specifically, the "performing, by a deep learning model, sound feature extraction, visual action feature extraction and physical sign change analysis on the fused multimodal data" involves processing and analyzing, by an advanced deep learning technology such as a convolutional neural network (CNN) and a recurrent neural network (RNN), a multimodal dataset integrating sound, visual and physiological data. Due to a fact that data of different modalities can express corresponding emotions separately, such a process involves extracting sound features from audio signals separately, such as features expressed by pitch, rhythm and volume; extracting visual action features from video data separately, such as features expressed by posture and behavior patterns; and separately analyzing physical sign changes from physiological data, such as features expressed by heart rate and body temperature variations. The sum of the features expressed by these different modalities collectively forms a multimodal feature vector, wherein the feature of each modality in the multimodal feature vector exists independently and can be analyzed separately. Integrating these features can comprehensively reflect physiological and behavioral states of the animals. Next, the "performing, by the generative adversarial network, emotion analysis on the multimodal feature" refers to using a deep learning framework such as a generative adversarial network (GAN) to enable the network to recognize and distinguish different emotion states through an adversarial training process. Such a process ultimately produces the animal emotion recognition results, transforms complex animal emotions and behaviors into understandable emotion labels, and provides a basis for further semantic mapping and language translation.

For a better understanding of solutions of embodiments of the present invention, the following example will be given as shown in FIG. 4 which is a schematic flowchart of obtaining animal emotion recognition results according to an embodiment of the present disclosure. Performing, by the deep learning model, fine-grained feature extraction on the data of each modality after obtaining the fused multimodal data particularly involves performing, by the deep learning model, on sound feature extraction, visual action feature extraction and physical sign change analysis to obtain a feature vector combination (i.e., a multimodal feature vector). Next, voiceprint features, action changes and physical sign variations in the data are analyzed by using a large model based on an emotion recognition generative adversarial network (GAN) to obtain an emotion classification label. For example, when low-frequency barks of animals are detected accompanied by stiffened limbs and dilated pupils, the model recognizes the animals as being in a highly-alert state through comparative inference, and further deduces potential underlying psychological activities (fear or confusion) via feature association. The feature association can include analyzing an association between features of different modalities and analyzing a temporal association between features at different time steps.

In this way, the fused multimodal data is comprehensively analyzed by the deep learning model, thereby accurately extracting the features such as animal sounds, visual actions and physical sign changes to form the multimodal feature vector. The deep emotion analysis is performed on these features by the generative adversarial network to obtain the animal emotion recognition results. Such a process not only improves the emotion recognition accuracy and depth, but also enhances the interpretation of the animal behaviors and psychological states, providing a powerful technical support for achieving more effective human-animal communication.

Specifically, in some further embodiments, if no data synchronization and normalization are performed in the preprocessing stage, the collected data can be timestamped, cleaned, temporally aligned, and fused in the preprocessing stage to obtain the fused multimodal data meeting the above standardized format. Afterwards, sound feature extraction, visual action feature extraction and physical sign change analysis are performed, by the deep learning model, on the fused multimodal data to obtain the multimodal feature vectors, which can be particularly implemented as shown in FIG. 5.

At S501, sound feature extraction, visual action feature extraction and physical sign change analysis are performed, by a deep learning model, on the fused multimodal data to obtain a first audio feature, a first visual feature and a first physical sign feature.

That is, the fused multimodal data includes audio signals of an audio modality, video data of a video modality and physiological data of a physical sign modality. The feature extraction is performed on the audio signals to obtain a first audio feature, the feature extraction is performed on the video data to obtain a first visual feature, and the feature extraction is performed on the physiological data to obtain a first physical sign feature.

A module for performing the feature extraction on various modalities can use networks such as a CNN, a RNN and a MLP (Multi-Layer Perceptron), which is not limited in embodiments of the present disclosure.

During implementation, the standardized structure stores the fused multimodal data. After the data of the modalities at each timestamp is extracted from the fused multimodal data, respectively, feature extraction can be performed on the data of the modalities, respectively.

The temporal alignment merely refers to a macro alignment time. However, in order to compensate for the difference in sampling rates of data of different modalities, it is necessary to perform frame level fine-tuning through data synchronization, such that the frame rates of different modalities are the same, that is, such that different modalities can achieve the same sampling rate in a high-level feature space. For example, assuming the frame rate of the first audio feature of the audio is 16 frames per second, the frame rates of the first visual feature and the first physical sign feature need to be aligned to 16 frames per second.

At S502, data synchronization is performed on the first audio feature, the first visual feature and the first physical sign feature to obtain a second audio feature, a second visual feature and a second physical sign feature.

The data synchronization refers to alignment of the frame rates of different modalities. During implementation, the frame rate can be increased by upsampling or decreased by downsampling to align the sampling rates of different modalities.

When no normalization is performed in the preprocessing stage, in order to align signal ranges of different modalities and prevent a single modality from dominating subsequent modelling, normalization can be completed through S503 below.

At S503, the second audio feature, the second visual feature and the second physical sign feature are normalized to obtain the multimodal feature vectors.

The normalization may include Z-score normalization or Min-Max scaling.

During implementation, if the multimodal data is relatively simple, normalization can be completed in the preprocessing stage, that is, an original signal is normalized. However, completing the normalization in the preprocessing stage may lead to the loss of some critical information. Therefore, to preserve more features, the normalization can be performed in the high-level feature space through S503 after the data synchronization.

In embodiments of the present disclosure, independent features of each modality can be extracted separately, and then frame-level feature alignment can be achieved through data synchronization, which can better describe states and responses of the animals at the same time step and improve the emotion recognition accuracy. Finally, it is possible to make signal ranges of different modalities consistent through normalization, prevent the single modality from dominating the emotion recognition, and further improve the animal emotion recognition accuracy.

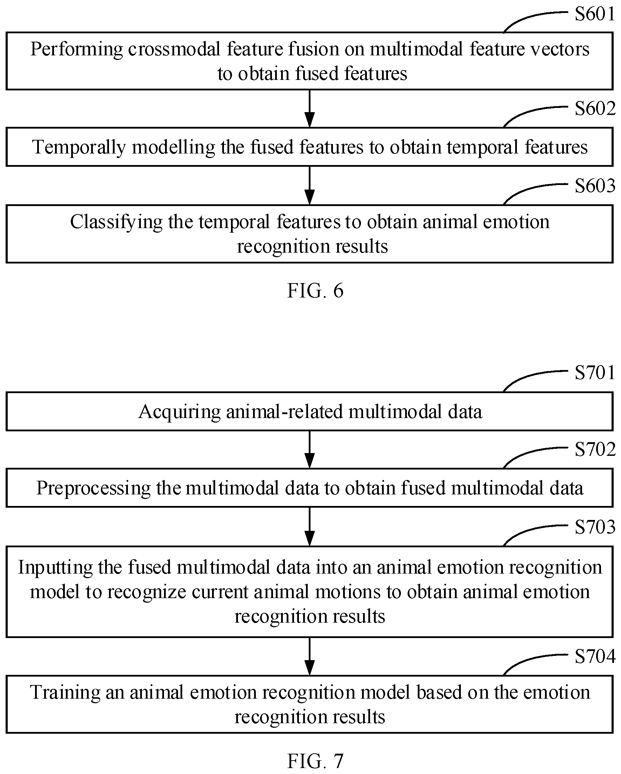

In some embodiments, the performing, by the generative adversarial network, emotion analysis on the multimodal feature to obtain the animal emotion recognition results can be implemented as including the following steps as shown in FIG. 6.

At S601, crossmodal feature fusion is performed on the multimodal feature vectors to obtain fused features.

The multimodal feature vectors include feature vectors of an audio modality, a video modality and a physical sign modality, such as the normalized second audio feature, the normalized second visual feature and the normalized second physical sign feature explained earlier.

It is possible to model an intrinsic association between different modalities by the feature fusion. It can be understood that each multimodal feature vector corresponds to a time step, and features of different modalities in the multimodal feature vectors at the same time step can be modelled by the feature fusion, and association patterns between different modalities at the same time step are mined.

For a better understanding of the animal emotions, in embodiments of the present disclosure, temporal correlations at different time steps are modelled temporally, as shown in step S602 below.

At S602, the fused features are temporally modelled to obtain temporal features.

The temporal features can be obtained by a neural network model processing temporal data.

At S603, the temporal features are classified to obtain the animal emotion recognition results.

In embodiments of the present disclosure, the association between the multimodal data at the same time step is modelled by the crossmodal feature fusion, and advanced features are mined. Afterwards, temporal features expressions of the animals are mined by temporal modelling, and further the temporal features for classification are generated. Finally, the animal emotions are recognized by means of the classification. The entire process can progressively improve the animal recognition accuracy.

In some embodiments, the deep learning model and the generative adversarial network are configured to construct an animal emotion recognition model. The animal emotion recognition model can be trained separately, or can be trained together with a pre-trained speech model in the S104. The emotion recognition model will be introduced below for its specific data processing flow and training method when the emotion recognition model is trained separately. Details will be omitted here. The deep learning model and the generative adversarial network are organically integrated into an animal emotion recognition model in embodiments of the present disclosure, which facilitates the end-to-end training and improves the animal emotion recognition accuracy.

In some embodiments, the present disclosure can dynamically adjust the frequency of obtaining the multimodal data. For example, if it is determined that a preset event is detected based on the emotion recognition results, the frequency of obtaining the multimodal data is increased. The preset event includes at least one of the following.

1). The emotion recognition results indicate that a first target emotion category is recognized and a confidence of the emotion recognition results is greater than a preset threshold.

A first target emotion category can be understood as emotions in high-risk states, such as anxiety, tension and an emotion for expressing hunger. During implementation, a list of emotions for the first target emotion category can be set as required. If the recognized emotion recognition results present in this list, the emotion recognition results indicate that the first target emotion category is recognized.

A confidence of the recognition is output together with the emotion recognition results to express the credibility of the emotion recognition results.

When the first target emotion category is recognized with high confidence, an increase in the frequency of obtaining the multimodal data, that is, an increase in the sampling rate of acquiring the multimodal data, is triggered.

2). The emotion recognition results indicate that a second target emotion category is recognized and continuously detected for n times, wherein n is a positive integer greater than 1.

A second target emotion category can be determined as required, which is not limited in embodiments of the present disclosure. For example, if an emotion for expressing "aggression" is continuously detected for n times or more, an increase in the sampling rate can be triggered.

3). Animal emotion variation values within a sliding time window to which the emotion recognition results belong is higher than a preset value.

During implementation, it is determined whether animal emotion variation values are greater than a preset value based on the emotion recognition results and further emotion recognition results within a sliding time window.

Emotion variations within the sliding window can be checked, and the animal emotion recognition results can be continuously monitored within the sliding window to obtain a plurality of emotion recognition results. The emotion variation values of the plurality of emotion recognition results within the sliding window are calculated.

During implementation, a statistical value of the classification probability distribution of the emotion recognition results within the sliding window can be calculated. The emotion recognition result is inherently a classification probability distribution in which a probability value belonging to each emotion is recorded. During implementation, each time step corresponds to an emotion recognition result, and a plurality of time steps are included within a sliding window. Therefore, the statistical values of the emotion recognition results at different time steps, such as variances, can be calculated as the emotion variation values.

The emotion variation value exceeding the preset value indicates severe animal emotion variation, which can improve the frequency of acquiring the animal-related multimodal data, for example, can improve the sampling rate of the multimodal data to facilitate real-time tracking of the animal emotion changes. During implementation, the original sampling rate is for example increased from m1 times/s to m2 times/s, wherein m2 is greater than m1.

In summary, in embodiments of the present disclosure, the frequency of obtaining the multimodal data can be appropriately increased through an event triggering mechanism to facilitate real-time observation of the animal emotion changes.

In some further embodiments, after the animal emotion states meet the preset event, the animal emotion changes can be continuously detected. With respect to the animals, the frequency of obtaining the multimodal data is reduced if the preset event remains undetected for a specified duration starting from the end of detection until the preset event begins.

For example, upon detecting that the animal emotions meet the preset event starting from a moment $t_s$, the sampling rate is increased. If the animal emotions do not meet the preset event any more until a moment $t_i$ and the preset event is not detected until a moment $t_i+T_s$, that is, the preset event is not detected for a duration of $T_s$, the sampling rate is reduced to a default frequency.

Thus, reducing the frequency can decrease the power consumption, reduce the number of detecting the animal emotion recognition results, and thus conserve computing and storage resources.

It can be known from the above explanation that in embodiments of the present disclosure, the animal language translation model includes an animal emotion recognition model. Based on the same inventive concept, embodiments of the present disclosure further provide a method for training an animal emotion recognition model separately. It should be noted that a processing flow of the animal emotion recognition model introduced in the method is equally applicable to a section for recognizing animal emotions in the animal language translation method, merely differing in the need for the adjustment of a model parameter in a training stage and the absence of the calculation of the training loss in an inference stage.

Specifically, the method for training the animal emotion recognition model includes the following steps as shown in FIG. 7.

At S701, animal-related multimodal data is acquired.

In embodiments of the present disclosure, the animals in the S701 can be the same as or different from those in the S101. The animal-related multimodal data in the training stage refers to a training sample for training the animal emotion recognition model.

At S702, the multimodal data is preprocessed to obtain fused multimodal data.

The preprocessing is the same as that explained earlier, which will be omitted here.

The fused multimodal data refers to data containing independently different modalities, enabling information transmitted by a single modality to be analyzed separately and association patterns between different modalities to be mined as well.

At S703, the fused multimodal data is input into the animal emotion recognition model to recognize current animal motions to obtain animal emotion recognition results.

At S704, the animal emotion recognition model is trained based on the emotion recognition results.

That is, in the training stage, the animal-related multimodal data as the training sample is preprocessed to obtain the fused multimodal data, in order to optimize the quality of the data and improve the accuracy of training the model. The animal emotion recognition results can be obtained by inferring and predicting the fused multimodal data, in order to train the model based on the emotion recognition results and improve the recognition accuracy of the animal emotion recognition model.

In some embodiments, the preprocessing the multimodal data to obtain the fused multimodal data includes: aggregating the multimodal data within the same timestamp range based on timestamps in the multimodal data to obtain the fused multimodal data. Further preprocessing operations have been explained earlier and will be omitted here. It should be emphasized herein that the fused multimodal data is obtained by aligning the data of different modalities with the same timestamp. Embodiments of the present disclosure can macroscopically align the multimodal data at the same time step through timestamp alignment, thereby improving the recognition accuracy of the animal emotion recognition model.

In some embodiments, as explained above, the animal emotion recognition model includes a deep learning model and a generative adversarial network. On this basis, the inputting the fused multimodal data into the animal emotion recognition model to recognize current animal motions to obtain animal emotion recognition results can be implemented as follows:

performing, by the deep learning model, sound feature extraction, visual action feature extraction and physical sign change analysis on the fused multimodal data to obtain multimodal feature vectors; and

performing, by the generative adversarial network, emotion analysis on the multimodal feature vectors to obtain the animal emotion recognition results.

The fused multimodal data is comprehensively analyzed by the deep learning model, thereby accurately extracting the features such as animal sounds, visual actions and physical sign changes to form the multimodal feature vector. Then, deep emotion analysis is performed on these features by the generative adversarial network to obtain the animal emotion recognition results. Such a process not only improves the emotion recognition accuracy and depth, but also enhances the interpretation of the animal behaviors and psychological states, thereby improving the recognition accuracy of the animal emotion recognition model.

In some embodiments, if data synchronization and normalization are not performed in the preprocessing stage, the collected data can be timestamped, cleaned, temporally aligned, and fused in the preprocessing stage to obtain the fused multimodal data meeting the above standardized format. Afterwards, continuously performing, by the deep learning model, sound feature extraction, visual action feature extraction and physical sign change analysis on the fused multimodal data to obtain the multimodal feature vectors can be particularly implemented as follows.

At a step A1, sound feature extraction, visual action feature extraction and physical sign change analysis are performed, by the deep learning model, on the fused multimodal data to obtain a first audio feature, a first visual feature and a first physical sign feature.

That is, the fused multimodal data includes audio signals of an audio modality, video data of a video modality and physiological data of a physical sign modality. The feature extraction is performed on the audio signals to obtain a first audio feature, the feature extraction is performed on the video data to obtain a first visual feature, and the feature extraction is performed on the physiological data to obtain a first physical sign feature.

During implementation, the deep learning model may include an audio feature extraction module, a video feature extraction module and a physical sign feature extraction module. Each module can use a network such as a CNN, a RNN and a MLP, which is not limited in embodiments of the present disclosure.

Correspondingly, the performing, by the deep learning model, sound feature extraction, visual action feature extraction and physical sign change analysis on the fused multimodal data to obtain a first audio feature, a first visual feature and a first physical sign feature can be implemented as follows:

step A11, performing, based on the audio feature extraction module in the deep learning model, feature extraction on audio signals in the fused multimodal data to obtain the first audio feature;
step A12, performing, based on the video feature extraction module in the deep learning model, feature extraction on video data in the fused multimodal data to obtain the first visual feature; and
step A13, performing, based on the physical sign feature extraction module in the deep learning model, feature extraction on physiological data in the fused multimodal data to obtain the first physical sign feature.

During implementation, the training sample is stored in the standardized structure storing the fused multimodal data. Feature extraction can be performed on the data, which is extracted from the fused multimodal data, respectively, of the modalities at the timestamps, respectively.

The temporal alignment merely refers to a macro alignment time. However, in order to compensate for the difference in sampling rates of data of different modalities, it is necessary to make the frame rates of different modalities the same through frame-level fine tuning. For example, assuming the frame rate of the first audio feature of the audio is 16 frames per second, the frame rates of the first visual feature and the first physical sign feature need to be aligned to 16 frames per second, such that the sampling rates for different modalities are aligned.

In embodiments of the present disclosure, features of different modalities are respectively extracted by different modules to facilitate feature modelling of the individual modalities. Important features of the individual modalities for emotion recognition are extracted to improve the animal emotion recognition accuracy.

At a step A2, data synchronization is performed on the first audio feature, the first visual feature and the first physical sign feature to obtain a second audio feature, a second visual feature and a second physical sign feature.

The data synchronization refers to alignment of the sampling rates of different modalities. During implementation, the frame rate can be increased by upsampling or decreased by downsampling to align the sampling rates for different modalities.

During implementation, the first visual feature of the image modality can be downsampled by a pooling operation or upsampled by an upsampling module of the neural network model. If the data synchronization is performed on the first audio feature of the audio modality, reference can be made to a processing approach of the visual modality.

The first physical sign feature of the physiological modality can refer to the processing approach of the visual modality. In addition, the data synchronization can be achieved by a traditional data interpolation algorithm or downsampling. For example, a time window is constructed with a step size of 1s, and one time window can include a plurality of time steps. Statistical values such as mean, variance, maximum and minimum of physical sign data are extracted within each time step as the first physical sign feature, and then extended into a feature sequence containing a plurality of data frames through the data synchronization. Each data frame can contain multidimensional statistics (such as a heart rate mean and a variation coefficient).

In summary, the performing data synchronization on the first audio feature, the first visual feature and the first physical sign feature to obtain a second audio feature, a second visual feature and a second physical sign feature can be systematically implemented as follows: processing frame counts of the first audio feature, the first visual feature and the first physical sign feature within the current time window as target frame counts, respectively, to obtain the second audio feature, the second visual feature and the second physical sign feature.

For example, the second audio feature, the second visual feature and the second physical sign feature can be obtained by processing the frame count of the first audio feature within the current time window as the target frame count and processing the frame counts of the first visual feature and the first physical sign feature within the current time window as the target frame counts, respectively, wherein the first audio feature serves as the second audio feature.

In embodiments of the present disclosure, frame rates or sampling rates of different modalities are aligned through data synchronization to achieve fine-tuned frame-level alignment within the same time window, thereby improving the animal emotion recognition accuracy.

When no normalization is performed in the preprocessing stage, in order to align signal ranges of different modalities and prevent a single modality from dominating subsequent modelling, the normalization can be completed through a step A3 below.

At a step A3, the second audio feature, the second visual feature and the second physical sign feature are normalized to obtain the multimodal feature vectors.

The normalization may include Z-score normalization or Min-Max scaling.

Certainly, if normalization has already been completed in the previous preprocessing, the step A3 can be omitted.

In embodiments of the present disclosure, independent features of each modality can be extracted separately, and then frame-level feature alignment can be achieved through the data synchronization, which can better describe animal states and responses at the same time step and improve the emotion recognition accuracy. Finally, it is possible to make signal ranges of different modalities consistent through normalization, prevent the single modality from dominating emotion recognition, and further improve the animal emotion recognition accuracy.

In some embodiments, each multimodal feature vector is a feature representation of a plurality of modalities at the same time step. During implementation, it is possible to model the features at the plurality of time steps within the same time window to better classify the animal emotion recognition results. On this basis, the performing, by the generative adversarial network, emotion analysis on the multimodal feature to obtain the animal emotion recognition results can be implemented as follows.

At a step B1, crossmodal feature fusion is performed on the multimodal feature vectors to obtain fused features.

The multimodal feature vectors include feature vectors of an audio modality, a video modality and a physical sign modality. For example, the normalized second audio feature, the normalized second visual feature and the normalized second physical sign feature explained earlier.

It is possible to model an intrinsic association between different modalities by the feature fusion. It can be understood that each multimodal feature vector corresponds to a time step, and features of different modalities in the multimodal feature vectors at the same time can be modelled by the feature fusion, and correlation patterns between different modalities at the same time step are mined.

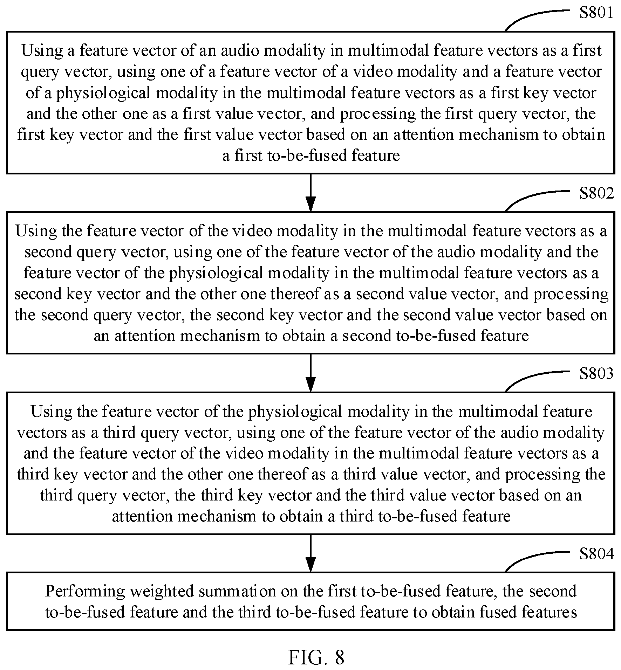

During implementation, obtaining the fused features at the same time step through a mid-level attention network can be implemented as shown in FIG. 8.

At S801, a feature vector of an audio modality in the multimodal feature vectors is used as a first query vector, one of a feature vector of a video modality and a feature vector of a physiological modality in the multimodal feature vectors is used as a first key vector and the other one is used as a first value vector, and the first query vector, the first key vector and the first value vector are processed based on an attention mechanism to obtain a first to-be-fused feature.

During implementation, if the normalization has already been completed in the preprocessing stage, the first audio feature can be used as the feature vector of the audio modality, the first visual feature can be used as the feature vector of the video modality, and the first physical sign feature can be used as the feature vector of the physiological modality.

If the normalization has not been performed in the preprocessing stage, the normalized second audio feature can be used as the feature vector of the audio modality, the normalized second visual feature can be used as the feature vector of the video modality, and the normalized second physical sign feature can be used as the feature vector of the physiological modality.

In order to further eliminate differences between feature spaces of different modalities and/or dimensional differences between the features of different modalities during implementation, in embodiments of the present disclosure, the feature vectors of the modalities can be aligned to dimensions of the features through a modality transformer, and feature representations of different modalities can be mapped to the same feature space to obtain the final used feature vectors: the feature vector of the audio modality, the feature vector of the video modality and the feature vector of the physiological modality.

For example, if the normalization has not been performed in the preprocessing stage, the normalized second audio feature is transformed into the feature vector of the audio modality by an audio modality transformer. Similarly, the normalized second visual feature is transformed into the feature vector of the video modality by a video modality transformer. Similarly, the normalized second physical sign feature is transformed into the feature vector of the physiological modality by a physical sign modality transformer.

Certainly, if the normalization has already been completed in the preprocessing stage, the first audio feature is transformed into the feature vector of the audio modality by the audio modality transformer. Similarly, the first visual feature is transformed into the feature vector of the video modality by the video modality transformer. Similarly, the first physical sign feature is transformed into the feature vector of the physiological modality by the physical sign modality transformer.

During implementation, the audio modality transformer, the video modality transformer and the physical sign modality transformer can be implemented by any one or a combination of at least one fully-connected layer, a small CNN and a small RNN, respectively, and function to project the features of different modalities onto a unified dimension and feature space. Each modality transformer requires not only alignment of dimensions, but also ensures that feature distributions are comparable.

One of the feature vector of the video modality and the feature vector of the physiological modality can be used as the first key vector and the other one thereof can be used as the first value vector, which is not limited in embodiments of the present disclosure.

At S802, the feature vector of the video modality in the multimodal feature vectors is used as a second query vector, one of the feature vector of the audio modality and the feature vector of the physiological modality in the multimodal feature vectors is used as a second key vector and the other one thereof is used as a second value vector, and the second query vector, the second key vector and the second value vector are processed based on an attention mechanism to obtain a second to-be-fused feature.

Similarly, one of the feature vector of the audio modality and the feature vector of the physiological modality can be used as the second key vector and the other one thereof can be used as the second value vector, which is not limited in embodiments of the present disclosure.

At S803, the feature vector of the physiological modality in the multimodal feature vectors is used as a third query vector, one of the feature vector of the audio modality and the feature vector of the video modality in the multimodal feature vectors is used as a third key vector and the other one thereof is used as a third value vector, and the third query vector, the third key vector and the third value vector are processed based on an attention mechanism to obtain a third to-be-fused feature.

Similarly, one of the feature vector of the audio modality and the feature vector of the video modality can be used as the third key vector and the other one thereof can be used as the third value vector, which is not limited in embodiments of the present disclosure.

During implementation, in the steps S801 through S803, the corresponding to-be-fused feature can be constructed by the corresponding cross attention module in each step.

At S804, weighted summation is performed on the first to-be-fused feature, the second to-be-fused feature and the third to-be-fused feature to obtain the fused features.

Thus, feature vectors of different modalities serve as query vectors to be fused with feature vectors of further modalities, ultimately obtaining fused features at the same timestep through weighted summation. Dynamic interaction of information between different modalities can be achieved by a crossmodal attention mechanism, improving the fusion effect. The fusion process imitates the extension of a transformer self-attention mechanism in a multimodal scene, such that the fused features can model association models between different modalities at the same time step, and the obtained fused features can be better expressed as critical features beneficial for emotion classification, thereby improving the animal emotion classification effectiveness.

In some embodiments, fixed weights can be set to perform weighted summation on the first to-be-fused feature, the second to-be-fused feature and the third to-be-fused feature.

In some further embodiments, in order to improve the feature expression capability of the fused features, weights can be dynamically generated to perform weighted summation on a plurality of to-be-fused features, which can be implemented as: with respect to any one of the first to-be-fused feature, the second to-be-fused feature and the third to-be-fused feature, a weight of any one of the to-be-fused features is dynamically generated based on any one of the to-be-fused features and a learnable parameter of a training stage. Any one of the to-be-fused features refers to each of the first to-be-fused feature, the second to-be-fused feature and the third to-be-fused feature. The weight of each to-be-fused feature is dynamically generated for the feature fusion.

For example, any of the to-be-fused features is input to the corresponding neural network layer with a learnable parameter. After the to-be-fused feature is processed by the neural network layer, the weight corresponding to the to-be-fused feature is output. The neural network layer can be for example a gated network.

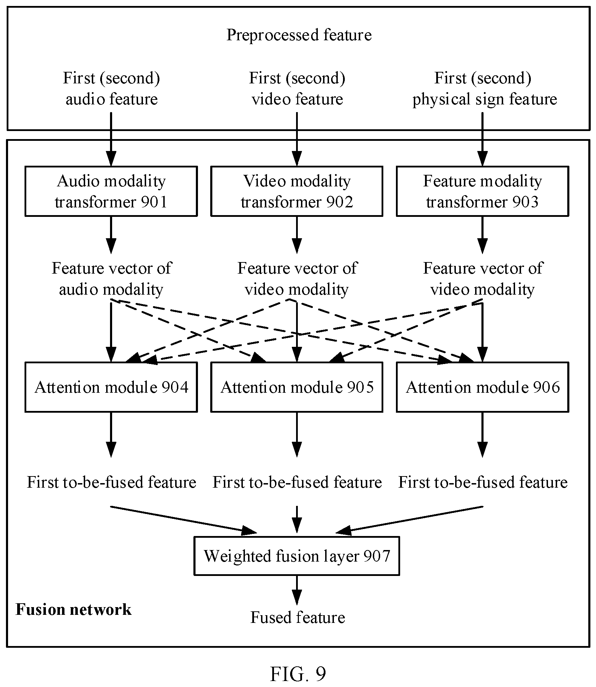

In one example, as shown in FIG. 9, the fusion network for fusing multimodal features at the same time step includes an audio modality transformer 901, a video modality transformer 902 and a feature modality transformer 903. The preprocessed features (such as the first audio feature, the first video feature and the first physical sign feature, or the normalized second audio feature, the normalized second visual feature and the normalized second physical sign feature) obtained from the corresponding modality by the preprocessing are input into the corresponding modality transformers to achieve dimension alignment and feature space alignment, thereby obtaining the feature vectors of the audio modality, the video modality and the physiological modality. Then, taking three sets of attention modules as examples, in the attention module 904, the feature vector of the audio modality serves as the first query vector while the feature vectors of the other two modalities serve as the first key vector and the first value vector, respectively; in the attention module 905, the feature vector of the video modality serves as the second query vector while the feature vectors of the other two modalities serve as the second key vector and the second value vector, respectively; and in the attention module 906, the feature vector of the feature modality serves as the third query vector while the feature vectors of the other two modalities serve as the third key vector and the third value vector, respectively. The attention module 904 outputs the first to-be-fused feature, the attention module 905 outputs the second to-be-fused feature, and the attention module 906 outputs the third to-be-fused feature.

A processing procedure of each attention module can be represented by the following formula (1):

$$\text{Attention}(Q_i, K_j, V_j) = \text{Softmax}\left(\frac{Q_i K_j^T}{\sqrt{d}}\right) V_j \quad (1)$$

wherein $Q_i$ represents the input Query vector (i.e., the corresponding query vector), $K_j$ represents the input Key vector, i.e., the key vector, $V_j$ represents the input Value vector, i.e., the value vector, d represents a dimension of the Key vector for scaling to prevent excessive gradients, and Softmax(.) is used to control an attention distribution to learn a critical effective feature by the attention mechanism.

A process of performing weighted summation on the three to-be-fused features can be completed by a weighted fusion layer 907 in FIG. 9. A processing procedure of the weighted fusion layer can be described as generating a dynamic weight based on the following formula (2) and completing the weighted summation based on the following formula (3):

$$\alpha_i = \frac{\exp\left(W_i \cdot X_i\right)}{\sum_j \exp\left(W_j \cdot X_j\right)} \quad (2)$$

wherein $\alpha_i$ is a normalized weighted weight of an $i^{th}$ modality, $W_i$ represents a learnable parameter of the $i^{th}$ modality in the training stage when the weighted summation is performed on a plurality of to-be-fused features, $X_i$ represents a to-be-fused feature of the $i^{th}$ modality or a feature vector of the $i^{th}$ modality, and a denominator is used to normalize fused weights of a plurality of modalities. Therefore, $W_j$ represents a learnable parameter of a $j^{th}$ modality in the training stage when the weighted summation is performed on the plurality of to-be-fused features, and $X_j$ represents a to-be-fused feature of the $j^{th}$ modality or a feature vector of the $j^{th}$ modality.

Determining the fused weights of the plurality of to-be-fused features in such a way can ensure that the sum of the weights of all the modalities is 1 by means of the normalization, making the feature fusion dynamically adaptive.

The weights of the to-be-fused features can be subjected to weighted summation based on the following formula (3) after being dynamically determined based on the formula (2):

$$F_t = \sum_{i=1}^{M} \alpha_i \cdot X_i \quad (3)$$

wherein $F_t$ represents the fused feature at a time step t, M is the total number of modalities and $\alpha_i$ represents the normalized weighted weight of the $i^{th}$ modality, and $X_i$ represents an $i^{th}$ to-be-fused feature.

Accordingly, dynamically determining the weights of the plurality of to-be-fused features at the same time step can enhance the contribution of important modalities to emotions and improve the animal emotion recognition accuracy.

During implementation, the learnable parameter of the weight for performing the weighted summation on the plurality of to-be-fused features in the training stage is optimized as the training progresses. In the inference stage, the learnable parameter can be fixed and a fused weight corresponding to the learnable parameter is mainly determined by the feature vector of the corresponding modality or the to-be-fused feature. As such, in the inference stage, the modalities are adaptively implemented to determine a weight of weighted fusion.

For an accurate understanding of the animal emotions, in embodiments of the present disclosure, temporal associations of different time steps are temporally modelled, as shown in the following step B2.

At a step B2, the fused features are temporally modelled to obtain temporal features.

The temporally modelling the fused features can be implemented as follows.

At a step C1, feature encoding is performed on a plurality of fused features at a plurality of time steps within a current time window to obtain a plurality of enhanced features at the plurality of time steps within the current time window.

Each time step corresponds to a respective fused feature, respectively. During implementation, feature transformation is performed on the plurality of fused features at the plurality of time steps respectively to obtain the plurality of enhanced features, wherein a feature dimension of the enhanced features is smaller than that of the fused features. That is, feature transformation is performed on the fused features corresponding to the plurality of time steps within the same time window (i.e., the current time window), respectively, to obtain the enhanced features corresponding to the individual time steps. The purpose of the feature transformation is to compress dimensions, improve the emotion inference speed and enhance the feature expression capability.

During implementation, feature compression and nonlinear transformation can be firstly performed on the fused features by an encoder to achieve the feature transformation and achieve the purpose of enhancing the feature expression capability. The encoder processes the fused features at the individual time steps, respectively, to obtain the enhanced features corresponding to the individual time steps, thereby obtaining the plurality of enhanced features within the same time window.

For example, the encoder may include an activation layer (ReLU), a layer normalization layer (LayerNorm) and a dropout layer for feature transformation. A processing procedure of the encoder can be as shown in the following formula (4):

$$Z_t = \text{Dropout}\left(\text{LayerNorm}\left(\text{ReLU}(W_1 \cdot F_t + b_1)\right)\right) \quad (4)$$

wherein $W_1$ represents a weight matrix of the ReLU, $b_1$ represents a bias of the ReLU, and $F_t$ represents fused features at the time step t. $Z_t$ represents the enhanced features obtained after feature transformation is performed by the encoder at the time step t.

The nonlinearity can be improved by the activation layer, the convergence can be accelerated by the layer normalization layer to improve the model training efficiency, and random dropout can be achieved the dropout layer to prevent overfitting.

In addition, except for an encoder structure expressed in the formula (4) above, a convolutional network (such as a 1D Conv), a transformer encoder, a residual fully-connected network and the like can be used to construct the encoder. Flexible selection of visual data types and temporal characteristics can be carried out during implementation.

At a step C2, the plurality of enhanced features at the plurality of time steps are temporally modelled to obtain a plurality of intermediate features within the current time window.

During implementation, a temporal evolution rule of emotions is modelled by a LSTM (Long Short-Term Memory). The enhanced features at each time step are processed by for example a BiLSTM (Bidirectional Long Short-Term Memory) to obtain intermediate features corresponding to the individual time steps respectively, thereby obtaining the plurality of intermediate features within the current time window.

In addition, in order to further improve the feature expression capability, in embodiments of the present disclosure, the enhanced features at the individual time steps are processed by the BiLSTM to obtain initial time features corresponding to the individual time steps output by the BiLSTM. The initial time features corresponding to the individual time steps are processed by a self-attention mechanism module to obtain the intermediate features corresponding to the individual time steps, respectively. In the self-attention mechanism module, Q (Query), K (Key) and V (Value) all result from the output of the BiLSTM. Generating a temporal context state at each time step by the BiLSTM can be represented as shown in the following formula (5):

$$h_t = BiLSTM(Z_t) \quad (5),$$

wherein $Z_t$ represents the enhanced features at the time step t, and $h_t$ represents the intermediate features at the time step t output by the BiLSTM, i.e., the context state at the time step t.

As such, a temporal evolution relationship of the animal emotions can be modelled by temporal modelling, in order to improve the animal emotion recognition accuracy.

At a step C3, weighted summation is performed on the plurality of intermediate features within the current time window to obtain the temporal features.

In embodiments of the present disclosure, the expression capability of the fused features at each time step is enhanced by feature encoding, and the plurality of time steps within the same time window are temporally modelled, such that temporal features can reflect temporal context associations, thereby improving the animal emotion recognition accuracy. In addition, the intermediate features at different time steps are fused in a weighted fusion manner to obtain the temporal features, such that the contribution of the features at different time steps to the final temporal features is adjusted, thereby mining feature expressions at important time steps and improving the animal emotion recognition accuracy.

In some embodiments, weighted summation can be performed on the plurality of intermediate features at the plurality of time steps within the same time window by adopting fixed weights to obtain the temporal features.

In order to improve the expression capability of the temporal features, the intermediate features at different time steps are fused by dynamic weights in embodiments of the present disclosure, which can be implemented as follows.

At a step D1, the plurality of intermediate features within the current time window are processed based on a weight generation network to obtain a plurality of weights corresponding to the plurality of time steps within the current time window.

That is, the intermediate features at the individual time steps can be input into the weight generation network, respectively, to obtain weights at the individual time steps. During implementation, the weight generation network can be a softmax network or a gated network, which is not limited in the present disclosure.

Taking the softmax network as an example, generating a weight at the time step t is as shown in the following formula (6):

$$\alpha_t = Softmax(W_\alpha \cdot h_t + b_\alpha) \quad (6),$$

wherein $\alpha_t$ represents the weight at the time step t, $W_\alpha$ represents a weight coefficient of the softmax network, $b_\alpha$ represents a bias of the softmax network, and $h_t$ represents an intermediate feature at the time step t.

During implementation, the final weight at each time step can be a normalized value.

At a step D2, weighted summation is performed on the plurality of intermediate features at the plurality of time steps within the current time window based on the plurality of weights to obtain the temporal features.

The obtained temporal features can be expressed as shown in the following formula (7):

$$h_{final} = \sum \alpha_t \cdot h_t \quad (7),$$

wherein $h_{final}$ represents the temporal features ultimately modelling a global context within the current time window, $\alpha_t$ represents a weight at the time step t, and $h_t$ represents an intermediate feature at the time step t.

In embodiments of the present disclosure, the global context within the same time window is temporally modelled to obtain the temporal features describing global features. Moreover, the contribution of different time steps is emphasized in the temporal features, thereby highlighting superior features for animal emotion recognition, suppressing secondary features, improving the classification ability of the resulting temporal features and enhancing the emotion recognition accuracy.

At a step B3, the temporal features are classified to obtain the animal emotion recognition results.

The classification is performed by a classification module, a network structure of the classification module can be built according to actual needs, which is not limited in embodiments of the present disclosure.

In embodiments of the present disclosure, associations between multimodal data at the same time step are modelled by crossmodal feature fusion, and advanced features are mined. Afterwards, temporal features expressions of the animals are mined by temporal modelling, and then temporal features for the classification are generated. Finally, the animal emotions are recognized by the classification. The entire process can progressively improve the animal recognition accuracy.

In embodiments of the present disclosure, the classification loss can be calculated based on the emotion recognition results and corresponding training labels to optimize the model parameter of the animal emotion recognition model.

In some embodiments, in order to improve the robustness of the animal emotion recognition model, the animal emotion recognition model can be trained by adversarial training. Adversarial training, which involves allowing the animal emotion recognition model to process noisy inputs in a way of adding disturbances, optimizes the model parameter, reduces the sensitivity of the animal emotion recognition model to noise, and makes technologically dirty data have no impacts on the recognition results of the animal emotion recognition model.

During implementation, as shown in FIG, 10, the training the animal emotion recognition model through adversarial training includes the following steps.

At S1001, disturbances are added to the fused features corresponding to the multimodal feature vectors at a current time step to generate fused features of multimodal noisy features.

During implementation, it is necessary to add the disturbances based on a disturbance intensity. The disturbance intensity can be randomly generated.

In embodiments of the present disclosure, in order to add the disturbances towards a direction of maximum loss and increase the training pressure of the animal emotion recognition model on a boundary sample, the disturbance intensity can be dynamically generated to add the disturbances to the fused features of the multimodal feature vectors, which can be implemented as follows.

At S10011, a first statistical value in a first classification probability distribution of a processing result at a previous time step relative to the current time step is obtained, wherein the processing result includes an emotion classification result obtained by performing emotion classification on the fused features at the previous time step.

That is, the fused features corresponding to the multimodal feature vectors at the previous time step are obtained, and subjected to emotion classification to obtain a first classification probability distribution. An emotion classification result is one first classification probability distribution expressing probability values of different animal emotion categories.

The first statistical value can be a maximum value in the first classification probability distribution or a difference between the maximum value and the minimum value.

At S10012, a first disturbance intensity is generated based on a negative correlation between an intensity and the first statistical value.

The negative correlation can be a linear relationship or a nonlinear relationship, which can be determined according to actual needs, which is not limited in embodiments of the present disclosure.

For example, in one possible implementation, a manner of determining the first disturbance intensity is as shown in the following formula (8):

$$\varepsilon = \varepsilon_0 \cdot \left(1 - p_{conf}\right)$$
$$p_{conf} = \max\left(Softmax\left(f(F_{t-1})\right)\right) \quad (8)$$

wherein $\varepsilon$ represents the first disturbance intensity, $\varepsilon_0$ represents a reference value of the disturbance intensity, $F_{t-1}$ represents the fused feature at the previous time step, i.e., a time step t-1. $Softmax(f(F_{t-1}))$ represents classification on the input for animal emotion classification to obtain the first classification probability distribution, and $\max(Softmax())$ represents the maximum value output by $Softmax()$ as the confidence $p_{conf}$.

At S10013, the fused features of the multimodal noisy features are generated based on the first disturbance intensity, the fused features corresponding to the multimodal feature vectors and a classification loss gradient of the fused features corresponding to the multimodal feature vectors.

The multimodal noisy features $F_t^{'}$ are input into the animal emotion recognition model, a process of performing the feature fusion on the multimodal data by the animal emotion recognition model is omitted, and the fused features of the obtained multimodal noisy features are considered as the fused features for subsequent temporal modelling and ultimately for animal emotion classification.

A process of ultimately obtaining the fused features $F_t^{'}$ of the multimodal noisy features can be as shown in the following formula (9):

$$F_t^{'} = F_t + \varepsilon \cdot \mathrm{sign}\left(\nabla_{F_t} L_{cls}(F_t, y)\right) \quad (9),$$

wherein $F_t^{'}$ represents the fused features of the multimodal noisy features at the time step t, $F_t$ represents the fused features of the multimodal feature vectors at the current time step, $L_{cls}(F_t, y)$ represents a classification loss for performing emotion classification on the fused features, which can be referred to as a cross-entropy loss, Y is a training label, and $\nabla_{F_t}$ represents calculation for a gradient of the classification loss of the fused features at the time step t.

Accordingly, an approach of generating the first disturbance intensity in such a way can reduce the disturbance of a high-confidence sample, improve a training pressure of the animal emotion recognition model on a boundary sample, enhance the robustness of the model, and improve the animal emotion recognition accuracy.

At S1002, the fused features of the multimodal noisy features are input into the animal emotion recognition model to recognize current animal motions to obtain animal emotion prediction results.

It can be understood that the fused features of the multimodal noisy features are regarded as Ft participating in the subsequent processing of the animal emotion recognition model. That is, the process of performing the feature fusion on the multimodal feature by the fusion network in the animal emotion recognition model is omitted, the fused features of the multimodal noisy features are continuously processed by using the module of the animal emotion recognition model after the fusion network, and further animal emotions are classified to obtain the animal emotion prediction results.

Here, it is necessary to distinguish different concepts. In the training stage, the emotion recognition results refer to animal emotions recognized based on the fused features of the multimodal feature vectors before the disturbances are added, and the emotion prediction results refer to animal emotions recognized based on the fused features of the multimodal noisy features after the disturbances are added. The emotion recognition results and the emotion prediction results are differentiated yet associated.

In embodiments of the present disclosure, the disturbances are added to the fused features at the time step t, which can enhance the robustness of the animal emotion recognition model through adversarial training.

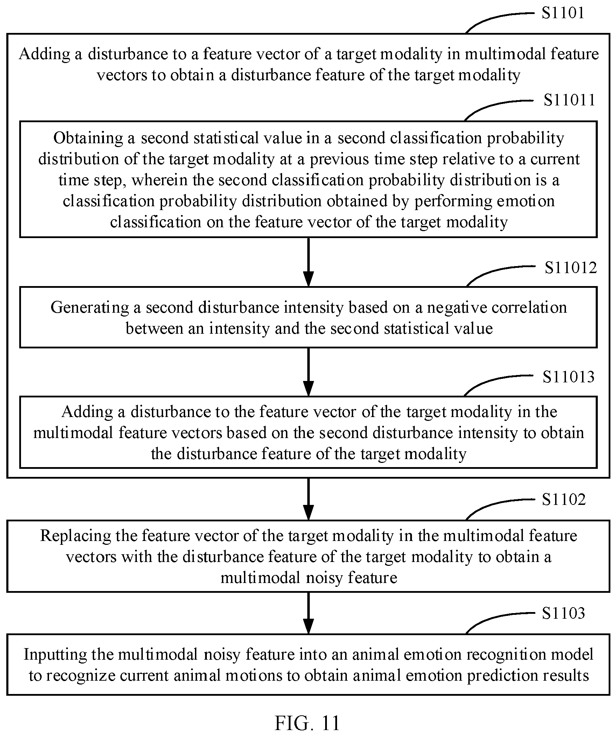

In further embodiments, in order to avoid introducing the disturbances too early to cause the model to become unstable during training, the animal emotion recognition model is trained by adversarial training, which can be implemented as including the following steps as shown in FIG. 11.

At S1101, a disturbance is added to a feature vector of a target modality in the multimodal feature vectors to obtain a disturbance feature of the target modality.

Types and numbers of modalities used in different training stages may vary. For example, in the initial stage, the disturbance is added to the video modality selected as the target modality. As the training progresses, the recognition accuracy and robustness of the animal emotion recognition model are improved, and audio and/or physiological modalities can be gradually added as target modalities.

The feature vectors of the video modality, the audio modality and the physiological modality in the multimodal feature vectors can all be used as the feature vector of the target modality, respectively.

When a plurality of target modalities exist, the disturbance is added to each target modality separately, and processing approaches for adding disturbances to different target modalities are consistent.

During implementation, taking one target modality as example, it is necessary to add the disturbance based on the disturbance intensity. The disturbance intensity can be randomly generated.

In embodiments of the present disclosure, in order to add the disturbance towards the direction of maximum loss and maximize the robustness of the model, the disturbance intensity depends on a classification confidence of the target modality at the previous time step. Moreover, the higher the classification confidence within the previous time window, the smaller the disturbance density. Specifically, adaptively determining the disturbance intensity to generate a disturbance feature can be implemented as shown in FIG. 11.

At S11011, a second statistical value in a second classification probability distribution of the target modality at a previous time step relative to a current time step is obtained, wherein the second classification probability distribution is a classification probability distribution obtained by performing emotion classification on the feature vector of the target modality.

During implementation, animal emotions can be classified based on the feature vector of the target modality at the previous time step to obtain an emotion classification result. The emotion classification result is a second classification probability distribution expressing probability values of different emotion categories. The second statistical value can be a maximum value in the second classification probability distribution or a difference between the maximum value and the minimum value.

At S11012, a second disturbance intensity is generated based on a negative correlation between an intensity and the second statistical value.

The negative correlation can be a linear relationship or a nonlinear relationship, which can be determined according to actual needs, which is not limited in embodiments of the present disclosure.

For example, in one possible implementation, an approach of determining the second disturbance intensity is as shown in the following formula (10):

$$\varepsilon_1 = \varepsilon_0 \cdot \left(1 - p_{conf}\right)$$
$$p_{conf} = \max\left(Softmax\left(f\left(\check{F}_{t-1}\right)\right)\right) \quad (10)$$

wherein $\varepsilon_1$ represents the second disturbance intensity, $\varepsilon_0$ represents a reference value of the disturbance intensity, and $\check{F}_{t-1}$ represents the classification feature of the feature vector of the target modality at the previous time step, i.e., a time step t-1. $Softmax\left(f(\check{F}_{t-1})\right)$ represents classification on the input $\check{F}_{t-1}$ for animal emotion classification to obtain the second classification probability distribution, and $\max(Softmax())$ represents the maximum value output by $Softmax()$ as the confidence $p_{conf}$.

The feature vector of the target modality (such as the feature vector of the audio modality) can inherently serve as its own classification feature. The feature vector of the target modality can be extracted by a classification model to obtain the classification feature. The classification feature is used to perform classification prediction on the animal emotion categories based on the target modality. The classification model can be determined according to actual needs, and the animal emotions can be roughly classified by an existing classification model according to a single target modality.

At S11013, a disturbance is added to the feature vector of the target modality in the multimodal feature vectors based on the second disturbance intensity to obtain the disturbance feature of the target modality.

During implementation, a process of ultimately obtaining the disturbance feature $F_t^{''}$ of the target modality based on the second disturbance intensity can be as shown in the following formula (11):

$$F_t^{''} = \check{F}_t + \varepsilon_1 \cdot \text{sign}\left(\nabla_{\check{F}_t} L_{cls}(\check{F}_t, y)\right) \quad (11)$$

wherein $F_t^{''}$ represents the disturbance feature of the target modality at the time step t, $\check{F}_t$ represents the feature vector of the target modality at the current time step, $L_{cls}(\check{F}_t, y)$ represents a classification loss for performing animal emotion classification on the classification feature of the feature vector of the target modality at the previous time step, which can be referred to as a cross-entropy loss, y is a training label, and $\nabla_{\check{F}_t}$ represents calculation for a gradient of the classification loss of the fused feature at the time step t.

By reasonably generating the second disturbance intensity and adding the disturbance to the corresponding feature vector of the target modality, the disturbances can be introduced to feature vectors of different modalities at appropriate stages to improve the training efficiency and the robustness of the animal emotion recognition model.

At S1102, the feature vector of the target modality in the multimodal feature vectors is replaced with the disturbance feature of the target modality to obtain a multimodal noisy feature.

That is, the multimodal feature vectors include feature vectors of an audio modality, a video modality and a physiological modality. Assuming that the video modality is the target modality, the feature vector of the audio modality and the feature vector of the physiological modality remain unchanged. The multimodal noisy feature can be obtained by replacing the feature vector of the video modality with the disturbance feature of the video modality.

Assuming that the video modality and the audio modality are the target modalities, the feature vector of the physiological modality remains unchanged. The multimodal noisy feature can be obtained by replacing the feature vector of the video modality with the disturbance feature of the video modality and the feature vector of the audio modality with the disturbance feature of the audio modality.

At S1103, the multimodal noisy feature is input into the animal emotion recognition model to recognize current animal motions to obtain animal emotion prediction results.

That is, the multimodal noisy feature is input into the animal emotion recognition model so as to complete the entire processing flow, which includes multimodal feature fusion and subsequent temporal modelling and classification, of the animal emotion recognition model, ultimately obtaining the emotion prediction result of the multimodal noisy feature.

It should be understood that it is still necessary to distinguish different concepts in such embodiments. In the training stage, emotion recognition results refer to animal emotions recognized based on the multimodal feature vectors before the disturbance is added. The emotion prediction results refer to animal emotions recognized based on the multimodal noisy feature after the disturbance is added. The emotion recognition results and the emotion prediction results are differentiated yet associated.

In embodiments of the present disclosure, the disturbance is added to the feature vector of the target modality, which can enhance the robustness of the animal emotion recognition model through adversarial training.

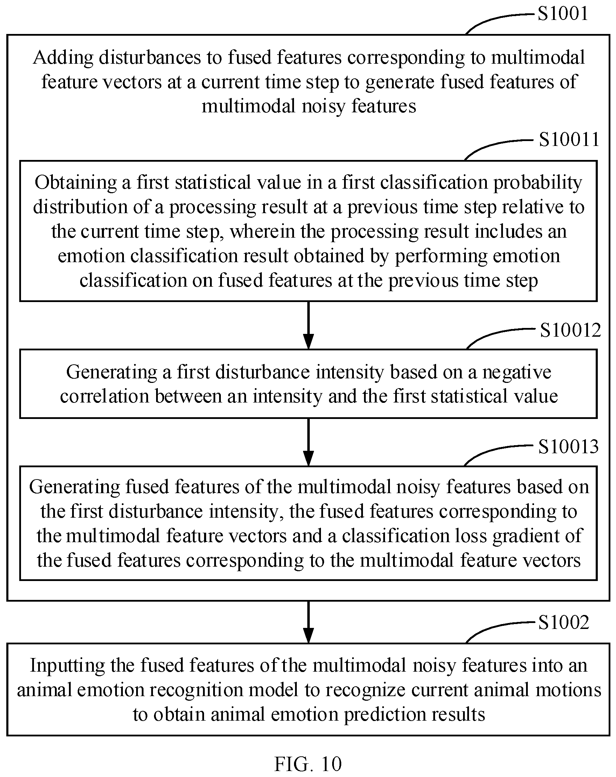

During implementation, the disturbance can be added separately to the feature vector of the video modality in the initial stage to complete the adversarial training. If the stability of the animal emotion recognition model meets a preset requirement, the adversarial training can be performed on feature vectors of further modalities. Afterwards, the disturbance can be continuously added to the fused feature of the multimodal feature vectors based on an approach as shown in FIG. 10 to continue the adversarial training.

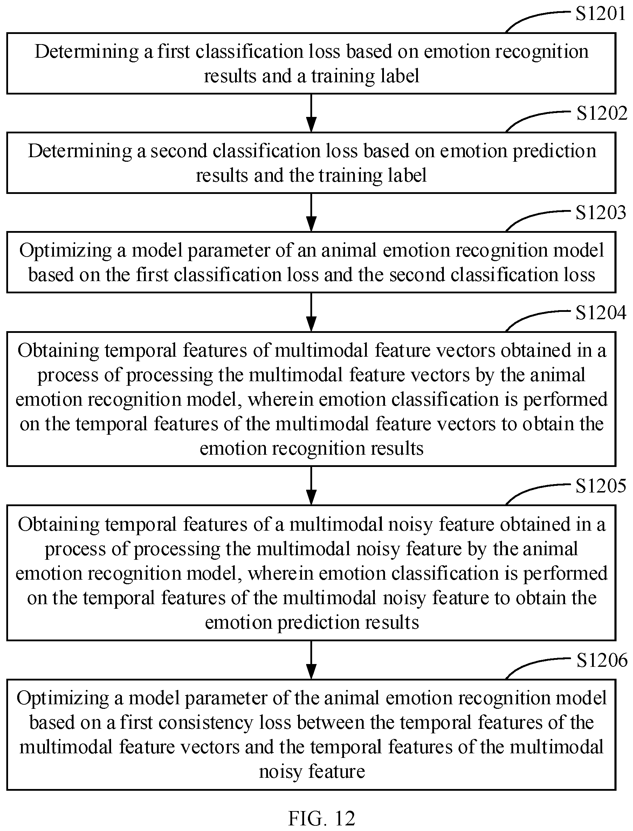

A model parameter of the animal emotion recognition model is optimized by the adversarial training. Particularly, training the animal emotion recognition model based on the emotion recognition results at a training loss level can be implemented as shown in FIG. 12.

At 1201, a first classification loss is determined based on the emotion recognition results and a training label.

At 1202, a second classification loss is determined based on the emotion prediction results and the training label.

At 1203, a model parameter of the animal emotion recognition model is optimized based on the first classification loss and the second classification loss.

That is, the fused features obtained before and after the disturbance is added are both interfered and predicted by the animal emotion recognition model, generating corresponding emotion recognition results or emotion prediction results. Both of these results determine the cross entropy loss with corresponding training labels, resulting in the first classification loss and the second classification loss.

The two classification losses are used to optimize the model parameter of the animal emotion recognition model, such that the animal emotion recognition model can not only handle the fused feature to which the disturbance is added, but also perform correct emotion classification on the fused feature obtained after the disturbance is added, improving the classification accuracy and robustness of the animal emotion recognition model.

In further embodiments, a consistency loss can be introduced in the adversarial training to optimize the model parameter, as shown in FIG. 12.

At 1204, temporal features of the multimodal feature vectors obtained in a process of processing the multimodal feature vectors by the animal emotion recognition model are acquired, wherein emotion classification is performed on the temporal features of the multimodal feature vectors to obtain the emotion recognition results.

At 1205, temporal features of the multimodal noisy feature obtained in a process of processing the multimodal noisy feature by the animal emotion recognition model are acquired, wherein emotion classification is performed on the temporal features of the multimodal noisy feature to obtain the emotion prediction results.

At 1206, the model parameter of the animal emotion recognition model is optimized based on a first consistency loss between the temporal features of the multimodal feature vectors and the temporal features of the multimodal noisy feature.

In embodiments of the present disclosure, the first consistency loss is used to measure the feature modelling capability of the model before and after the disturbance is added to increase the robustness of the model.

In addition, as shown in FIG. 12, a second consistency loss between the emotion recognition results and the emotion prediction results can be determined, and the model parameter of the animal emotion recognition model is optimized based on the second consistency loss.

The emotion recognition results and the emotion prediction results are classification probability distributions, which represent probabilities belonging to different animal emotion categories. Therefore, the parameter of the animal emotion recognition model is optimized by restricting final outputs to be consistent.

In embodiments of the present disclosure, the feature modelling capability of the model before and after the disturbance is added can be measured by the second consistency loss to increase the robustness of the model.

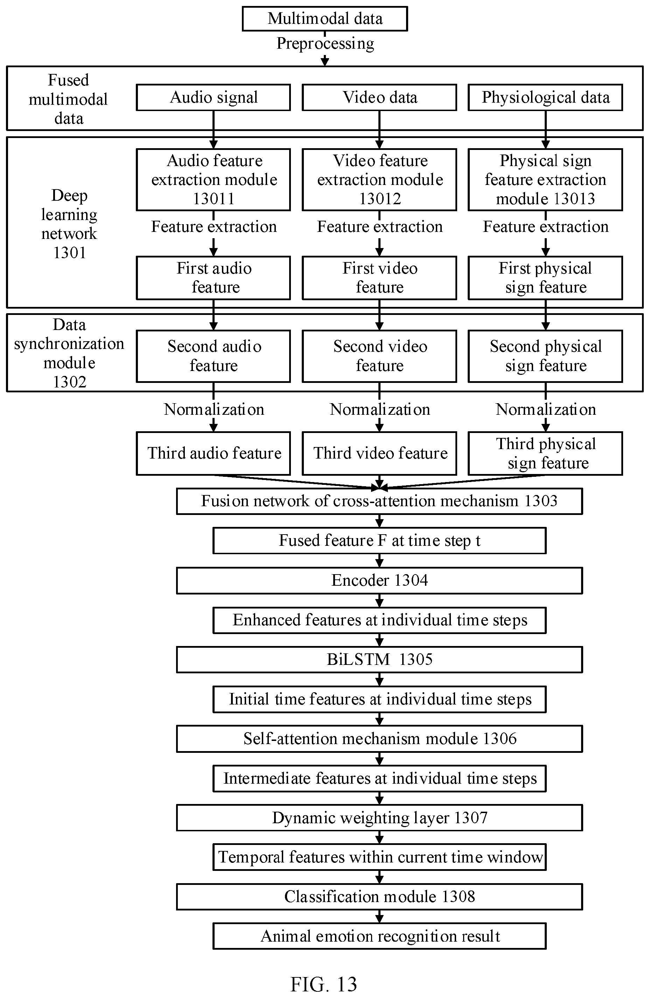

As shown in FIG. 13 which is a structural diagram of a main framework of an animal emotion recognition model according to an embodiment of the present disclosure, multimodal data is preprocessed, and the preprocessing includes cleaning the data, and macroscopically aligning the same actual multimodal data based on timestamps to obtain fused multimodal data in a standardized format. The fused multimodal data includes audio signals, video data and physiological data.

Feature extraction is performed on the audio signals in the fused multimodal data by an audio feature extraction module 13011 in the deep learning network 1301 to obtain a first audio feature. Feature extraction is performed on the video data in the fused multimodal data by a video feature extraction module 13012 to obtain a first visual feature. Feature extraction is performed on the physiological data in the fused multimodal data by a physical sign feature extraction module 13013 to obtain a first physical sign feature. A data synchronization module 1302 is configured to perform data synchronization on the first audio feature, the first visual feature and the first physical sign feature to align frequencies of different modalities to obtain a second audio feature, a second visual feature and a second physical sign feature. Then, the second audio feature, the second visual feature and the second physical sign feature are normalized to obtain the normalized second audio feature (which can be simply referred to as a third audio feature), the normalized second visual feature (which can be simply referred to as a third visual feature) and the normalized second physical sign feature (which can be simply referred to as a third physical sign feature). The third audio feature, the third visual feature and the third physical sign feature are processed by a fusion network 1303 based on a cross attention mechanism to obtain the fused feature Ft at a time step t. The fused multimodal data at each time step within the current time window is processed as described above to obtain the fused features corresponding to the individual time steps, respectively. The fused features at the individual time steps within the current time window are processed by an encoder 1304 to obtain enhanced features at the individual time steps. The enhanced features at the individual time steps are input to a BiLSTM 1305 to obtain initial time features at the individual time steps. The initial time features at the individual time steps are input into a self-attention mechanism module 1306 to obtain the intermediate features at the individual time steps. Weighted summation is performed on the intermediate features at the individual time steps by a dynamic weighting layer 1307 to obtain temporal features $h_{final}$ within the current time window. The temporal features $h_{final}$ are input to a classification module 1308 to obtain the animal emotion recognition results.

In the inference stage, a processing flow of the animal emotion recognition model is the same as that shown in FIG. 13. In the training stage, additional adversarial training is performed in embodiments of the present disclosure. Specifically, the robustness and boundary processing capability of the animal emotion recognition model can be improved in a manner of adding the disturbance. The disturbance can be added at each time step. Regardless of the method for adding the disturbance, each time step will result in fused features, which the disturbance, of the multimodal noisy feature.

The fused features of the multimodal noisy features at a plurality of time steps are processed by the encoder 1304 in FIG. 13, the BiLSTM 1305, the self-attention mechanism module 1306 and the dynamic weighting layer 1307 to obtain temporal features $h_{final}$' including the disturbance, wherein the temporal features $h_{final}$' including the disturbance are processed by the classification module 1308 to obtain the emotion prediction results. Therefore, classification losses of the emotion recognition results and the emotion prediction results within the same time window can be calculated, respectively. Consistency losses of the temporal features $h_{final}$ and the temporal features $h_{final}$' within the same time window need to be calculated. These losses can be used to optimize the model parameter of the animal recognition model.

Specifically, the above three losses are collectively referred to as adversarial losses, and an expression for the adversarial losses is as shown in the following formula (12):

$$L_{total} = L_{cls}(F_t, y) + \lambda_1 \cdot L_{cls}\left(F_t^{'}, y\right) + \lambda_2 \cdot L_{consistency}$$

$$L_{consistency} = \left\| f(F_t) - f\left(F_t^{'}\right) \right\|^2 \quad (12)$$

wherein $L_{total}$ represents an adversarial loss, $L_{cls}(F_t, y)$ represents a classification loss of the emotion recognition results obtained before the disturbance is added, $L_{cls}\left(F_t^{'}, y\right)$ represents a classification loss of the emotion prediction results obtained after the disturbance is added, $L_{consistency}$ represents the consistency loss (such as a first consistency loss or a second consistency loss), and $\lambda$ represents a weight coefficient.

The entire model parameter is optimized during training under these three joint constraints contained in the adversarial loss. A disturbance path is highly coupled, despite appearing seemingly independent. It can be understood that three branches (normal input, perturbation input and consistency constraint) share the parameter, and are optimized towards a unified final target. In this way, the robustness and generalization ability of the model to abnormal samples can be improved, and the misclassification of pseudo label samples is reduced.

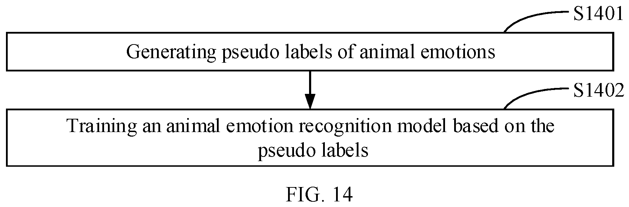

In some embodiments, due to a limited number of annotated labels in the sample data, in order to effectively utilize the sample data, as shown in FIG. 14, the method can further be implemented as follows.

At S1401, pseudo labels of animal emotions are generated.

The pseudo labels and expert labels are relative concepts. The expert labels refer to training labels obtained by accurately annotating the sample data. The pseudo labels in embodiments of the present disclosure mainly refer to results output by inferring and analyzing the sample data by the animal emotion recognition model, and the pseudo labels are a part of the training labels.

At S1402, the animal emotion recognition model is trained based on the pseudo labels.

In embodiments of the present disclosure, the sample data can be effectively utilized by automatically generating the pseudo labels, and thus the generalization ability of the animal emotion recognition models can be improved.

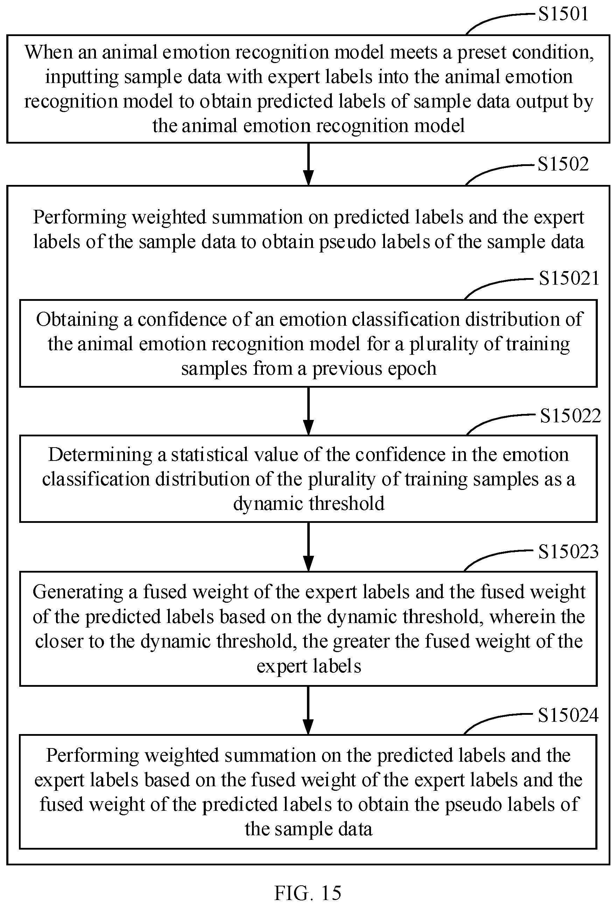

In some embodiments, in order to efficiently utilize the sample data with the expert labels to generate the pseudo labels of the animal emotions, as shown in FIG. 15, the method can be implemented as follows.

At S1501, if the animal emotion recognition model meets a preset condition, the sample data with the expert labels is input into the animal emotion recognition model to obtain predicted labels of the sample data output by the animal emotion recognition model.

In order to ensure that the pseudo labels can be reasonably predicted, embodiments of the present disclosure require the animal emotion recognition model to meet the preset condition, that is, requires the output result of the animal emotion recognition model to have a certain accuracy. During implementation, the preset condition includes at least one of the following:

1), the animal emotion recognition model, based on a training epoch count of a training sample with an expert label, has reached a target epoch count; and

2), the animal emotion recognition model meets a set condition for proceeding to the next training stage based on an emotion recognition accuracy of the training sample with the expert label.

The set condition can refer to that the animal emotion recognition accuracy is greater than a preset accuracy threshold, or the accuracy is not increased significantly, but varies within a certain range. For example, the variation range of the accuracy is within a target range.

In summary, both the condition 1) and the condition 2) enable the animal emotion recognition model to learn a certain knowledge and predict animal emotions reasonably after being trained and optimized based on the sample data with the expert label. At this point, generating the pseudo labels by the animal emotion recognition model can improve the quality of the generated pseudo labels.

The predicted label of the sample data output by the animal emotion recognition model is the animal emotion classification distribution. That is, the animal emotion recognition model outputs the animal emotion recognition results based on the sample data. The predicted label includes probability distributions of various animal emotion categories.

At S1502, weighted summation is performed on the predicted labels and the expert labels of the sample data to obtain the pseudo labels of the sample data.

In embodiments of the present disclosure, weighted summation is performed on the predicted label and the expert label inferred by the animal emotion recognition model to generate the pseudo label. Thus, based on the pseudo label, the sample data with the expert label can be reasonably reused to improve the utilization rate of the sample data and the generalization ability of the animal emotion recognition model.

In one implementation, weighted summation can be performed on the predicted label and the expert label.

In some further implementations, generating the pseudo label by weighted summation can be implemented as shown in FIG. 15.

At S15021, a confidence of the emotion classification distribution of the animal emotion recognition model for a plurality of training samples from a previous epoch is obtained.

The plurality of training samples from the previous epoch can be all the samples used in the previous training process, or unannotated samples without expert labels used in the previous training process.

At S15022, a statistical value of the confidence in the emotion classification distribution of the plurality of training samples is determined as a dynamic threshold.

For each training sample, the predicted label corresponding to the training sample can be obtained by the inference of the animal emotion recognition model. The predicted label includes probabilities belonging to various animal emotion categories. During implementation, the maximum probability can be taken as the confidence of the emotion classification distribution of the training sample.

For example, an unannotated sample is input into an animal emotion recognition model, and the animal emotion recognition module outputs the emotion classification distribution of the unannotated sample, that is, probability distributions belonging to various animal emotion categories. Assuming the emotion classification distribution is [0.15, 0.10, 0.70, 0.05], the maximum value 0.7 is obtained as the confidence of the emotion classification distribution of the training sample.

A plurality of confidences are obtained corresponding to a plurality of training samples. Thus, the statistical values of the plurality of confidences can be determined. The statistical value can be understood as a mean, a maximum value and other values for representing the overall recognition confidence of the animal emotion recognition model.

For example, during implementation, the 80th percentile of the confidence distribution can be taken as a pseudo label acceptance threshold, i.e., the dynamic threshold, for the current epoch.

If the dynamic threshold is determined by using the unannotated sample from the previous epoch, the inference and prediction capabilities of the animal emotion recognition model for the unannotated sample are reflected, and the accuracy of the predicted label generated by the animal emotion recognition model can be reflected.

At S15023, a fused weight of the expert labels and the fused weight of the predicted labels are generated based on the dynamic threshold, wherein the closer to the dynamic threshold, the greater the fused weight of the expert labels.

Exemplarily, the fused weight of the expert label can be determined based on the following formula (13):

$$\alpha = \exp(-\beta(p_i - p_{thresh})^2) \quad (13)$$

wherein $\alpha$ represents the fused weight of the expert label, a complement corresponding to $\alpha$, i.e., (1-$\alpha$), is the fused weight of the predicted label, $p_{thresh}$ represents the dynamic threshold, $\beta$ represents a hyperparameter adjusting a slope of a curve, and $p_i$ represents a confidence of the predicted label, i.e., a maximum probability in the predicted label.

Based on the formula (13), it can be deduced that $\alpha$ represents the confidence of the expert label. When the confidence of the predicted label is exactly close to the dynamic threshold, ($p_i$ - $p_{thresh}$) approaches 0, the contribution of the expert label is extremely high, and therefore the predicted label will be handled conservatively in a fusion process.

When the confidence of the predicted label is much higher than the dynamic threshold, ($p_i$ - $p_{thresh}$) is much greater than 0, $\alpha$ rapidly tends to 0, and the fused weight of the predicted label is significantly improved. As a result, the predicted label is automatically trusted in the fusion process. When the confidence of the predicted label is less than the threshold, the predicted label is unreliable and should be excluded from the fusion for generating the pseudo label.

At S15024, weighted summation is performed on the predicted labels and the expert labels based on the fused weight of the expert labels and the fused weight of the predicted labels to obtain the pseudo labels of the sample data.

An expression of generating the pseudo labels is as shown in the following formula (14):

$$y_{va} = \alpha \cdot y_{expert\_va} + (1 - \alpha) \cdot y_{pseudo\_va} \quad (14)$$

wherein $\alpha$ represents the fused weight of the expert label, (1 - $\alpha$) represents the fused weight of the predicted label,

$y_{expert\_va}$ represents the expert label, $y_{pseudo\_va}$ represents the predicted label, and $y_{va}$ represents the generated pseudo label.

On this basis, the predicted label with a confidence score in close proximity to the dynamic threshold resides in a borderline interval, making it subject to influence from noise and uncertainty of the animal emotion recognition model. At this point, increasing the fused weight of the expert label can effectively suppress the drift of the label and the generation of an inferior pseudo label, avoiding interference with the animal emotion recognition model. As the performance of the animal emotion recognition model is improved, the overall confidence score distribution will shift to the right (i.e., is increased), and the dynamic threshold will be increased. Thus, the fused pseudo label can become increasingly superior by the dynamic threshold, gradually increasing the trust in the pseudo label and accelerating a self-evolution loop of the model. Ultimately, generating the pseudo label by the method provided in embodiments of the present disclosure can suppress the weight of a low-confidence predicted label, effectively reduce the accumulated error caused by the diffusion of the pseudo label, and ultimately improve the animal emotion recognition model accuracy.

On this basis, the pseudo label is generated by using an annotated sample with an expert label to improve the utilization rate of the annotated sample and the capability of the animal emotion recognition model to generate the pseudo label. As the quality of the pseudo label generated by the animal emotion recognition model is improved, the predicted label can be generated by the animal emotion recognition model for the unannotated sample. When the confidence of the predicted label is higher than the dynamic threshold, the predicted label can serve as a pseudo label to construct a (unannotated sample and pseudo label) sample pair and continuously optimize the animal emotion recognition model. For example, in experimental measurements, there are 1000 sample data with expert labels. The pseudo labels are generated for the unannotated sample by prediction, wherein the pseudo labels with a confidence of ≥0.85 account for 60%. The model parameter can be optimized by using these high-confidence pseudo labels.

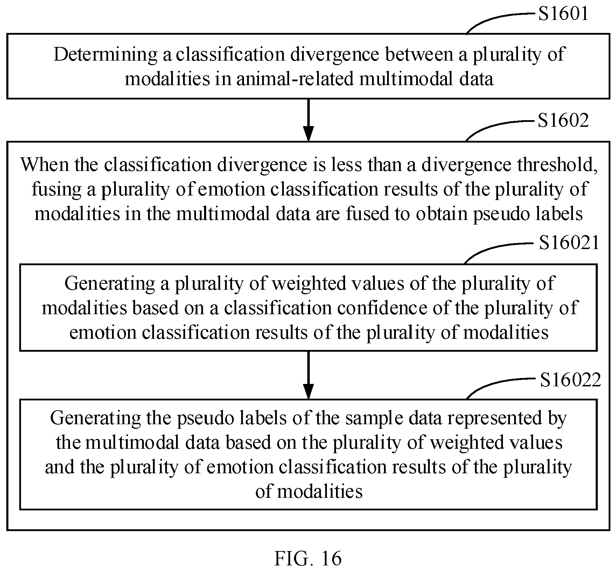

In some embodiments, animals may express emotions separately from different modalities. Therefore, animal emotion categories can be roughly recognized from single modal data. During implementation, in order to mine more pseudo labels and superior sample data that should be learned, in embodiments of the present disclosure generating the pseudo labels of the animal emotions can be implemented as shown in FIG. 16.

At S1601, a classification divergence between a plurality of modalities in the animal-related multimodal data is determined.

The multimodal data includes data of the audio modality, the video modality and the physiological modality. Data of each modality can theoretically be used separately to recognize the animal emotion categories.

The classification divergence is used to measure a discrepancy between animal emotion categories expressed by data of different modalities. The greater the classification divergence, the greater the discrepancy, indicating classification conflicts between different modalities.

In one possible implementation, the classification divergence can be described based on the discrepancy between feature vectors of data of different modalities, as shown in the following formula (15):

$$A_{avg} = \frac{\cos(f_{audio}, f_{video}) + \cos(f_{audio}, f_{bio}) + \cos(f_{video}, f_{bio})}{3} \quad (15)$$

wherein $A_{avg}$ represents a divergence index, and the larger the divergence index, the smaller the classification divergence; and $f_{audio}$ represents a to-be-compared audio vector which can be represented by the feature vector of the audio modality output by the audio modality transformer shown in FIG. 13. Likewise, $f_{video}$ represents a to-be-compared video vector which can be represented by the feature vector of the video modality output by the video modality transformer shown in FIG. 13. Likewise, $f_{bio}$ represents a to-be-compared physiological vector which can be represented by the feature vector of the physiological modality output by the physical sign modality transformer shown in FIG. 13. It can be understood that the to-be-compared audio vector, the to-be-compared audio vector and the to-be-compared physiological vector can be in the same feature space, and are not limited to the feature vectors shown in FIG. 13 during implementation.

In some further embodiments, the classification divergence can be specifically represented based on the following method.

At a step E1, a plurality of emotion classification results are determined based on the animal-related multimodal data, wherein the data of each modality corresponds to a respective emotion classification result.

The multimodal data includes data of the audio modality, the video modality and the physiological modality. The following operations are performed on the data of each modality as the data of the target modality:

performing feature extraction on the data of the target modality to obtain a classification feature; and
performing animal emotion classification based on the classification feature to obtain an emotion classification result, wherein emotion classification result includes the probability distribution belonging to the animal emotions.

At a step E2, the classification divergence between the plurality of emotion classification results of the multimodal data is determined.

It is possible to calculate the discrepancy in the emotion classification results between every two modalities in the plurality of modalities, and then calculate the mean. For example, this discrepancy can be represented by a difference, a L2 distance or a cosine similarity.

At S1602, when the classification divergence is less than a divergence threshold, a plurality of emotion classification results of the plurality of modalities in the multimodal data are fused to obtain the pseudo labels.

That is, animal emotion recognition is performed on the data of each modality separately to obtain the emotion classification results corresponding to the individual modalities. During implementation, animal emotion recognition is performed on data of the individual modalities by the animal emotion recognition model or animal emotion recognition is performed on the individual modalities by further classification models, respectively.

In embodiments of the present disclosure, small classification divergence indicates that the emotion categories represented by the individual modalities in the multimodal data tend to be consistent. The multimodal data is a superior sample, and corresponding pseudo labels can be automatically generated for training the model.

In one possible implementation, weighted summation is performed on the emotion classification results of the plurality of modalities by adopting the fixed weights based on empirical values.

In some further embodiments, in order to improve the quality of the pseudo label, the pseudo label can be generated based on a dynamic weight, which can be particularly implemented as shown in FIG. 16.

At S16021, a plurality of weighted values of the plurality of modalities are generated based on a classification confidence of the plurality of emotion classification results of the plurality of modalities.

That is, each modality corresponds to a weighted value, respectively.

For each modality, the classification confidence of the emotion classification result can be understood as the maximum probability in the emotion classification result. The classification confidence of the emotion classification result is essentially consistent with the confidence of the emotion classification distribution described above in terms of the meaning.

Exemplarily, a process of generating the weighted value of each modality is as shown in the following formula (16):

$$w_m = Softmax(p_{conf}) \quad (16)$$

wherein $p_{conf}$ represents the classification confidence of the emotion classification result of the corresponding modality. *Softmax* is used to represent a function of generating the weighted value. In addition to *Softmax,* a gated network can be employed, which is not limited in embodiments of the present disclosure.

At S16022, the pseudo label of the sample data represented by the multimodal data is generated based on the plurality of weighted values and the plurality of emotion classification results of the plurality of modalities.

Exemplarily, in order to improve the credibility of the pseudo label, the pseudo label can be generated by an activation layer, which is implemented as shown in the following formula (17):

$$y_{pseudo} = Softmax\left(\sum_m w_m \cdot y_m\right) \quad (17)$$

wherein *Softmax* is the activation layer, $y_m$ is an emotion classification result of an $m^{th}$ modality, and $w_m$ is a weighted value of the $m^{th}$ modality. Important contents can be effectively selected by the activation layer, such that the credibility of the pseudo label is improved.

In embodiments of the present disclosure, the weight is generated by the classification confidence, and the recognition accuracy of the individual modalities for animal emotion categories can be measured. The higher the accuracy, the greater the contribution to the animal emotion classification, which can improve the quality of the generated pseudo label.

In some embodiments, when the classification divergence is not less than the divergence threshold, the multimodal data is stored in an observation pool. After the multimodal data is stored the observation pool, available multimodal data can be selected based on a preset strategy as training samples, or corresponding training labels can be obtained by manual annotation. Therefore, sample data for which the pseudo label can not be automatically generated can be selected by the classification divergence for being processed in subsequent processes.

In some embodiments, the quality of the generated pseudo label needs to be controlled to ensure that the superior pseudo label participates in the training of the animal emotion recognition model. Therefore, when the confidence of the pseudo label is greater than the dynamic threshold, the sample data corresponding to the pseudo label is determined as the sample data for training the animal emotion recognition model. The dynamic threshold is determined based on the statistical value which is a statistical value of the confidence of the emotion classification distribution of the emotion recognition model for a plurality of training samples in the previous epoch. An approach of determining the dynamic threshold has been explained above and will be omitted here.

The confidence of the pseudo label can be understood as the maximum probability in the probability distribution of the generated pseudo label. If the pseudo label is generated based on the weighted summation on the expert label and the predicted label, the confidence of the pseudo label is the confidence of the predicted label used by the pseudo label.

By selecting the high-confidence pseudo label to participate in the training of the animal emotion recognition model, the impact of an inferior label on the model can be reduced, and the efficiency of training the animal emotion recognition model can be improved.

In some embodiments, if the confidence of the pseudo label is not greater than the dynamic threshold, the sample data corresponding to the pseudo label is stored in an observation pool. As a result, sample data of the pseudo label with poor quality can be selectively removed, which facilitates subsequent analysis and optimization of the sample data and the label quality.

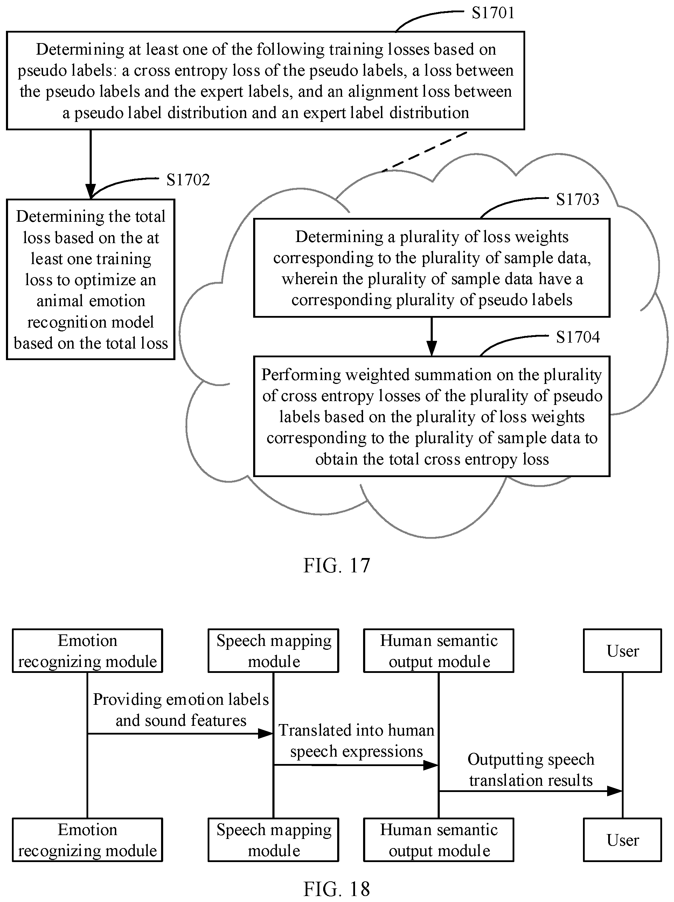

In some embodiments, regardless of the method for generating the pseudo label, the pseudo label and its corresponding sample data can be used to optimize the animal emotion recognition model, which can be implemented as shown in FIG. 17.

At S1701, at least one of the following training losses is determined based on the pseudo labels: a cross entropy loss of the pseudo labels, a loss between the pseudo labels and the expert labels, and an alignment loss between the pseudo label distribution and the expert label distribution.

Among them, the cross entropy loss of each pseudo label is used to measure the cross entropy loss between the emotion classification distribution output by the animal emotion recognition model after the sample data is processed and the corresponding pseudo label.

If the sample data has corresponding expert labels, the loss between the generated pseudo label and the expert label can be introduced to constrain the output of the animal recognition model to be as consistent as possible with the expert label, thereby improving the utilization rate of the annotated samples.

The alignment loss between the pseudo label distribution and the expert label distribution, as the name suggests, is used to constrain probability distributions of a plurality of pseudo labels generated and their corresponding expert labels in an annotated sample set in terms of animal emotion categories to be consistent.

At S1702, the total loss is determined based on the at least one training loss to optimize the animal emotion recognition model based on the total loss.

In embodiments of the present disclosure, the generation of the superior emotion classification distribution by the animal emotion recognition model is constrained by introducing the cross entropy loss, and the performance of the model on the unannotated data is optimized. The loss between the pseudo label and the expert label can enable the animal emotion recognition model to learn the annotated data more fully. Aligning the loss enables the animal emotion recognition model to learn superior label distributions and improve model convergence efficiency.

In some embodiments, the total loss includes a total cross entropy loss of the plurality of sample data, and the determining the total cross entropy loss can be implemented as shown in FIG. 17.

At S1703, a plurality of loss weights corresponding to the plurality of sample data are determined, wherein the plurality of sample data have a corresponding plurality of pseudo labels.

The loss weight corresponding to the sample data can be dynamically generated, and the loss weight is used to measure the contribution of the sample data to the total loss, thereby improving the learning rate of the superior sample data while weakening the learning of relatively inferior sample data.

Generating the loss weight can be implemented as follows: generating a plurality of loss weights corresponding to the plurality of sample data based on the discrepancy between a plurality of pseudo labels and their corresponding expert labels of a plurality of sample data. The discrepancy has an inverse correlation with the loss weight. Exemplarily, the inverse correlation can be expressed as shown in the following formula (18):

$$w_i = \frac{P_e(y_i)}{\left(P_p(y_i) + \varepsilon\right)} \qquad (18)$$

wherein $w_i$ represents the loss weight of the ith sample data, $P_e(y_i)$ represents the expert label, $P_p(y_i)$ represents the pseudo label, and $\varepsilon$ represents a constant parameter with a value below a preset lower limit, the value of $\varepsilon$ is extremely low, which can be used to prevent the denominator from being 0 without influencing the calculation of the loss weight.

An approach of determining the loss weight ensures that the closer the pseudo label is to the expert label, the higher the weight, i.e., higher weight can be generated for the sample data of the superior pseudo label, enabling the animal emotion recognition model to learn a superior feature modelling approach.

At S1704, weighted summation is performed on the plurality of cross entropy losses of the plurality of pseudo labels based on the plurality of loss weights corresponding to the plurality of sample data to obtain the total cross entropy loss.

Exemplarily, the total cross entropy loss can be expressed as shown in the following formula (19):

$$L_{total} = \sum w_i \cdot \text{CrossEntropy}(y_{soft}, \widehat{y}_i) \quad (19)$$

wherein $w_i$ represents the loss weight of the ith sample data; $y_{soft}$ represents the animal emotion classification distribution inferred by the animal emotion recognition model for the ith sample data, and is a probability distribution; as the model parameter is adjusted, the prediction result for the same sample data may vary; and $\widehat{y}_i$ represents the generated pseudo label.

In embodiments of the present disclosure, reasonable loss weights are generated for different sample data, such that the total cross entropy loss can be used to measure the shortcomings of the animal emotion recognition model, facilitating targeted adjustment of the model parameter and improving the model training efficiency.

In some embodiments, the total loss includes a total alignment loss of the plurality of sample data, and the total alignment loss is used to measure a distribution disparity between the pseudo labels and the expert labels of the plurality of sample data. In a training stage, the total alignment loss is minimized by optimizing the model parameter of the animal emotion recognition model.

The distribution disparity between the pseudo label and the expert label can be referred to as a KL (Kullback-Leibler Divergence) loss. Correspondingly, an approach of calculating the total divergence loss is as shown in the following formula (20):

$$L_{KL} = \sum P_e(y_k) \cdot \log\left(\frac{P_e(y_k)}{(P_p(y_k) + \varepsilon)}\right) \quad (20)$$

wherein $L_{KL}$ represents the KL divergence loss, $P_e(y_k)$ represents a distribution probability of the expert label on a kth animal emotion category, $P_p(y_k)$ represents a probability distribution of the pseudo label on the kth animal emotion category, and a value of $\varepsilon$ is extremely low, which can be used to prevent the denominator from being 0 without influencing the calculation of the loss weight.

In some further embodiments, the distribution disparity between the pseudo label and the expert label can be represented as a Jensen-Shannon divergence (JS divergence) between the pseudo label and the expert label. The JS divergence corresponding to the distribution probability of each animal emotion category is as shown in the following formula (21):

$$L_{JS} = \frac{1}{2}D_{KL}(p_{expert}, M) + \frac{1}{2}D_{KL}(p_{pseudo}, M)$$
$$M = \frac{1}{2}(p_{expert} + p_{pseudo}) \quad (21)$$

wherein $p_{expert}$ represents a distribution probability of the expert label on the kth animal emotion category, and $p_{pseudo}$ represents a probability distribution of the pseudo label on the kth animal emotion category. When the total divergence loss is determined, the JS divergences of the individual animal emotion categories are summated after being calculated based on the following formula (21).

In addition, the distribution disparity between the pseudo label and the expert label can be combined with the KL divergence loss and the JS divergence loss mentioned above, as shown in the following formula (22) below:

$$L_{total} = \lambda_1 L_{KL}(p_{expert}||p_{pseudo}) + \lambda_2 L_{JS}(p_{expert}, p_{pseudo}) \quad (22)$$

wherein the KL divergence loss and the JS divergence loss between the pseudo label and the expert label for each animal emotion category can be calculated, respectively, and then subjected to weighted summation, wherein $\lambda_1$ and $\lambda_2$ are weights, which can be determined based on empirical values.

In some embodiments, in order to prevent the KL divergence caused by zero values in the probability distribution on the kth animal emotion category, the pseudo label and the expert label in the formulas (21) and (22) can be updated for the probability distribution of the kth animal emotion category, as shown in the following formula (23):

$$\tilde{P}_{(y_k)} = \frac{P_{(y_k)} + \delta}{1 + K\delta} \quad (23)$$

wherein K represents the total number of animal emotion categories, and $\delta$ is a positive number arbitrarily close to zero.

In embodiments of the present disclosure, for sample data with expert labels, by aligning the probability distributions of the pseudo label and the expert label on a plurality of animal emotion categories, results output by the animal emotion recognition model for the sample data with the expert labels are constrained to be close to a distribution of the expert labels, which can prevent the drift of the pseudo label and improve the classification accuracy of the animal emotion recognition model.

In some embodiments, the total loss includes the total distance loss of the plurality of sample data. Determining the total distance loss can be implemented as: determining a distance between the pseudo label and the corresponding expert label in the plurality of sample data; and determining the statistical value of the distance between the plurality of sample data to obtain the total distance loss.

During implementation, the distance between the label and the corresponding expert label can be represented by a L2 distance. Correspondingly, the following formula (24) shows the distance between the label and the corresponding expert label:

$$L_{va} = ||y_{expert\_va} - y_{pseudo\_va}||^2 \quad (24)$$

wherein $y_{expert\_va}$ represents the expert label, $y_{pseudo\_va}$ represents the pseudo label, and $L_{va}$ represents the distance between the expert label and the pseudo label.

The distance loss can enable the predicted pseudo label closer to the expert label, improving the classification accuracy of the animal emotion recognition model.

In summary, in embodiments of the present disclosure, the training the animal emotion recognition model can include the following three stages.

At a stage 1, the animal emotion recognition model is trained by sample data with expert labels to optimize the animal emotion recognition model. At this stage, the model parameter can be optimized by adversarial losses from adversarial training (as shown in the formula (12)).

At a stage 2, semi-supervised training is performed by the pseudo label to increase the utilization rate of the sample.

At this stage, adversarial loss and the total loss for the pseudo labels can be used to optimize the model parameter.

At a stage 3, fine-tuned training is performed on the animal emotion recognition model based on actual application scenarios to enable the animal emotion recognition model to adapt to specific animal emotion recognition.

In some embodiments, after the animal emotion recognition model is trained, superior samples can be selected based on the prediction results in the inference stage to optimize the model. For example, if a preset event is continuously detected for multiple times in the inference stage of the animal emotion recognition model, multimodal data corresponding to the preset event is acquired to serve as newly-added sample data. The preset event includes at least one of the following:

1), the emotion recognition results indicate that a first target emotion category is recognized and a confidence of the emotion recognition results is greater than a preset threshold;
2), the emotion recognition results indicate that a second target emotion category is recognized and continuously detected for n times, wherein n is a positive integer greater than 1; and
3), animal emotion variation values within a sliding time window to which the emotion recognition results belong is higher than a preset value.

The preset events have been explained earlier and will be omitted here.

It should be understood that by means of the preset event, data samples of designing critical animal emotions can be captured, and used to optimize the model parameter and improve the recognition accuracy of the animal emotion recognition model.

For example, a pet began to exhibit an emotion label associated with an "aggression" emotion at 12:02:01, and lasted for 3 times. An event recording window is from 12:02:01 to 12:02:10. An end time can be automatically determined in ways that no "aggression" is detected for N consecutive seconds (such as 10 seconds) or a set "cooldown period" has elapsed after the most recent detection of the "aggression" without new anomalies, indicating the end of the window.

At this point, the sampling rate is increased since the preset event is triggered, and the collected multimodal data is marked as an "aggression event sample". The multimodal data is used to construct the training samples, in order to enhance the learning intensity of the animal emotion recognition model for an "aggression" category. An enhancement measure includes: 1) prioritizing the addition of an incremental training set to the sample data corresponding to the preset event to increase the sampling learning probability; 2) during training, assigning higher loss weights to high-risk categories, which can lead to overfitting of rare/high-risk labels in the model; and 3) if necessary, initiating a fine-tuning process to shorten a response cycle of new data to the model. In the training stage, corresponding pseudo labels are generated. If the confidence of the pseudo labels is greater than the dynamic threshold, the pseudo labels enter the enhanced sample set for enhanced training. Otherwise, the pseudo labels enter the observation pool.

In some alternate embodiments, the performing semantic mapping and language translation on the emotion recognition results to translate animal languages into human languages to obtain language translation results includes:

extracting an emotion label and a sound feature from the emotion recognition results, and converting the sound feature into a standardized sound vector;

performing, by a pretrained language model, semantic mapping on the emotion label and the sound vector to obtain an emotion intention; and

performing, by a language generator, language translation on the emotion intention to generate a corresponding human language to obtain the language translation result.

Specifically, the "pre-trained language model" refers to an artificial intelligence model that has been trained on a large amount of data and is capable of understanding and processing a natural language. The model is used here to perform "semantic mapping" on animal emotion labels, i.e., the animal emotion states obtained from the emotion recognition module, with sound vectors, that is, to correspond the animal sound features to emotion semantics in the human language, in order to recognize the animal emotion intentions. Next, the "language generator" is a process of converting an animal non-speech communication into a human-understandable language based on these emotion intentions. Such a process involves converting the animal emotions and intentions into a concrete text or speech output, i.e., the "language conversion result", enabling humans to intuitively understand animal "languages" and achieve human-animal effective communication.

For a better understanding of solutions of embodiments of the present invention, the following example will be given, as shown in FIG. 18 which is a schematic flowchart of translating animal languages into human languages according to an embodiment of the present disclosure. After the emotion recognition results are obtained by the emotion recognition module, the animal emotion labels and sound features are provided, and then communicated to a speech mapping module responsible for converting the animal sound features into human-understandable speech expressions. Next, a human semantic output module receives the converted speech information, outputs the converted speech information as a language conversion result, and ultimately presents these results to the user, achieving real-time translation from the animal languages to the human languages and emotion communication. The entire process involves emotion recognition, feature extraction and mapping, and generation of the human languages, aiming to promote the human-animal effective communication.

In this way, semantic mapping is performed between the emotion labels and the sound vectors by the pre-trained language model, thereby accurately capturing and understanding the animal emotion intentions, and then translating these emotion intentions into the human languages by a language generator. Such a process not only achieves precise interpretation of the animal emotions, but also enables animal non-speech communication into the human-understandable languages, greatly promoting human-animal communication and interpretation, and improving the transparency of animal emotion expression and the communication efficiency.

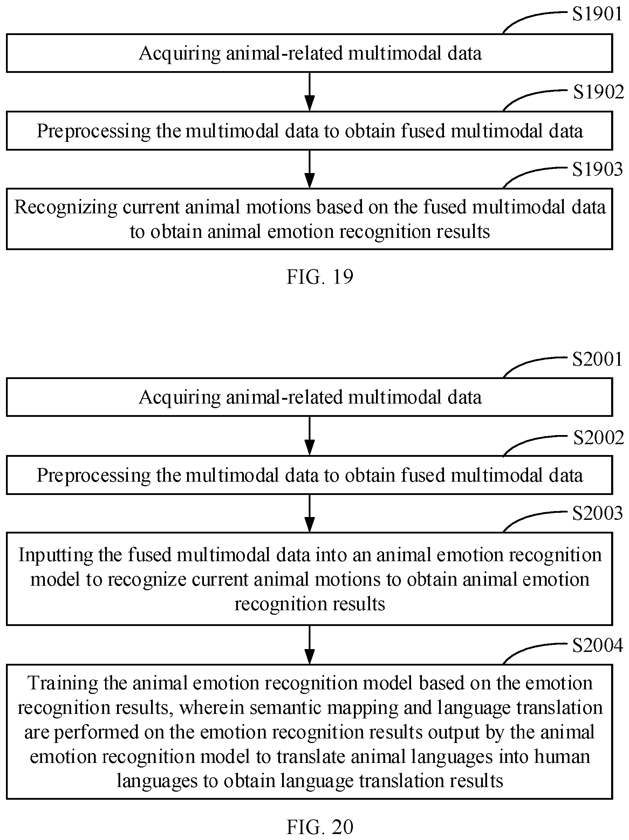

Based on the same technical concept, embodiments of the present disclosure further provide an animal emotion recognition method, as shown in FIG. 19, including:

step S1901, acquiring animal-related multimodal data;
step S1902, preprocessing the multimodal data to obtain fused multimodal data; and
step S1903, recognizing current animal motions based on the fused multimodal data to obtain animal emotion recognition results.

The steps S1901 through S903 correspond to the steps S101 through 103 mentioned above. Implementations and effects of the same steps and their related contents refer to the contents described above, and will be omitted here. In addition, embodiments of aspects, except for aspects in FIG. 1, of the embodiments of the animal language translation method are applicable to the animal emotion recognition method shown in FIG. 19, and will be omitted here.

Based on the same inventive concept, embodiments of the present disclosure further provide a method for training an animal emotion recognition model in an animal language translation model. The animal language translation model includes the animal emotion recognition model and a mapping network model for implementing the step S104. The mapping network model includes the pre-trained language model and/or the language generator.

As shown in FIG, 20 which is a schematic flowchart of a method for training an animal emotion recognition model in an animal language translation model, the method includes:

S2001, acquiring animal-related multimodal data;
S2002, preprocessing the multimodal data to obtain fused multimodal data;
S2003, inputting the fused multimodal data into the animal emotion recognition model to recognize current animal motions to obtain animal emotion recognition results; and
S2004, training the animal emotion recognition model based on the emotion recognition results, wherein semantic mapping and language translation are performed on the emotion recognition results output by the animal emotion recognition model to translate animal languages into human languages to obtain language translation results.

The steps S2001 through S2004 correspond to the steps S701 through 704 mentioned above. Implementations and effects of the same steps and their related contents refer to the contents described above, and will be omitted here. In addition, embodiments of aspects, except for aspects in FIG. 7, of the embodiments of the method for training the animal emotion recognition model related to FIG. 7 are applicable to the method shown in FIG. 20, and will be omitted here.

In addition to the contents explained earlier, embodiments of the present disclosure further provide the following alternate implementations. The following embodiments can be applied to the embodiments related to FIG. 1 to FIG. 20 mentioned above.

In some alternate embodiments, the method further includes:

if specific sound data is detected and no emotion matching history exists, annotating the specific sound data to obtain an updated emotion label; and
dynamically updating the sample data based on the updated emotion label to adjust the model parameter according to the updated sample data.

Specifically, when the specific animal sound data is detected, and does not exist in the emotion matching history, i.e., no previous emotion label corresponds to the specific animal sound data, a labeling process will be triggered. The "annotation" here refers to manual assignment of an emotion label to these specific sound data to "obtain the updated emotion label", and the emotion label describes an animal emotion state when the animal makes that sound. Subsequently, the newly annotated emotion label is incorporated in a sample database to achieve "dynamic updating of the sample data based on the updated emotion label". Such updated sample data is used to adjust the model parameter, that is, to "such that the model parameter is adjusted based on the updated sample data", thereby optimizing and improving the emotion recognition capability of the model for newly-added sound data, ensuring that the system can adapt to new or uncommon animal sounds, improving the recognition accuracy, and enhancing the adaptive learning capability of the system.

For understanding solutions of embodiments of the present disclosure, the following example will be given. In the event of indistinguishable vocalization patterns or anomalies, the user reminds a user inputting relevant labels or recognition information. For example, if the system detects a specific action and sound combination without a significant emotion expression matching history, the user can annotate a phonetic symbol by an interface, such as a "help" or "hunger" status. After manual annotation is performed by the user, the system adjusts the current audio and the data parameter of the corresponding behavior label, thereby updating weights of an existing model. After the annotation is completed by the user, the system updates the dataset in real time, and continuously optimize and retrain relevant generative models, thereby gradually improving the recognition rate of similar or new emotion event categories.

In this way, manual annotation is performed when the specific sound data is detected and the emotion matching history is absent, and the emotion labels are updated accordingly, such that the sample database can be continuously expanded and enriched, and the model parameter can be dynamically updated, the capability of the system to recognize emotions in new sound data is enhanced, the adaptability and accuracy of the model are improved, continuous evolution of a cross-species communication system can be achieved, and communication intentions of the animals are better understood and responded.

In some alternate embodiments, the method further includes:

collecting multimodal data within a preset time window to obtain animal emotion change data;
performing feature extraction on the emotion change data to obtain emotion change features; and
updating the emotion label based on a gap between the emotion change features within the current time window and the emotion change features within a previous time window.

Specifically, the "preset time window" refers to a specific period of time set for analyzing animal emotion changes. "Collecting the multimodal data during such a period of time" refers to that collecting information including animal sounds, behaviors and physical signs to collect data on animal emotion states. Next, by means of a process of "feature extraction", critical information representative of the animal emotion changes is recognized from these data, that is, the "emotion change features". Then, discrepancies between the emotion features extracted within the current time window and the features within the previous time window are compared. If these discrepancies indicate a significant change in the animal emotion state, "the emotion label is updated" based on such a change to reflect a latest animal emotion state. Such a process involves continuous monitoring and dynamic analysis of the animal emotion states to ensure real-time and accurate emotion recognition.

In this way, by collecting the animal-related multimodal data within a preset time window, the animal emotion changes can be comprehensively captured and feature extraction can be performed on these data to recognize the emotion change features. Secondly, the emotion labels are dynamically updated based on the discrepancies between the emotion features within the current time window and the emotion features within the previous time window, such that real-time and accurate monitoring and response of the animal emotion states can be achieved. Such a process not only improves the emotion recognition accuracy, but also enhances the real-time and deep human-animal communication, enabling humans to better understand and respond to animal emotion needs.

In some alternate embodiments, the acquiring multimodal data within a preset time window to obtain animal emotion change data includes:

acquiring multimodal data within a preset time window; and
inputting the multimodal data into an emotion period recognition model to obtain animal emotion change data by the emotion period recognition model.

Specifically, the "preset time window" refers to a specific period of time set for monitoring and analyzing animal emotion changes. During this period of time, the "multimodal data" is acquired, and includes different types of information such as animal sounds, behavioral movements and physical signs. These data are then input into the "emotion period recognition model", and the emotion period recognition model is an algorithm specially designed to process and analyze temporal data, such as a long short-term memory network (LSTM) or a gated recurrent unit (GRU). The model recognizes and extracts features related to the animal emotion states by analyzing these multimodal data, thereby "obtaining the animal emotion change data". These data reflect the animal emotion states within continuous time windows, providing a foundation for further emotion recognition and label update. In this way, the animal emotion states can be dynamically tracked and accurately recognized in the solution.

In this way, by collecting the animal-related multimodal data within a preset time window and inputting these data into the emotion period recognition model, the animal emotion changes can be accurately captured and analyzed. Such a method can recognize and understand animal emotion dynamics within the consecutive periods, thereby providing richer and more accurate emotion change data. These data not only enhance deep and real-time emotion recognition, but also improve the quality and efficiency of the human-animal communication, enabling humans to carefully and immediately respond to animal emotion needs and behavior changes.

In some alternate embodiments, updating the emotion label based on a gap between the emotion change features within the current time window and emotion change features within a previous time window includes:

calculating a Euclidean distance difference between the emotion change features within the current time window and the emotion change feature within the previous time window to obtain an emotion gap; and
if the emotion gap exceeds a preset emotion gap threshold, upgrading the emotion label to obtain an updated emotion label.

Specifically, the "emotion change features within the current time window" refer to an emotion-related feature extracted from the animal-related multimodal data during a specific period of time, while the "emotion change features within the previous time window" are corresponding features within the previous period of time. A quantified "emotion gap" can be obtained by calculating the "Euclidean distance difference" between the emotion features within these two time windows, which is a method of measuring the distance between two points in a multidimensional space.

A Euclidean distance difference between the emotion change features within the current time window and the emotion change features within the previous time window meets the formula: [D (Wi,W{i-1})=\sqrt{\sm{n=1}{N}(x{i,n}-x{i-1,n})2}], that is, $D(W_i, W_{i-1}) = \sqrt{\sum_{n=1}^{N}(x_{i,n} - x_{i-1,n})^2}$ , wherein D(W_i,W_{i-1}) and $D(W_i, W_{i-1})$ represent a Euclidean distance between emotion change features within an $i^{th}$ time window and emotion change features within a $(i-1)^{th}$ time window, which is used to quantify an extent of emotion feature changes within two consecutive time windows. x_{i,n} and $x_{i,n}$ represent the value of an nth feature within the $i^{th}$ time window, which may include sound pitch, rhythm, amplitude, behavior frequency, heart rate changes of physical signs, and the like. x_{i-1,n} and $x_{i-1,n}$ represent a value of the nth feature within the $(i-1)^{th}$ time window. Sum represents a summation operation while SQRT represents a square operation.

The emotion gap is obtained by calculating the Euclidean distance difference between the emotion change feature within the current time window and the emotion change features within the previous time window, which reflects an extent of animal emotion state changes within two consecutive time windows. If the gap exceeds a "preset emotion gap threshold", which is a pre-set boundary indicating a significant animal emotion state change, the "emotion label upgrade" can be triggered. Based on the emotion gap and change patterns, the emotion label will be updated from one state (such as "alert") to another state conforming to a current emotion state (such as "anxiety"), thereby obtaining the "updated emotion label". Such a process makes emotion recognition more dynamic and accurate, which can respond to the significant animal emotion change in real time.

In this way, the extent of animal emotion state changes can be quantified by calculating the Euclidean distance difference between the emotion change features within the current time window and the emotion change features within the previous time window, and the emotion gap is obtained, When the emotion gap exceeds the preset threshold, the emotion label will be automatically upgraded to obtain the updated emotion label. Such a method makes emotion recognition more sensitive and accurate, and can capture and respond to the significant animal emotion changes in real time, thereby improving the human-animal communication quality and efficiency, and ensuring that humans can understand and respond to animal emotion needs appropriately in real time.

In some alternate embodiments, the method further includes:

scoring an emotion weight corresponding to each time window to obtain an emotion weight score; and accumulating the emotion weight scores corresponding to similar emotion time windows within consecutive periods of time, and if an accumulated result is greater than a set upgrade threshold, updating the emotion label.

Specifically, the "emotion weight score" refers to a process of quantitatively evaluating the animal emotion states within each time window, wherein each time window contains the multimodal data collected during a specific period of time. The emotion features are extracted by analyzing these data and assigned different weights based on an intensity or significance of the features, and the emotion weight score within each time window is calculated. Next, the weight scores within the time windows with similar emotion features over the consecutive periods of time are accumulated. If the accumulated score exceeds a "set upgrade threshold", which is a predefined value for determining whether the emotion state is significant enough to trigger the label update, the emotion label will be updated to reflect the animal emotion state change. Such a process makes emotion recognition more dynamic and accurate, which can respond to continuous animal emotion changes in real time.

In this way, by assigning weights to the emotion states within each time window and scoring the emotion states, this solution can quantify and track the animal emotion changes over the consecutive periods of time. The weight scores within the time windows with similar emotion features are accumulated. When the accumulated result exceeds the preset upgrade threshold, the emotion label will be automatically updated to more accurately reflect the current animal emotion state. Such a method improves the emotion recognition sensitivity and adaptability, and makes the human-animal communication more precise and timelier, thereby enhancing the capability to understand and respond to the animal emotion needs.

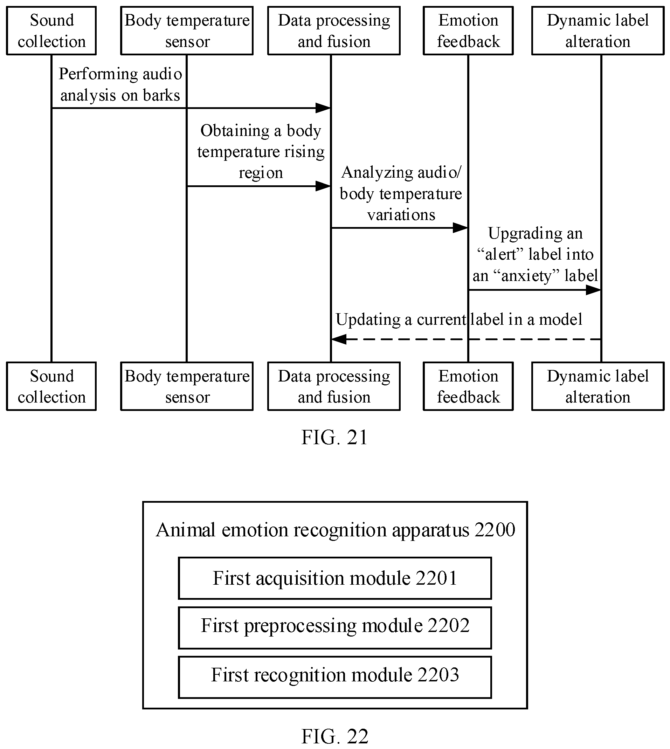

For understanding solutions of embodiments of the present invention, the following example will be given as shown in FIG. 21 which is a schematic flowchart of updating emotion labels according to an embodiment of the present disclosure. Bark audio and temperature data of animals are collected by sound collection and body temperature sensors, and then sent to a data processing and fusion module for analysis to recognize audio features and body temperature changes. Analysis results are used to perform emotion feedback, and dynamically adjust the animal emotion labels based on emotion meanings of audio and body temperature changes, such as upgrading an "alert" state to an "anxiety" state. Such a process involves real-time monitoring and continuous emotion state assessment to ensure that animal emotion changes can be reflected in the label update intermediately, thereby improving the accuracy of understanding and responding to the animal emotion states.

In this way, by collecting the animal emotion change data within the consecutive time windows and extracting the emotion change features from these data, subtle changes in the animal emotions can be monitored and quantified in real time. By comparing the discrepancies between these change features and standard emotion features and updating the emotion labels when a change quantity exceeds a preset threshold, dynamic animal emotion changes can be more accurately captured, further continuous tracking and real-time response to the animal emotion states are achieved, the emotion recognition sensitivity and accuracy are improved, and the human-animal emotion communication effectiveness is enhanced.

Embodiments of apparatuses of the present disclosure will be described below, which can be used to perform the animal emotion recognition method in the above embodiments of the present disclosure. For details not disclosed in embodiments of apparatuses in the present disclosure, reference is made to embodiments of the animal emotion recognition method described above in the present disclosure.

Based on the same technical concept, the present disclosure further provides an animal emotion recognition apparatus 2200, as shown in FIG. 22, including:

a first acquisition module 2201, configured to acquire animal-related multimodal data;
a first preprocessing module 2202, configured to preprocess the multimodal data to obtain fused multimodal data; and
a first recognition module 2203, configured to recognize current animal motions based on the fused multimodal data to obtain animal emotion recognition results.

In some embodiments, the multimodal data includes animal sound data, animal behavior data and animal physical sign data.

In some alternate embodiments, the preprocessing, by the first preprocessing module 2202, the multimodal data to obtain the fused multimodal data includes:

cleaning the multimodal data to obtain cleaned multimodal data;
normalizing the cleaned multimodal data to obtain normalized multimodal data; and
temporally aligning and fusing the normalized multimodal data to obtain the fused multimodal data.

In some alternate embodiments, the recognizing, by the first recognition module 2203, the current animal motions based on the fused multimodal data to obtain animal emotion recognition results includes:

performing, by a deep learning model, sound feature extraction, visual action feature extraction and physical sign change analysis on the fused multimodal data to obtain multimodal feature vectors; and
performing emotion analysis, by a generative adversarial network, on the multimodal feature vectors to obtain the animal emotion recognition results.

In some alternate embodiments, the first recognition module 2203 includes:

a first fusion unit, configured to perform crossmodal feature fusion on the multimodal feature vectors to obtain fused features;
a first temporal modelling unit, configured to temporally model the fused feature to obtain temporal features; and
a first classification unit, configured to classify the temporal features to obtain the animal emotion recognition results.

In some alternate embodiments, the first recognition module 2203 includes:

a first extraction unit, configured to perform, by a deep learning model, sound feature extraction, visual action feature extraction and physical sign change analysis on the fused multimodal data to obtain a first audio feature, a first visual feature and a first physical sign feature;
a first synchronization unit, configured to perform data synchronization on the first audio feature, the first visual feature and the first physical sign feature to obtain a second audio feature, a second visual feature and a second physical sign feature; and
a first normalization unit, configured to normalize the second audio feature, the second visual feature and the second physical sign feature to obtain the multimodal feature vectors.

In some alternate embodiments, the deep learning model and the generative adversarial network are configured to construct an animal emotion recognition model.

In some alternate embodiments, the animal emotion recognition apparatus 2200 further includes:

a frequency adjustment module, configured to, if a preset event is detected based on the emotion recognition results, increase a frequency of acquiring the multimodal data, wherein
the preset event includes at least one of the following:

the emotion recognition results indicate that a first target emotion category is recognized and a confidence of the emotion recognition results is greater than a preset threshold;
the emotion recognition results indicate that a second target emotion category is recognized and continuously detected for n times, wherein n is a positive integer greater than 1; and
animal emotion variation values within a sliding time window to which the emotion recognition results belong are higher than a preset value.

In some alternate embodiments, the frequency adjustment module is further configured to:

with respect to the animals, reduce the frequency of acquiring the multimodal data if the preset event remains undetected for a specified duration starting from the end of detection until the preset event begins.

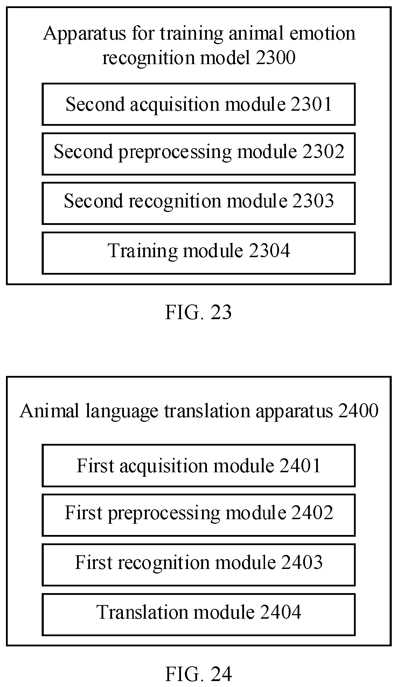

Based on the same technical concept, as shown in FIG. 23, the present disclosure further provides an apparatus 2300 for training an animal emotion recognition model, including:

a second acquisition module 2301, configured to acquire animal-related multimodal data;

a second preprocessing module 2302, configured to preprocess the multimodal data to obtain fused multimodal data;

a second recognition module 2303, configured to input the fused multimodal data into the animal emotion recognition model to recognize current animal motions to obtain animal emotion recognition results; and

a training module 2304, configured to train the animal emotion recognition model based on the emotion recognition results.

In some alternate embodiments, the animal emotion recognition model includes a deep learning model and a generative adversarial network.

The second recognition module 2303 is configured to:

perform, by the deep learning model, sound feature extraction, visual action feature extraction and physical sign change analysis on the fused multimodal data to obtain multimodal feature vectors; and

perform emotion analysis, by the generative adversarial network, on the multimodal feature vectors to obtain the animal emotion recognition results.

In some alternate embodiments, the second recognition module 2303 includes:

a second extraction unit, configured to perform, by the deep learning model, sound feature extraction, visual action feature extraction and physical sign change analysis on the fused multimodal data to obtain a first audio feature, a first visual feature and a first physical sign feature;

a second synchronization unit, configured to perform data synchronization on the first audio feature, the first visual feature and the first physical sign feature to obtain a second audio feature, a second visual feature and a second physical sign feature; and

a second normalization unit, configured to normalize the second audio feature, the second visual feature and the second physical sign feature to obtain the multimodal feature vectors.

In some alternate embodiments, the second extraction unit is particularly configured to:

perform, based on an audio feature extraction module in the deep learning model, feature extraction on audio signals in the fused multimodal data to obtain the first audio feature;

perform, based on a video feature extraction module in the deep learning model, feature extraction on video data in the fused multimodal data to obtain the first visual feature; and

perform, based on a physical sign feature extraction module in the deep learning model, feature extraction on physiological data in the fused multimodal data to obtain the first physical sign feature.

In some alternate embodiments, the second synchronization unit is particularly configured to process frame counts of the first audio feature, the first visual feature and the first physical sign feature within a current time window as a target frame count, respectively, to obtain the second audio feature, the second visual feature and the second physical sign feature.

In some alternate embodiments, wherein the second recognition module 2303 includes:

a second fusion unit, configured to perform crossmodal feature fusion on the multimodal feature vectors to obtain fused features;

a second temporal modelling unit, configured to temporally model the fused features to obtain temporal features; and

a second classification unit, configured to classify the temporal features to obtain the animal emotion recognition results.

In some alternate embodiments, wherein the second fusion unit is configured to:

use a feature vector of an audio modality in the multimodal feature vectors as a first query vector, use one of a feature vector of a video modality and a feature vector of a physiological modality in the multimodal feature vectors as a first key vector and the other one as a first value vector, and process the first query vector, the first key vector and the first value vector based on an attention mechanism to obtain a first to-be-fused feature;

use the feature vector of the video modality in the multimodal feature vectors as a second query vector, use one of the feature vector of the audio modality and the feature vector of the physiological modality in the multimodal feature vectors as a second key vector and the other one as a second value vector, and process the second query vector, the second key vector and the second value vector based on the attention mechanism to obtain a second to-be-fused feature;

use the feature vector of the physiological modality in the multimodal feature vectors as a third query vector, use one of the feature vector of the audio modality and the feature vector of the video modality in the multimodal feature vectors as a third key vector and the other one as a third value vector, and process the third query vector, the third key vector and the third value vector based on the attention mechanism to obtain a third to-be-fused feature; and

perform weighted summation on the first to-be-fused feature, the second to-be-fused feature and the third to-be-fused feature to obtain the fused features.

In some alternate embodiments, wherein with respect to any one of the first to-be-fused feature, the second to-be-fused feature and the third to-be-fused feature, a weight of any one of the to-be-fused features is dynamically generated based on any one of the to-be-fused features and a learnable parameter of a training stage.

In some alternate embodiments, wherein the second temporal modelling unit is particularly configured to:

performing feature encoding on a plurality of fused features at a plurality of time steps within a current time window to obtain a plurality of enhanced features at the plurality of time steps within the current time window;

temporally model the plurality of enhanced features at the plurality of time steps to obtain a plurality of intermediate features within the current time window; and

perform weighted summation on the plurality of intermediate features within the current time window to obtain the temporal features.

In some alternate embodiments, wherein the second temporal modelling unit is particularly configured to: perform feature transformation on the plurality of fused features at the plurality of time steps to obtain the plurality of enhanced features, wherein a feature dimension of the enhanced features is smaller than a feature dimension of the fused features.

In some alternate embodiments, wherein the second temporal modelling unit is particularly configured to:

process, based on a weight generation network, the plurality of intermediate features within the current time window to obtain a plurality of weights corresponding to the plurality of time steps within the current time window; and

perform, based on the plurality of weights, weighted summation on the plurality of intermediate features at the plurality of time steps within the current time window to obtain the temporal features.

In some alternate embodiments, the apparatus 2300 further includes an adversarial training module which is configured to train the animal emotion recognition model through adversarial training.

In some alternate embodiments, wherein the adversarial training module includes:

a first disturbance unit, configured to add disturbances to the fused features corresponding to the multimodal feature vectors at a current time step to generate fused features of multimodal noisy features; and

a first prediction unit, configured to input the fused features of the multimodal noisy features into the animal emotion recognition model to recognize current animal motions to obtain animal emotion prediction results.

In some alternate embodiments, the first disturbance unit is configured to acquire a first statistical value in a first classification probability distribution of a processing result at a previous time step relative to the current time step, wherein the processing result includes an emotion classification result obtained by performing emotion classification on the fused features at the previous time step;

generate a first disturbance intensity based on a negative correlation between an intensity and the first statistical value; and

based on the first disturbance intensity, the fused features corresponding to the multimodal feature vectors and a classification loss gradient of the fused features corresponding to the multimodal feature vectors, generate the fused features of the multimodal noisy features.

In some alternate embodiments, the adversarial training module includes:

a second disturbance unit, configured to add a disturbance to a feature vector of a target modality in the multimodal feature vectors to obtain a disturbance feature of the target modality;

a replacement unit, configured to replace the feature vector of the target modality in the multimodal feature vectors with a disturbance feature of the target modality to obtain a multimodal noisy feature; and

a second prediction unit, configured to input the multimodal noisy features into the animal emotion recognition model to recognize current animal motions to obtain animal emotion prediction results.

The second disturbance unit is configured to:

obtain a second statistical value in a second classification probability distribution of the target modality at the previous time step(relative to the current time step, wherein the second classification probability distribution is a classification probability distribution obtained by performing emotion classification on the feature vector of the target modality;

generate a second disturbance intensity based on a negative correlation between the intensity and the second statistical value; and

based on the second disturbance intensity, add the disturbance to the feature vector of the target modality in the multimodal feature vectors to obtain the disturbance feature of the target modality.

In some alternate embodiments, the training module includes:

a first loss determination unit, configured to determine a first classification loss based on the emotion recognition results and a training label;

a second loss determination unit, configured to determine a second classification loss based on the emotion recognition results and the training label;

a training unit, configured to optimize a model parameter of the animal emotion recognition model based on the first classification loss and the second classification loss.

In some alternate embodiments, the training module is further configured to:

acquire temporal features of the multimodal feature vectors obtained in a process of processing the multimodal feature vectors by the animal emotion recognition model, wherein emotion classification is performed on the temporal features of the multimodal feature vectors to obtain the emotion recognition results;

acquire temporal features of the multimodal noisy features obtained in a process of processing the multimodal noisy features by the animal emotion recognition model, wherein emotion classification is performed on the temporal features of the multimodal noisy features to obtain the emotion prediction results; and

optimize the model parameter of the animal emotion recognition model based on a first consistency loss between the temporal features of the multimodal feature vectors and the temporal features of the multimodal noisy features.

In some alternate embodiments, the training module is further configured to:

determine a second consistency loss between the emotion recognition results and the emotion prediction results; and optimize the model parameter of the animal emotion recognition model based on the second consistency loss.

In some alternate embodiments, the second preprocessing module is configured to, based on a timestamp in the multimodal data, aggregate the multimodal data within the same timestamp range to obtain the fused multimodal data.

In some alternate embodiments, the apparatus 2300 further includes:

a pseudo label generation module, configured to generate pseudo labels of the animal emotions; and a training module, configured to train the animal emotion recognition model based on the pseudo labels.

In some alternate embodiments, the pseudo label generation module includes:

a first output unit, configured to, when the animal emotion recognition model meets a preset condition, input sample data with an expert label into the animal emotion recognition model to obtain a predicted label of the sample data output by the animal emotion recognition model; and

a first generation unit, configured to perform weighted summation on the predicted label of the sample data and the expert label to obtain the pseudo label of the sample data.

In some alternate embodiments, wherein the first generation unit is configured to:

acquire a confidence of the emotion classification distribution of the animal emotion recognition model for a plurality of training samples from a previous epoch;

determine a statistical value of the confidence in the emotion classification distribution of the plurality of training samples as a dynamic threshold;

generate a fused weight of the expert label based on the dynamic threshold as well as a fused weight of the predicted label, wherein the closer to the dynamic threshold, the greater the fused weight of the expert label; and

based on the fused weight of the expert label and the fused weight of the predicted label, perform weighted summation on the predicted label and the expert label to obtain the pseudo label of the sample data.

In some alternate embodiments, the preset condition includes at least one of the following:

the animal emotion recognition model, based on a training epoch count of a training sample with an expert label, has reached a target epoch count; and

the animal emotion recognition model meets a set condition for proceeding to the next training stage based on an emotion recognition accuracy of the training sample with the expert label.

In some alternate embodiments, the pseudo label generation module includes:

a second output unit, configured to determine a classification divergence between a plurality of modalities in the animal-related multimodal data, wherein the classification divergence is used to measure a discrepancy between animal emotion categories expressed by data of different modalities; and

a second generation unit, configured to, when the classification divergence is less than a divergence threshold, fuse a plurality of emotion classification results of the plurality of modalities in the multimodal data to acquire the pseudo labels.

In some alternate embodiments, wherein the second generation unit is configured to:

generate a plurality of weighted values of the plurality of modalities based on classification confidences of the plurality of emotion classification results of the plurality of modalities; and

based on the plurality of weighted values and the plurality of emotion classification results, generate the pseudo labels of the sample data represented by the multimodal data.

In some alternate embodiments, the apparatus 2300 further includes:
a storage module, configured to, when the classification divergence is not less than the divergence threshold, store the multimodal data in an observation pool.

In some alternate embodiments, the apparatus 2300 further includes:

a selection module, configured to, when the confidence of the pseudo label is greater than a dynamic threshold, determine the sample data corresponding to the pseudo label as sample data for training the animal emotion recognition model, wherein

the dynamic threshold is determined based on a statistical value which is a statistical value of a confidence of the emotion classification distribution of the emotion recognition model for the plurality of training samples from the previous epoch.

In some alternate embodiments, the training module includes:

a loss determination unit, configured to determine, based on the pseudo label, at least one of the following training losses: a cross entropy loss of the pseudo label, a loss between the pseudo label and the expert label, and an alignment loss between the pseudo label distribution and the expert label distribution; and

an optimization unit, configured to determine the total loss based on the at least one training loss to optimize the animal emotion recognition model based on the total loss.

In some alternate embodiments, the total loss includes a total cross entropy loss of the plurality of sample data, and the determining, by the loss determination unit, the total cross entropy loss includes:

generating a plurality of loss weights corresponding to the plurality of sample data, wherein the plurality of sample data have a corresponding plurality of pseudo labels; and

based on the plurality of loss weights corresponding to the plurality of sample data, performing weighted summation on the plurality of cross entropy losses of the plurality of pseudo labels to obtain the total cross entropy loss.

In some alternate embodiments, the loss determination unit is configured to generate, based on the discrepancy between the plurality of pseudo labels and the corresponding expert labels, a plurality of loss weights corresponding to the plurality of sample data, wherein
the discrepancy has an inverse correlation with the loss weights.

In some alternate embodiments, the total loss includes a total distance loss of the plurality of sample data, and the determining, by the loss determination unit, the total distance loss includes:

determining a distance between the pseudo labels and the corresponding expert labels in the plurality of sample data; and
determining a statistical value of the distance of the plurality of sample data to obtain the total distance loss.

In some alternate embodiments, the total loss includes a total alignment loss of the plurality of sample data, and the total alignment loss is used to measure a distribution difference between the pseudo labels and the expert labels of the plurality of sample data; and in a training stage, the total alignment loss is minimized by optimizing the model parameter of the animal emotion recognition model.

In some alternate embodiments, the apparatus 2300 further includes:
a sample adding module, configured to, if a preset event is continuously detected for multiple times in an inference stage of the animal emotion recognition model, obtain the multimodal data corresponding to the preset event as newly-added sample data:
the preset event includes at least one of the following:

the emotion recognition results indicate that a first target emotion category is recognized and a confidence of the emotion recognition results is greater than a preset threshold;
the emotion recognition results indicate that a second target emotion category is recognized and continuously detected for n times, wherein n is a positive integer greater than 1; and
animal emotion variation values within a sliding time window to which the emotion recognition results belong is higher than a preset value.

Based on the same technical concept, as shown in FIG. 24, the present disclosure further provides an animal language translation apparatus 2400, including:

a first acquisition module 2401, configured to acquire animal-related multimodal data, wherein the multimodal data includes animal sound data, animal behavior data and animal physical sign data;
a first preprocessing module 2402, configured to preprocess the multimodal data to obtain fused multimodal data;
a first recognition module 2304, configured to recognize current animal motions based on the fused multimodal data to obtain animal emotion recognition results; and
a translation module 2404, configured to perform semantic mapping and language translation on the emotion recognition results to translate animal languages into human languages to obtain language translation results.

In some alternate embodiments, the obtaining, by the first acquisition module 2401, the animal-related multimodal data includes:

collecting sound wave information made by the animal to obtain the animal sound data;
collecting animal body languages and animal action changes to obtain the animal behavior data; and
collecting animal physiologic indicators to obtain the animal physical sign data.

In some alternate embodiments, the preprocessing, by the first preprocessing module 2402, the multimodal data to obtain the fused multimodal data includes:

denoising the multimodal data for cleaning the data to acquire the cleaned multimodal data;
normalizing the cleaned multimodal data to obtain normalized multimodal data; and
temporally aligning and fusing the normalized multimodal data to obtain the fused multimodal data.

In some alternate embodiments, the recognizing, by the first recognition module 2403, the current animal motions based on the fused multimodal data to obtain the animal emotion recognition results includes:

perform, by a deep learning model, sound feature extraction, visual action feature extraction and physical sign change analysis on the fused multimodal data to obtain multimodal feature vectors; and

perform emotion analysis, by a generative adversarial network, on multimodal features to obtain the animal emotion recognition results. Furthermore, apparatuses as shown in FIG. 22 through FIG. 25 can further include the following modules and/or units.

In some alternate embodiments, the performing, by the translation module 2404, the semantic mapping and the language translation on the emotion recognition results to translate the animal languages into the human languages to obtain the language translation results includes:

extracting an emotion label and a sound feature from the emotion recognition results, and converting the sound feature into a standardized sound vector;

performing, by a pretrained language model, semantic mapping on the emotion label and the sound vector to obtain an emotion intention; and

performing, by a language generator, language translation on the emotion intention to generate corresponding human languages to obtain the language translation results.

In some alternate embodiments, the apparatus further includes a first updating module configured to, if specific sound data is detected and no emotion matching history exists, annotate the specific sound data to obtain an updated emotion label; and

dynamically update the sample data based on the updated emotion label to adjust the model parameter according to the updated sample data.

In some alternate embodiments, the apparatus further includes a second updating module, which is configured to: collect multimodal data within a preset time window to obtain animal emotion change data;

perform feature extraction on the emotion change data to obtain an emotion change feature; and

update the emotion label based on a disparity between the emotion change feature within the current time window and an emotion change feature within a previous time window.

In some alternate embodiments, the collecting, by the second updating module, the multimodal data within the preset time window to obtain the animal emotion change data includes:

collecting the multimodal data within the preset time window; and

inputting the multimodal data into an emotion period recognition model to obtain the animal emotion change data by the emotion period recognition model.

In some alternate embodiments, the updating, by the second updating module, the emotion label based on the disparity between the emotion change feature within the current time window and the emotion change feature within previous time window includes:

calculating a Euclidean distance difference between the emotion change feature within the current time window and the emotion change feature within the previous time window to obtain an emotion disparity; and

if the emotion disparity exceeds a preset emotion disparity threshold, upgrading the emotion label to obtain an updated emotion label.

In some alternate embodiments, the second updating module is configured to:

score an emotion weight corresponding to each time window to obtain an emotion weight score; and

accumulate the emotion weight scores corresponding to similar emotion time windows within continuous time periods, and if an accumulated result is greater than a set upgrading threshold, update the emotion label.

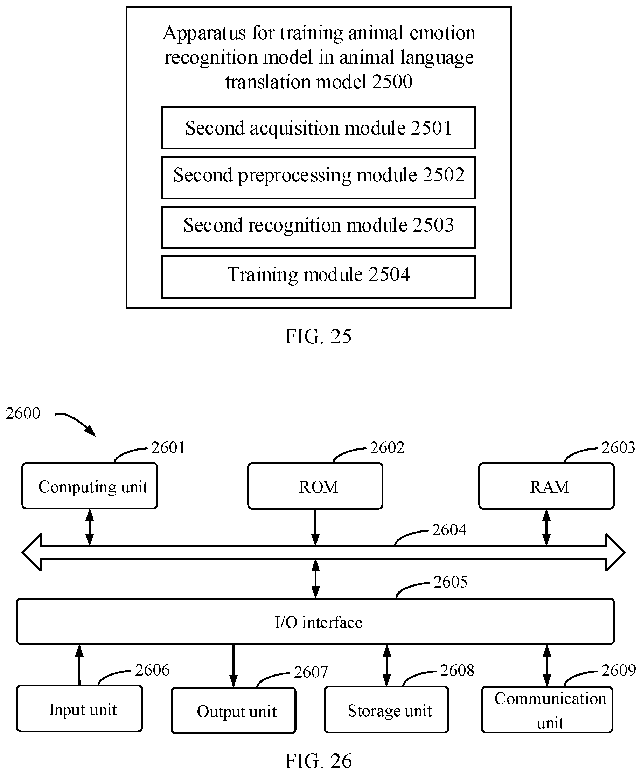

Based on the same technical concept, as shown in FIG. 25, the present disclosure further provides an apparatus 2500 for training an animal emotion recognition model in an animal language translation model, including:

a second acquisition module 2501, configured to acquire animal-related multimodal data;

a second preprocessing module 2502, configured to preprocess the multimodal data to obtain fused multimodal data;

a second recognition module 2503, configured to input the fused multimodal data into the animal emotion recognition model to recognize current animal motions to obtain animal emotion recognition results; and

a training module 2504, configured to train the animal emotion recognition model based on the emotion recognition results, wherein semantic mapping and language translation are performed on the emotion recognition results output by the animal emotion recognition model to translate animal languages into human languages to obtain language translation results.

It should be noted that further modules and/or units in the apparatus 2300 for training the animal emotion recognition model are applicable to the apparatus 2500 for training the animal emotion recognition model in the animal language translation model as well.

In the technical solution of the present disclosure, acquisition, storage, application and the like of involved user personal information comply with relevant laws and regulations, and do not violate public order and good morals.

According to embodiments of the present disclosure, the present disclosure further provides an electronic device, a readable storage medium and a computer program product.

FIG. 26 shows a schematic block diagram of an example electronic device 2600 which may be configured to implement embodiments of the present disclosure. The electronic device is intended to represent various forms of digital computers, such as a laptop computer, a desktop computer, a workstation, a personal digital assistant, a server, a blade server, a mainframe computer, and further appropriate computers. The electronic device may represent various forms of mobile apparatuses, such as a personal digital assistant, a cellular phone, a smart phone, a wearable device, and further similar computing apparatuses. Components shown herein, their connections and relationships, and their functions are merely examples, and are not intended to limit implementations of the present disclosure described and/or claimed herein.

As shown in FIG. 8, the electronic device 2600 includes a computing unit 2601 which may perform various appropriate actions and processes according to a computer program stored in a read only memory (ROM) 2602 or a computer program loaded from a storage unit 2608 into a random-access memory (RAM) 2603. Various programs and data required for the operation of the electronic device 2600 may be also stored in the RAM 2603. The computing unit 2601, the ROM 2602, and the RAM 2603 are connected with each other through a bus 2604. An input/output (I/O) interface 2605 is also connected to the bus 2604.

A plurality of components in the device 2600 are connected to the I/O interface 2605, and include an input unit 2606, such as a keyboard, a mouse, and the like; an output unit 2607, such as various types of displays, speakers, and the like; a storage unit 2608, such as a magnetic disk, an optical disk, and the like; and a communication unit 2609, such as a network card, a modem, a wireless communication transceiver, and the like. The communication unit 2609 allows the device 2600 to exchange information/data with further devices through a computer network, such as Internet, and/or various telecommunication networks.

The computing unit 2601 may be a variety of general-purpose and/or dedicated-purpose processing components with processing and computing capabilities. Some examples of the computing unit 2601 include, but are not limited to, a central processing unit (CPU), a graphic processing unit (GPU), various dedicated artificial intelligence (AI) computing chips, various computing units running a machine learning model algorithm, a digital signal processor (DSP), and any appropriate processor, controller, microcontroller, and the like. The computing unit 2601 performs various methods described above, such as the animal language translation method, the animal emotion recognition method, any one of the methods for training the animal emotion recognition model, and the like. For example, in some embodiments, the animal language translation method, the animal emotion recognition method and any one of the methods for training the animal emotion recognition model may be implemented as a computer software program tangibly contained in a machine readable medium, such as the storage unit 2608. In some embodiments, a part or all of the computer program may be loaded and/or installed into the device 2600 via the ROM 2602 and/or the communication unit 2609. When the computer program is loaded into the RAM 2603 and executed by the computing unit 2601, one or more steps distributed by an application as described above may be performed. Alternatively, in further embodiments, the computing unit 2601 may be configured to perform the animal language translation method, the animal emotion recognition method and any one of the methods for training the animal emotion recognition model in any other appropriate means (for example, by means of a firmware).

Various implementations of systems and technologies described herein may be implemented in a digital electronic circuitry, an integrated circuitry, a field programmable gate array (FPGA), a dedicated application specific integrated circuit (ASIC), an application specific standard product (ASSP), a system on a chip (SOC), a complex programmable logic device (CPLD), a computer hardware, a firmware, a software, and/or a combination thereof. These implementations may include: implemented in one or more computer programs. The one or more computer programs may be executed and/or interpreted on a programmable system including at least one programmable processor. The programmable processor may be a dedicated or general-purpose programmable processor, may receive data and instructions from a storage system, at least one input apparatus and at least one output apparatus, and transmit the data and the instructions to the storage system, the at least one input apparatus and the at least one output apparatus.

Program codes for implementing the method according to the present disclosure may be written in any combination of one or more programming languages. These program codes may be provided to a processor or a controller of a general-purpose computer, a dedicated-purpose computer, or further programmable data processing apparatuses, such that the program codes, when being executed by the processor or the controller, cause functions/operations specified in the flowchart and/or the block diagram to be implemented. The program codes may be executed entirely on a machine, executed partly on a machine, executed partly on a machine as a stand-alone software package and executed partly on a remote machine, or executed entirely on a remote machine or a server.

In the context of the present disclosure, the machine-readable medium may be a tangible medium which may contain or store a program for use by or in conjunction with an instruction execution system, apparatus or device. The machine-readable medium may be a machine-readable signal medium or a machine-readable storage medium. The machine-readable medium may include, but is not limited to, an electronic, magnetic, optical, electromagnetic, infrared, or semiconductor system, apparatus, or device, or any suitable combination thereof. More specific examples of the machine-readable storage medium may include an electrical connection based on one or more wires, a portable computer disk, a hard disk, a random-access memory (RAM), a read-only memory (ROM), an erasable programmable read-only memory (EPROM or flash memory), an optical fiber, a portable compact disc read-only memory (CD-ROM), an optical storage device, a magnetic storage device, or any suitable combination thereof.

To provide interaction with a user, the systems and technologies described here may be implemented on a computer that has a display apparatus (for example, a cathode ray tube (CRT) or a liquid crystal display (LCD) monitor) for displaying information to a user; and a keyboard and a pointing apparatus (for example, a mouse or a trackball) through which a user can provide input to the computer. Other types of apparatuses may further be used to provide interaction with the user. For example, feedback provided to a user can be any form of sensory feedback (for example, visual feedback, auditory feedback or tactile feedback); and input from the user may be received in any form (including acoustic input, speech input or tactile input).

The systems and technologies described herein may be implemented in a computing system (for example, as a data server) which includes a back-end component, or a computing system (for example, as an application server) which includes a middleware component, or a computing system (for example, as a user computer with a graphical user interface or a web browser through which the user may interact with the implementations of the systems and technologies described herein) which includes a front-end component, or a computing system which includes any combination of the back-end component, the middleware component or the front-end component. The components of the system may be connected to each other through any form or medium of digital data communication (for example, a communication network). Examples of the communication network include a local area network (LAN), a wide area network (WAN) and the Internet.

A computer system may include a client and a server. The client and the server are generally far away from each other and usually interact through the communication network. The relationship between the client and the server is generated by virtue of computer programs performed on respective computers and having a client-server relationship with each other.

It should be understood that various forms of processes shown above may be used to reorder, add or delete steps. For example, steps described in the present disclosure may be executed in parallel, sequentially, or in different orders provided that desired results of the technical solution disclosed in the present disclosure may be achieved, which is not limited here.

The foregoing specific implementations do not constitute a limitation on the protection scope of the present disclosure. Those skilled in the art should understand that various modifications, combinations, sub-combinations and substitutions can be made according to design requirements and further factors. Any modifications, equivalent substitutions and improvements made within the spirit and principle of the present disclosure shall be included in the protection scope of the present disclosure.

**Claims**

1. An animal emotion recognition method comprising:

   acquiring animal-related multimodal data;
   preprocessing the multimodal data to obtain fused multimodal data; and
   recognizing current animal motions based on the fused multimodal data to obtain animal emotion recognition results.

2. The method of claim 1, wherein the multimodal data comprises animal sound data, animal behavior data and animal physical sign data.

3. The method of claim 1, wherein the preprocessing the multimodal data to obtain fused multimodal data comprises:

cleaning the multimodal data to obtain cleaned multimodal data;
normalizing the cleaned multimodal data to obtain normalized multimodal data; and
temporally aligning and fusing the normalized multimodal data to obtain the fused multimodal data.

4. The method of claim 1, wherein the recognizing the current animal emotions based on the fused multimodal data to obtain animal emotion recognition results comprises:

performing, by a deep learning model, sound feature extraction, visual action feature extraction and physical sign change analysis on the fused multimodal data to obtain multimodal feature vectors; and
performing, by a generative adversarial network, emotion analysis on the multimodal feature vectors to obtain the animal emotion recognition results.

5. The method of claim 4, wherein the performing emotion analysis, by a generative adversarial network, on the multimodal feature vectors to obtain the animal emotion recognition results comprises:

performing crossmodal feature fusion on the multimodal feature vectors to obtain fused features;
temporally modelling the fused features to obtain temporal features; and
classifying the temporal features to obtain the animal emotion recognition results.

6. The method of claim 4, wherein the performing, by a deep learning model, sound feature extraction, visual action feature extraction and physical sign change analysis on the fused multimodal data to obtain multimodal feature vectors comprises:

performing, by a deep learning model, sound feature extraction, visual action feature extraction and physical sign change analysis on the fused multimodal data to obtain a first audio feature, a first visual feature and a first physical sign feature;
performing data synchronization on the first audio feature, the first visual feature and the first physical sign feature to obtain a second audio feature, a second visual feature and a second physical sign feature; and
normalizing the second audio feature, the second visual feature and the second physical sign feature to obtain the multimodal feature vectors.

7. The method of claim 4, wherein the deep learning model and the generative adversarial network are configured to construct an animal emotion recognition model.

8. The method of any one of claims 1 to 7, further comprising:

if it is determined that a preset event is detected based on the emotion recognition results, increasing a frequency of acquiring the multimodal data; and
the preset event comprises at least one of the following:

the emotion recognition results indicate that a first target emotion category is recognized and a confidence of the emotion recognition results is greater than a preset threshold;
the emotion recognition results indicate that a second target emotion category is recognized and continuously detected for n times, wherein n is a positive integer greater than 1; and
animal emotion variation values within a sliding time window to which the emotion recognition results belong is higher than a preset value.

9. The method of claim 8, further comprising:
with respect to the animal, reducing the frequency of acquiring the multimodal data if the preset event remains undetected for a specified duration starting from the end of detection until the preset event begins.

10. A method for training an animal emotion recognition model, comprising:

acquiring animal-related multimodal data;
preprocessing the multimodal data to obtain fused multimodal data;
inputting the fused multimodal data into the animal emotion recognition model to recognize current animal motions to obtain animal emotion recognition results; and
training the animal emotion recognition model based on the emotion recognition results.

11. The method of claim 10, wherein the animal emotion recognition model comprises a deep learning model and a generative adversarial network;

the inputting the fused multimodal data into the animal emotion recognition model to recognize current animal motions to obtain animal emotion recognition results comprises:

performing, by a deep learning model, sound feature extraction, visual action feature extraction and physical sign change analysis on the fused multimodal data to obtain multimodal feature vectors; and

performing, by a generative adversarial network, emotion analysis on the multimodal feature vectors to obtain the animal emotion recognition results.

12. The method of claim 11, wherein the performing, by a deep learning model, sound feature extraction, visual action feature extraction and physical sign change analysis on the fused multimodal data to obtain multimodal feature vectors comprises:

performing, based on the deep learning model, sound feature extraction, visual action feature extraction and physical sign change analysis on the fused multimodal data to obtain a first audio feature, a first visual feature and a first physical sign feature;

performing data synchronization on the first audio feature, the first visual feature and the first physical sign feature to obtain a second audio feature, a second visual feature and a second physical sign feature; and

normalizing the second audio feature, the second visual feature and the second physical sign feature to obtain the multimodal feature vectors.

13. The method of claim 12, wherein the performing, based on the deep learning model, sound feature extraction, visual action feature extraction and physical sign change analysis on the fused multimodal data to obtain a first audio feature, a first visual feature and a first physical sign feature comprises:

performing, based on an audio feature extraction module in the deep learning model, feature extraction on audio signals in the fused multimodal data to obtain the first audio feature;

performing, based on a video feature extraction module in the deep learning model, feature extraction on video data in the fused multimodal data to obtain the first visual feature; and

performing, based on a physical sign feature extraction module in the deep learning model, feature extraction on physiological data in the fused multimodal data to obtain the first physical sign feature.

14. The method of claim 12, wherein the performing data synchronization on the first audio feature, the first visual feature and the first physical sign feature to obtain a second audio feature, a second visual feature and a second physical sign feature comprises:

processing frame counts of the first audio feature, the first visual feature and the first physical sign feature within a current time window as target frame counts, respectively, to obtain the second audio feature, the second visual feature and the second physical sign feature.

15. The method of claim 11, wherein the performing emotion analysis, by a generative adversarial network, on the multimodal feature vectors to obtain the animal emotion recognition results comprises:

performing crossmodal feature fusion on the multimodal feature vectors to obtain fused features;

temporally modelling the fused feature to obtain temporal features; and

classifying the temporal features to obtain the animal emotion recognition results.

16. The method of claim 15, wherein the performing crossmodal feature fusion on the multimodal feature vectors to obtain fused features comprises:

using a feature vector of an audio modality in the multimodal feature vectors as a first query vector, using one of a feature vector of a video modality and a feature vector of a physiological modality in the multimodal feature vectors as a first key vector and the other one as a first value vector, and processing the first query vector, the first key vector and the first value vector based on an attention mechanism to obtain a first to-be-fused feature;

using the feature vector of the video modality in the multimodal feature vectors as a second query vector, using one of the feature vector of the audio modality and the feature vector of the physiological modality in the multimodal feature vectors as a second key vector and the other one as a second value vector, and processing the second query vector, the second key vector and the second value vector based on the attention mechanism to obtain a second to-be-fused feature;

using the feature vector of the physiological modality in the multimodal feature vectors as a third query vector, using one of the feature vector of the audio modality and the feature vector of the video modality in the multimodal feature vectors as a third key vector and the other one as a third value vector, and processing the third query vector, the third key vector and the third value vector based on the attention mechanism to obtain a third to-be-fused feature; and

performing weighted summation on the first to-be-fused feature, the second to-be-fused feature and the third to-be-fused feature to obtain the fused features.

17. The method of claim 16, wherein with respect to any one of the first to-be-fused feature, the second to-be-fused feature and the third to-be-fused feature, a weight of any one of the to-be-fused features is dynamically generated based on any one of the to-be-fused features and a learnable parameter of a training stage.

18. The method of claim 15, wherein the temporally modelling the fused feature to obtain temporal features comprises:

performing feature encoding on a plurality of fused features at a plurality of time steps within a current time window to obtain a plurality of enhanced features at the plurality of time steps within the current time window;

temporally modelling the plurality of enhanced features at the plurality of time steps to obtain a plurality of intermediate features within the current time window; and

performing weighted summation on the plurality of intermediate features within the current time window to obtain the temporal features.

19. The method of claim 18, wherein the performing feature encoding on a plurality of fused features at a plurality of time steps within a current time window to obtain a plurality of enhanced features at the plurality of time steps within the current time window comprises:

performing feature transformation on the plurality of fused features at the plurality of time steps to obtain the plurality of enhanced features, wherein a feature dimension of the enhanced features is smaller than a feature dimension of the fused features.

20. The method of claim 18, wherein the performing weighted summation on the plurality of intermediate features within the current time window to obtain the temporal features comprises:

processing, based on a weight generation network, the plurality of intermediate features within the current time window to obtain a plurality of weights corresponding to the plurality of time steps within the current time window; and

performing, based on the plurality of weights, weighted summation on the plurality of intermediate features at the plurality of time steps within the current time window to obtain the temporal features.

21. The method of claim 11, further comprising:

training the animal emotion recognition model through adversarial training.

22. The method of claim 21, wherein the training the animal emotion recognition model through adversarial training comprises:

adding disturbances to the fused features corresponding to the multimodal feature vectors at a current time step to generate fused features of multimodal noisy features; and

inputting the fused features of the multimodal noisy features into the animal emotion recognition model to recognize current animal motions to obtain animal emotion prediction results.

23. The method of claim 22, wherein the adding disturbances to the fused features corresponding to the multimodal feature vectors at a current time step to generate fused features of multimodal noisy features comprises:

obtaining a first statistical value in a first classification probability distribution of a processing result at a previous time step relative to the current time step, wherein the processing result comprises an emotion classification result obtained by performing emotion classification on the fused features at the previous time step;

generating a first disturbance intensity based on a negative correlation between an intensity and the first statistical value; and

generating, based on the first disturbance intensity, the fused features corresponding to the multimodal feature vectors and a classification loss gradient of the fused features corresponding to the multimodal feature vectors, the fused features of the multimodal noisy features.

24. The method of claim 21, wherein the training the animal emotion recognition model through adversarial training comprises:

adding disturbances to a feature vector of a target modality in the multimodal feature vectors to obtain a disturbance feature of the target modality;
replacing the feature vector of the target modality in the multimodal feature vectors with the disturbance feature of the target modality to obtain a multimodal noisy feature; and
inputting the multimodal noisy feature into the animal emotion recognition model to recognize current animal motions to obtain the animal emotion prediction results.

25. The method of claim 24, wherein the adding disturbances to a feature vector of a target modality in the multimodal feature vectors to obtain a disturbance feature of the target modality comprises:

obtaining a second statistical value in a second classification probability distribution of the target modality at the previous time step relative to the current time step, wherein the second classification probability distribution is a classification probability distribution obtained by performing emotion classification on the feature vector of the target modality;
generating a second disturbance intensity based on a negative correlation between an intensity and the second statistical value; and
adding, based on the second disturbance intensity, disturbances to the feature vector of the target modality in the multimodal feature vectors to obtain the disturbance feature of the target modality.

26. The method of claim 22 or 24, wherein the training the animal emotion recognition model based on the emotion recognition results comprises:

determining a first classification loss based on the emotion recognition results and a training label;
determining a second classification loss based on the emotion prediction results and the training label; and
optimizing a model parameter of the animal emotion recognition model based on the first classification loss and the second classification loss.

27. The method of any one of claims 22 to 26, further comprising:

acquiring the temporal features of the multimodal feature vectors obtained in a process of processing the multimodal feature vectors by the animal emotion recognition model, wherein emotion classification is performed on the temporal features of the multimodal feature vectors to obtain the emotion recognition results;
acquiring temporal features of the multimodal noisy features obtained in a process of processing the multimodal noisy features by the animal emotion recognition model, wherein emotion classification is performed on the temporal features of the multimodal noisy feature to obtain the emotion prediction results; and
optimizing, based on a first consistency loss between the temporal features of the multimodal feature vectors and the temporal features of the multimodal noisy features, the model parameter of the animal emotion recognition model.

28. The method of any one of claims 22 to 26, further comprising:

determining a second consistency loss between the emotion recognition results and the emotion prediction results; and
optimizing, based on the second consistency loss, the model parameter of the animal emotion recognition model.

29. The method of any one of claim 10 to 28, wherein the preprocessing the multimodal data to obtain fused multimodal data comprises:
aggregating, based on a timestamp in the multimodal data, the multimodal data within a same timestamp range to obtain the fused multimodal data.

30. The method of any one of claims 10 to 29, further comprising:

generating pseudo labels of animal emotions; and
training, based on the pseudo labels, the animal emotion recognition model.

31. The method of claim 30, wherein the generating pseudo labels of animal emotions comprises:

when the animal emotion recognition model meets a preset condition, inputting sample data with expert labels into the animal emotion recognition model to obtain predicted labels of the sample data output by the animal emotion recognition model; and
performing weighted summation on the predicted labels of the sample data and the expert labels to obtain the pseudo labels of the sample data.

32. The method of claim 31, wherein the performing weighted summation on the predicted labels of the sample data and the expert labels to obtain the pseudo labels of the sample data comprises:

obtaining a confidence of the emotion classification distribution of the animal emotion recognition model for a plurality of training samples from a previous epoch;
determining a statistical value of the confidence in the emotion classification distribution of the plurality of training samples as a dynamic threshold;
generating, based on the dynamic threshold, a fused weight of the expert labels as well as a fused weight of the predicted labels, wherein the closer to the dynamic threshold, the greater the fused weight of the expert labels; and
performing, based on the fused weight of the expert labels and the fused weight of the predicted labels, weighted summation on the predicted labels and the expert labels to obtain the pseudo labels of the sample data.

33. The method of claim 31, wherein the preset condition comprises at least one of:

the animal emotion recognition model, based on a training epoch count of a training sample with an expert label, has reached a target epoch count; and; and
the animal emotion recognition model meets a set condition for proceeding to the next training stage based on an emotion recognition accuracy of the training sample with the expert label.

34. The method of claim 30, wherein the generating pseudo labels of animal emotions comprises:

determining a classification divergence between a plurality of modalities in the animal-related multimodal data, wherein the classification divergence is used to measure a discrepancy between animal emotion categories expressed by different modal data; and
when the classification divergence is less than a divergence threshold, fusing a plurality of emotion classification results of the plurality of modalities in the multimodal data to obtain the pseudo labels.

35. The method of claim 34, wherein the fusing a plurality of emotion classification results of the plurality of modalities in the multimodal data to obtain the pseudo labels comprises:

Generating, based on a classification confidence of the plurality of emotion classification results of the plurality of modalities, a plurality of weighted values of the plurality of modalities; and
generating, based on the plurality of weighted values and the plurality of emotion classification results, the pseudo labels of the sample data represented by the multimodal data.

36. The method of claim 34, further comprising:
when the classification divergence is not less than the divergence threshold, storing the multimodal data in an observation pool.

37. The method of claim 30, further comprising:

when the confidence of the pseudo labels is greater than a dynamic threshold, determining the sample data corresponding to the pseudo labels as sample data for training the animal emotion recognition model, wherein the dynamic threshold is determined based on a statistical value which is a statistical value of a confidence of the emotion classification distribution of the emotion recognition model for the plurality of training samples from the previous epoch.

38. The method of any one of claims 30 to 37, wherein the training the animal emotion recognition model based on the pseudo label comprises:

determining, based on the pseudo labels, at least one of the following training losses: a cross entropy loss of the pseudo labels, a loss between the pseudo labels and the expert labels, and an alignment loss between a pseudo label distribution and an expert label distribution; and
determining the total loss based on the at least one training loss to optimize the animal emotion recognition model based on the total loss.

39. The method of claim 38, wherein the total loss comprises a total cross entropy loss of the plurality of sample data, and the determining the total cross entropy loss comprises:

generating a plurality of loss weights corresponding to the plurality of sample data, wherein the plurality of sample data have a corresponding plurality of pseudo labels; and
performing, based on the plurality of loss weights corresponding to the plurality of sample data, weighted summation on the plurality of cross entropy losses of the plurality of pseudo labels to obtain the total cross entropy loss.

40. The method of claim 39, wherein the generating a plurality of loss weights corresponding to the plurality of sample data comprises:

generating, based on the discrepancy between the plurality of pseudo labels and their corresponding expert labels, a plurality of loss weights corresponding to the plurality of sample data, wherein
the discrepancy has an inverse correlation with the loss weights.

41. The method of claim 38, wherein the total loss comprises a total distance loss of the plurality of sample data, and the determining the total distance loss comprises:

determining a distance between pseudo labels and corresponding expert labels in the plurality of sample data; and
determining a statistical value of the distance of the plurality of sample data to obtain the total distance loss.

42. The method of claim 38, wherein the total loss comprises a total alignment loss of the plurality of sample data, and the total alignment loss is used to measure a distribution difference between the pseudo labels and the expert labels of the plurality of sample data; and in a training stage, the total alignment loss is minimized by optimizing the model parameter of the animal emotion recognition model.

43. The method of any one of claims 10 to 42, further comprising:
if a preset event is continuously detected for multiple times in an inference stage of the animal emotion recognition model, acquiring multimodal data corresponding to the preset event to serve as newly-added sample data:
the preset event comprises at least one of the following:

the emotion recognition results indicate that a first target emotion category is recognized and a confidence of the emotion recognition results is greater than a preset threshold;
the emotion recognition results indicate that a second target emotion category is recognized and continuously detected for n times, wherein n is a positive integer greater than 1; and
animal emotion variation values within a sliding time window to which the emotion recognition results belong is higher than a preset value.

44. An animal language translation method, comprising:

acquiring animal-related multimodal data, wherein the multimodal data comprises animal sound data, animal behavior data and animal physical sign data;
preprocessing the multimodal data to obtain fused multimodal data;
recognizing current animal motions based on the fused multimodal data to obtain animal emotion recognition results; and
performing semantic mapping and language translation on the emotion recognition results to translate animal languages into human languages to obtain language translation results.

**45.** The method of claim 44, wherein the acquiring animal-related multimodal data comprises:

collecting sound wave information made by the animals to obtain the animal sound data;
collecting animal body languages and animal action changes to obtain the animal behavior data; and
collecting animal physiological indicators to obtain the animal physical sign data.

**46.** The method of claim 44, wherein the preprocessing the multimodal data to obtain fused multimodal data comprises:

denoising the multimodal data for cleaning the data to obtain the cleaned multimodal data;
normalizing the cleaned multimodal data to obtain normalized multimodal data; and
temporally aligning and fusing the normalized multimodal data to obtain the fused multimodal data.

**47.** The method of claim 44, wherein the recognizing the current animal emotions based on the fused multimodal data to obtain animal emotion recognition results comprises:

performing, by a deep learning model, sound feature extraction, visual action feature extraction and physical sign change analysis on the fused multimodal data to obtain multimodal feature vectors; and
performing, by a generative adversarial network, emotion analysis on the multimodal features to obtain the animal emotion recognition results.

**48.** The method of claim 44, wherein the performing semantic mapping and language translation on the emotion recognition results to translate animal languages into human languages to obtain language translation results comprises:

extracting an emotion label and a sound feature from the emotion recognition results, and converting the sound feature into a standardized sound vector;
performing, by a pretrained language model, semantic mapping on the emotion label and the sound vector to obtain an emotion intention; and
performing, by a language generator, language translation on the emotion intention to generate a corresponding human language to obtain the language translation results.

**49.** The method of any one of claims 44 to 48, further comprising:

if specific sound data is detected and no emotion matching history exists, annotating the specific sound data to obtain an updated emotion label; and
dynamically updating the sample data based on the updated emotion label to adjust the model parameter according to the updated sample data.

**50.** The method of any one of claims 44 to 48, further comprising:

collecting multimodal data within a preset time window to obtain animal emotion change data;
performing feature extraction on the emotion change data to obtain emotion change features; and
updating the emotion label based on a disparity between the emotion change features within the current time window and the emotion change features within a previous time window.

**51.** The method of claim 50, wherein the collecting multimodal data within a preset time window to obtain animal emotion change data comprises:

collecting multimodal data within a preset time window; and
inputting the multimodal data into an emotion period recognition model to obtain animal emotion change data by the emotion period recognition model.

**52.** The method of claim 50, wherein the updating the emotion label based on a disparity between the emotion change features within the current time window and the emotion change features within a previous time window comprises:

calculating a Euclidean distance difference between the emotion change features within the current time window and the emotion change features within the previous time window to obtain an emotion disparity; and
if the emotion disparity exceeds a preset emotion disparity threshold, upgrading the emotion label to obtain an updated emotion label.

**53.** The method of claim 52, further comprising:

scoring emotion weight corresponding to each time window to obtain an emotion weight score; and accumulating the emotion weight scores corresponding to similar emotion time windows within continuous time periods, and if an accumulated result is greater than a set upgrading threshold, updating the emotion label.

**54.** A method for training an animal emotion recognition model in an animal language translation model, comprising:

acquiring animal-related multimodal data;
preprocessing the multimodal data to obtain fused multimodal data;
inputting the fused multimodal data into the animal emotion recognition model to recognize current animal motions to obtain animal emotion recognition results; and
training the animal emotion recognition model based on the emotion recognition results, wherein semantic mapping and language translation are performed on the emotion recognition results output by the animal emotion recognition model to translate animal languages into human languages to obtain language translation results.

**55.** An animal emotion recognition apparatus, comprising:

a first acquisition module, configured to acquire animal-related multimodal data;
a first preprocessing module, configured to preprocess the multimodal data to obtain fused multimodal data; and
a first recognition module, configured to recognize current animal motions based on the fused multimodal data to obtain animal emotion recognition results.

**56.** An apparatus for training an animal emotion recognition model, comprising:

a second acquisition module, configured to acquire animal-related multimodal data;
a second preprocessing module, configured to preprocess the multimodal data to obtain fused multimodal data;
a second recognition module, configured to input the fused multimodal data into the animal emotion recognition model to recognize current animal motions to obtain animal emotion recognition results; and
a training module, configured to train the animal emotion recognition model based on the emotion recognition results.

**57.** An animal language translation apparatus, comprising:

a first acquisition module, configured to acquire animal-related multimodal data, wherein the multimodal data comprises animal sound data, animal behavior data and animal physical sign data;
a first preprocessing module, configured to preprocess the multimodal data to obtain fused multimodal data;
a first recognition module, configured to recognize current animal motions based on the fused multimodal data to obtain animal emotion recognition results; and
a translation module, configured to perform semantic mapping and language translation on the emotion recognition results to translate animal languages into human languages to obtain language translation results.

**58.** An apparatus for training an animal emotion recognition model in an animal language translation model, comprising:

a second acquisition module, configured to acquire animal-related multimodal data;
a second preprocessing module, configured to preprocess the multimodal data to obtain fused multimodal data;
a second recognition module, configured to input the fused multimodal data into the animal emotion recognition model to recognize current animal motions to obtain animal emotion recognition results; and
a training module, configured to train the animal emotion recognition model based on the emotion recognition results, wherein semantic mapping and language translation are performed on the emotion recognition results output by the animal emotion recognition model to translate animal languages into human languages to obtain language translation results.

**59.** An electronic device, comprising:

at least one processor; and

a memory communicatively connected to the at least one processor, wherein
the memory stores an instruction executable by the at least one processor, and the instruction is executed by the at least one processor to cause the at least one processor to perform any one of the methods of any one of claims 1 to 54.

60. A non-transitory computer-readable storage medium storing a computer instruction, wherein the computer instruction is used to cause a computer to perform the method of any one of claims 1 to 54.

61. A computer program product comprising a computer program that, when executed by a processor, implements the method according to any one of claims 1 to 54.

Acquiring animal-related multimodal data — S101

Preprocessing the multimodal data to obtain fused multimodal data — S102

Recognizing current animal emotions based on the fused multimodal data to obtain animal emotion recognition results — S103

Performing semantic mapping and language translation on the emotion recognition results to translate animal languages into human languages to obtain language translation results — S104

FIG. 1

| Animal | Audio collector | Camera device | Body temperature detection | Data processing module |
|---|---|---|---|---|

Animal makes a vocalization

Send captured sound data

Limb actions and behaviors

Send video/image data

Body temperature and heart rate

Send physical sign data

| Animal | Audio collector | Camera device | Body temperature detection | Data processing module |
|---|---|---|---|---|

FIG. 2

```
┌──────────────┐      ┌──────────────────┐
│  Sound data  │─────▶│ Data preprocessing│
│              │      │ and normalization │
└──────────────┘      └──────────────────┘

┌──────────────┐      ┌──────────────────┐      ┌──────────┐      ┌──────────┐
│  Image data  │─────▶│ Data preprocessing│────▶│ Temporal │─────▶│Fused data│
│              │      │ and normalization │     │alignment │      │          │
└──────────────┘      └──────────────────┘      └──────────┘      └──────────┘

┌──────────────────┐  ┌──────────────────┐
│ Body temperature │─▶│ Data preprocessing│
│ and heartbeat data│ │ and normalization │
└──────────────────┘  └──────────────────┘
```

FIG. 3

```
                  ┌────────────────────────┐
                  │  Fused multimodal data │
                  └────────────────────────┘

┌──────────────┐      ┌────────────────────────┐      ┌──────────────┐
│ Sound feature│      │ Visual feature extraction│     │ Physical sign│
│  extraction  │      │                         │      │change analysis│
└──────────────┘      └────────────────────────┘      └──────────────┘

              ┌────────────────────────────┐
              │ Feature vector combination │
              └────────────────────────────┘

              ┌────────────────────────────┐
              │ Emotion recognition generative│
              │     adversarial network     │
              └────────────────────────────┘

              ┌────────────────────────────┐
              │ Emotion classification label│
              └────────────────────────────┘
```

FIG. 4

S501

Performing, by a deep learning model, sound feature extraction, visual action feature extraction and physical sign change analysis on fused multimodal data to obtain a first audio feature, a first visual feature and a first physical sign feature

S502

Performing data synchronization on the first audio feature, the first visual feature and the first physical sign feature to obtain a second audio feature, a second visual feature and a second physical sign feature

S503

Normalizing the second audio feature, the second visual feature and the second physical sign feature to obtain multimodal feature vectors

FIG. 5

S601

Performing crossmodal feature fusion on multimodal feature vectors to obtain fused features

S602

Temporally modelling the fused features to obtain temporal features

S603

Classifying the temporal features to obtain animal emotion recognition results

FIG. 6

S701

Acquiring animal-related multimodal data

S702

Preprocessing the multimodal data to obtain fused multimodal data

S703

Inputting the fused multimodal data into an animal emotion recognition model to recognize current animal motions to obtain animal emotion recognition results

S704

Training an animal emotion recognition model based on the emotion recognition results

FIG. 7

S801

Using a feature vector of an audio modality in multimodal feature vectors as a first query vector, using one of a feature vector of a video modality and a feature vector of a physiological modality in the multimodal feature vectors as a first key vector and the other one as a first value vector, and processing the first query vector, the first key vector and the first value vector based on an attention mechanism to obtain a first to-be-fused feature

S802

Using the feature vector of the video modality in the multimodal feature vectors as a second query vector, using one of the feature vector of the audio modality and the feature vector of the physiological modality in the multimodal feature vectors as a second key vector and the other one thereof as a second value vector, and processing the second query vector, the second key vector and the second value vector based on an attention mechanism to obtain a second to-be-fused feature

S803

Using the feature vector of the physiological modality in the multimodal feature vectors as a third query vector, using one of the feature vector of the audio modality and the feature vector of the video modality in the multimodal feature vectors as a third key vector and the other one thereof as a third value vector, and processing the third query vector, the third key vector and the third value vector based on an attention mechanism to obtain a third to-be-fused feature

S804

Performing weighted summation on the first to-be-fused feature, the second to-be-fused feature and the third to-be-fused feature to obtain fused features

FIG. 8

Preprocessed feature

First (second) audio feature | First (second) video feature | First (second) physical sign feature

Audio modality transformer 901 | Video modality transformer 902 | Feature modality transformer 903

Feature vector of audio modality | Feature vector of video modality | Feature vector of video modality

Attention module 904 | Attention module 905 | Attention module 906

First to-be-fused feature | First to-be-fused feature | First to-be-fused feature

Weighted fusion layer 907

**Fusion network**

Fused feature

FIG. 9

S1001

Adding disturbances to fused features corresponding to multimodal feature vectors at a current time step to generate fused features of multimodal noisy features

S10011

Obtaining a first statistical value in a first classification probability distribution of a processing result at a previous time step relative to the current time step, wherein the processing result includes an emotion classification result obtained by performing emotion classification on fused features at the previous time step

S10012

Generating a first disturbance intensity based on a negative correlation between an intensity and the first statistical value

S10013

Generating fused features of the multimodal noisy features based on the first disturbance intensity, the fused features corresponding to the multimodal feature vectors and a classification loss gradient of the fused features corresponding to the multimodal feature vectors

S1002

Inputting the fused features of the multimodal noisy features into an animal emotion recognition model to recognize current animal motions to obtain animal emotion prediction results

FIG. 10

```
                                                               ⌐ S1101
┌──────────────────────────────────────────────────────────────────┐
│  Adding a disturbance to a feature vector of a target modality in  │
│  multimodal feature vectors to obtain a disturbance feature of the │
│  target modality                                                   │
│                                                        ⌐ S11011    │
│  ┌──────────────────────────────────────────────────────────────┐ │
│  │  Obtaining a second statistical value in a second            │ │
│  │  classification probability distribution of the target       │ │
│  │  modality at a previous time step relative to a current      │ │
│  │  time step, wherein the second classification probability    │ │
│  │  distribution is a classification probability distribution   │ │
│  │  obtained by performing emotion classification on the        │ │
│  │  feature vector of the target modality                       │ │
│  └──────────────────────────────────────────────────────────────┘ │
│                              │                                     │
│                              ▼                         ⌐ S11012    │
│  ┌──────────────────────────────────────────────────────────────┐ │
│  │  Generating a second disturbance intensity based on a        │ │
│  │  negative correlation between an intensity and the second    │ │
│  │  statistical value                                           │ │
│  └──────────────────────────────────────────────────────────────┘ │
│                              │                                     │
│                              ▼                         ⌐ S11013    │
│  ┌──────────────────────────────────────────────────────────────┐ │
│  │  Adding a disturbance to the feature vector of the target    │ │
│  │  modality in the multimodal feature vectors based on the     │ │
│  │  second disturbance intensity to obtain the disturbance      │ │
│  │  feature of the target modality                              │ │
│  └──────────────────────────────────────────────────────────────┘ │
└──────────────────────────────────────────────────────────────────┘
                              │
                              ▼                          ⌐ S1102
┌──────────────────────────────────────────────────────────────────┐
│  Replacing the feature vector of the target modality in the        │
│  multimodal feature vectors with the disturbance feature of the    │
│  target modality to obtain a multimodal noisy feature              │
└──────────────────────────────────────────────────────────────────┘
                              │
                              ▼                          ⌐ S1103
┌──────────────────────────────────────────────────────────────────┐
│  Inputting the multimodal noisy feature into an animal emotion      │
│  recognition model to recognize current animal motions to obtain    │
│  animal emotion prediction results                                 │
└──────────────────────────────────────────────────────────────────┘
```

FIG. 11

S1201

Determining a first classification loss based on emotion recognition results and a training label

S1202

Determining a second classification loss based on emotion prediction results and the training label

S1203

Optimizing a model parameter of an animal emotion recognition model based on the first classification loss and the second classification loss

S1204

Obtaining temporal features of multimodal feature vectors obtained in a process of processing the multimodal feature vectors by the animal emotion recognition model, wherein emotion classification is performed on the temporal features of the multimodal feature vectors to obtain the emotion recognition results

S1205

Obtaining temporal features of a multimodal noisy feature obtained in a process of processing the multimodal noisy feature by the animal emotion recognition model, wherein emotion classification is performed on the temporal features of the multimodal noisy feature to obtain the emotion prediction results

S1206

Optimizing a model parameter of the animal emotion recognition model based on a first consistency loss between the temporal features of the multimodal feature vectors and the temporal features of the multimodal noisy feature

FIG. 12

Multimodal data

Preprocessing

| Fused multimodal data | Audio signal | Video data | Physiological data |

| Deep learning network 1301 | Audio feature extraction module 13011 | Video feature extraction module 13012 | Physical sign feature extraction module 13013 |

Feature extraction — Feature extraction — Feature extraction

| | First audio feature | First video feature | First physical sign feature |

| Data synchronization module 1302 | Second audio feature | Second video feature | Second physical sign feature |

Normalization — Normalization — Normalization

| Third audio feature | Third video feature | Third physical sign feature |

Fusion network of cross-attention mechanism 1303

Fused feature F at time step t

Encoder 1304

Enhanced features at individual time steps

BiLSTM 1305

Initial time features at individual time steps

Self-attention mechanism module 1306

Intermediate features at individual time steps

Dynamic weighting layer 1307

Temporal features within current time window

Classification module 1308

Animal emotion recognition result

FIG. 13

```
                                                          ┌── S1401
┌─────────────────────────────────────────────────────┐
│      Generating pseudo labels of animal emotions     │
└─────────────────────────────────────────────────────┘
                          │
                          ▼                ┌── S1402
┌─────────────────────────────────────────────────────┐
│  Training an animal emotion recognition model based on the │
│                    pseudo labels                     │
└─────────────────────────────────────────────────────┘
```

FIG. 14

S1501

When an animal emotion recognition model meets a preset condition, inputting sample data with expert labels into the animal emotion recognition model to obtain predicted labels of sample data output by the animal emotion recognition model

S1502

Performing weighted summation on predicted labels and the expert labels of the sample data to obtain pseudo labels of the sample data

S15021

Obtaining a confidence of an emotion classification distribution of the animal emotion recognition model for a plurality of training samples from a previous epoch

S15022

Determining a statistical value of the confidence in the emotion classification distribution of the plurality of training samples as a dynamic threshold

S15023

Generating a fused weight of the expert labels and the fused weight of the predicted labels based on the dynamic threshold, wherein the closer to the dynamic threshold, the greater the fused weight of the expert labels

S15024

Performing weighted summation on the predicted labels and the expert labels based on the fused weight of the expert labels and the fused weight of the predicted labels to obtain the pseudo labels of the sample data

FIG. 15

S1601

Determining a classification divergence between a plurality of
modalities in animal-related multimodal data

S1602

When the classification divergence is less than a divergence threshold,
fusing a plurality of emotion classification results of the plurality of
modalities in the multimodal data are fused to obtain pseudo labels

S16021

Generating a plurality of weighted values of the plurality of
modalities based on a classification confidence of the plurality of
emotion classification results of the plurality of modalities

S16022

Generating the pseudo labels of the sample data represented by
the multimodal data based on the plurality of weighted values
and the plurality of emotion classification results of the plurality
of modalities

FIG. 16

S1701

Determining at least one of the following training losses based on pseudo labels: a cross entropy loss of the pseudo labels, a loss between the pseudo labels and the expert labels, and an alignment loss between a pseudo label distribution and an expert label distribution

S1702

Determining the total loss based on the at least one training loss to optimize an animal emotion recognition model based on the total loss

S1703

Determining a plurality of loss weights corresponding to the plurality of sample data, wherein the plurality of sample data have a corresponding plurality of pseudo labels

S1704

Performing weighted summation on the plurality of cross entropy losses of the plurality of pseudo labels based on the plurality of loss weights corresponding to the plurality of sample data to obtain the total cross entropy loss

FIG. 17

| Emotion recognizing module | Speech mapping module | Human semantic output module | User |
|---|---|---|---|

Providing emotion labels and sound features

Translated into human speech expressions

Outputting speech translation results

| Emotion recognizing module | Speech mapping module | Human semantic output module | User |
|---|---|---|---|

FIG. 18

S1901

Acquiring animal-related multimodal data

S1902

Preprocessing the multimodal data to obtain fused multimodal data

S1903

Recognizing current animal motions based on the fused multimodal data to obtain animal emotion recognition results

FIG. 19

S2001

Acquiring animal-related multimodal data

S2002

Preprocessing the multimodal data to obtain fused multimodal data

S2003

Inputting the fused multimodal data into an animal emotion recognition model to recognize current animal motions to obtain animal emotion recognition results

S2004

Training the animal emotion recognition model based on the emotion recognition results, wherein semantic mapping and language translation are performed on the emotion recognition results output by the animal emotion recognition model to translate animal languages into human languages to obtain language translation results

FIG. 20

| Sound collection | Body temperature sensor | Data processing and fusion | Emotion feedback | Dynamic label alteration |
|---|---|---|---|---|

Performing audio analysis on barks

Obtaining a body temperature rising region

Analyzing audio/ body temperature variations

Upgrading an "alert" label into an "anxiety" label

Updating a current label in a model

| Sound collection | Body temperature sensor | Data processing and fusion | Emotion feedback | Dynamic label alteration |
|---|---|---|---|---|

FIG. 21

Animal emotion recognition apparatus 2200

First acquisition module 2201

First preprocessing module 2202

First recognition module 2203

FIG. 22

Apparatus for training animal emotion
recognition model 2300

Second acquisition module 2301

Second preprocessing module 2302

Second recognition module 2303

Training module 2304

FIG. 23

Animal language translation apparatus 2400

First acquisition module 2401

First preprocessing module 2402

First recognition module 2403

Translation module 2404

FIG. 24

Apparatus for training animal emotion recognition model in animal language translation model 2500

Second acquisition module 2501

Second preprocessing module 2502

Second recognition module 2503

Training module 2504

FIG. 25

2600

2601
Computing unit

2602
ROM

2603
RAM

2604

2605
I/O interface

2606
Input unit

2607
Output unit

2608
Storage unit

2609
Communication unit

FIG. 26

## INTERNATIONAL SEARCH REPORT

| | International application No. |
|---|---|
| | **PCT/CN2025/113694** |

**A. CLASSIFICATION OF SUBJECT MATTER**

G10L17/26(2013.01)i; G10L25/63(2013.01)i; G10L15/24(2013.01)i; G10L25/30(2013.01)i

According to International Patent Classification (IPC) or to both national classification and IPC

**B. FIELDS SEARCHED**

Minimum documentation searched (classification system followed by classification symbols)

IPC: G10L

Documentation searched other than minimum documentation to the extent that such documents are included in the fields searched

Electronic data base consulted during the international search (name of data base and, where practicable, search terms used)

CNABS; CNTXT; CNKI: CJFD: 万方, WANFANG; 超星, CHAOXING; VEN; USTXT; EPTXT; WOTXT; IEEE: 多模态, 情感, 情绪, 心情, 状态, 声音, 行为, 动作, 体征, 表情, 识别, 融合, 特征, 动物, 宠物, 模型, 训练, multi?mod+, mood?, condition, emotion, voice, action, expression, recogni+, feature, fusion, train+, model?

**C. DOCUMENTS CONSIDERED TO BE RELEVANT**

| Category* | Citation of document, with indication, where appropriate, of the relevant passages | Relevant to claim No. |
|---|---|---|
| PX | CN 119943059 A (BEIJING BAIDU NETCOM SCIENCE AND TECHNOLOGY CO., LTD.) 06 May 2025 (2025-05-06) <br> claims 1-14, and description, paragraphs 3-157 | 1-15, 21, 43-61 |
| X | CN 119049086 A (GUANGZHOU JIAKE ELECTRONIC TECHNOLOGY CO., LTD.) 29 November 2024 (2024-11-29) <br> description, paragraphs 3-128 | 1-15, 18-21, 29, 43, 55, 56, 59-61 |
| Y | CN 119049086 A (GUANGZHOU JIAKE ELECTRONIC TECHNOLOGY CO., LTD.) 29 November 2024 (2024-11-29) <br> description, paragraphs 3-128 | 16, 17, 22-54, 57-61 |
| Y | KR 20190125668 A (SUH HYEON MIN) 07 November 2019 (2019-11-07) <br> description, paragraphs 1-77 | 44-54, 57-61 |
| PY | CN 120299449 A (ANHUI XINGHONGYE INTELLIGENT TECHNOLOGY CO., LTD.) 11 July 2025 (2025-07-11) <br> description, paragraphs 6-35 | 16, 17, 22-43, 59-61 |

☐ Further documents are listed in the continuation of Box C.    ☑ See patent family annex.

| | |
|---|---|
| *   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **22 October 2025** | **24 October 2025** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| | International application No. |
|---|---|
| | **PCT/CN2025/113694** |

| Patent document cited in search report | | | Publication date (day/month/year) | Patent family member(s) | Publication date (day/month/year) |
|---|---|---|---|---|---|
| CN | 119943059 | A | 06 May 2025 | None | |
| CN | 119049086 | A | 29 November 2024 | None | |
| KR | 20190125668 | A | 07 November 2019 | None | |
| CN | 120299449 | A | 11 July 2025 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411793938